# EUROPEAN PATENT APPLICATION

(11) **EP 3 929 295 A1**
(43) Date of publication of application: **29.12.2021**
(21) Application number: 20382569.0
(22) Date of filing: 26.06.2020
(51) Int. Cl.: C12N 15/113

(54) **ARTIFICIAL RNAS FOR MODULATING RNA FRAGMENTS**

(71) Applicant: Universitat Pompeu Fabra, 08002 Barcelona (ES)
(72) Inventor: DÍEZ ANTÓN, Juana María, 08002 Barcelona (ES); DOTU RODRÍGUEZ, Ivan Javier, 08002 Barcelona (ES); TALLÓ PARRA, Marc, 08002 Barcelona (ES)
(74) Representative: Hoffmann Eitle

(57) **Abstract**

The present invention relates to an artificial RNA having at least one hybridization region against one or more target disruption structures of one or more RNA fragments, wherein such an artificial RNA suitable for disrupting by hybridization one or more target disruption structures of one or more RNA fragments, thereby modulating the functionality of the one or more RNA fragments.

## Description

### Field of the invention

The present invention relates to the field of biotechnology. In particular, the present invention relates to artificial RNAs as defined in the present invention. More particularly, the present invention relates to artificial RNAs suitable for disrupting by hybridization one or more target disruption structures of one or more RNA fragments, thereby modulating the one or more RNA fragments.

### Background of the invention

A vast amount of research and development is underway to extend the range of medicines in general, such as antivirals to treat pathogens currently without commercial treatments or to treat cancer. Also, with the tendency of viruses and tumoral cells to mutate and become drug resistant, the need of the hour is to develop and refresh the pipeline of antitumorals and/or antivirals with new therapies. The opportunities available for this market, according to a recent market report (https://www.mordorintelligence.com/industry-reports/global-antiviral-drugs-market-industry), are (i) an increasing need for broad-spectrum antiviral drugs and (ii) access to antiviral drugs in the pharmerging markets.

Current approved and effective antiviral treatments include molecules that specifically interact with viral proteins essential for the viral life cycle. Developing these therapeutic molecules is costly and highly time consuming. Moreover, every molecule functions solely for a specific virus. Multiple efforts have been made to develop RNA-based antiviral treatments to target the viral RNA genome [Brice A Sullenger and Smita Nair. From the RNA world to the clinic. Science 352 (6292), 1417-1420]. These include the design of 19 nucleotide-long micro RNAs (miRNAs) that specifically interact with viral RNA genomes, leading to their subsequent degradation by the cellular RISC system. Although successful in cell culture, a major drawback for their clinical use is the rapid emergence and selection of mutations in the RNA genome that inhibit the interaction with the miRNAs. Another drawback of miRNAs as antiviral molecules is their stability, as they are naturally degraded by the cellular decay machinery.

Because of the cost and time required to develop new therapeutic agents against viral proteins, RNA-based therapeutics has generated a lot of attention in the last years. RNA-based therapies have been able to address targets untreatable with antibody and small-molecule approaches [Ling-Ling Chen. The biogenesis and emerging roles of circular RNAs. Nature Reviews Molecular Cell Biology 17, 205-211 (2016), doi:10.1038/nrm.2015.32]. Consequently, multiple companies have been created to develop RNA-based therapies to treat multiple diseases. For instance, the company Moderna is developing an mRNA-based vaccine against infection caused by SARS-CoV-2 (Covid-19). These companies focus on the use of antisense, siRNAs, aptamers and microRNA mimics/anti-microRNAs. However, these molecules (i) are rapidly degraded and (ii) share with protein- and antibodies-based therapies the emergence of drug resistance by the high tendency of viruses to mutate.

The present inventors have particularly focused their approach on stable artificial RNAs, such as circular RNAs. Circular RNAs (circRNAs) are back-splicing products of precursor mRNAs that appear naturally in the cell (see review [Ling-Ling Chen. The biogenesis and emerging roles of circular RNAs. Nature Reviews Molecular Cell Biology 17, 205-211 (2016), doi:10.1038/nrm.2015.32]). Previously thought to be irrelevant byproducts, are now proven to perform several functions, such as miRNA sponges and RBP (RNA binding proteins) sponges. Circular RNA has no ends. This is very important, since most mRNA degradation pathways in the cell require 5'or 3'ends for the cellular exonucleases to carry out their degradation function. Consequently, circRNAs are extremely stable molecules. The potential of circRNAs as novel therapeutic platforms have not been fully exploited.

WO2017/222911 discloses the use of circular RNAs generated with exogenous introns to stimulate immune response or circular RNAs generated with endogenous introns to prevent immune recognition of foreign RNA.

WO0061595 discloses a covalently-closed multiple antisense (CMAS)-oligo, which is constructed to form a closed type by ligation using complementary primer, and a ribbon-type antisense (RiAS)-oligo, which is composed of two loops containing multiple antisense sequences and a stem connecting the two loops that is constructed to by ligation using complementary sequences at both 5 prime ends.

WO 2013/162350 A2 relates to the use of circular RNAs composed of 2 purine rich domains that can target viral pyrimidine rich regions in order to form triple helices that can block viral replication.

CN 108165549 A relates to the use of circular RNAs as micro-RNA sponges. It presents an artificial formulation of the well-known function of endogenous circRNAs, where multiple partial complementarity regions target mature micro-RNAs that activate AGO2 pathway upon hybridization.

In addition, there is an increasing need for better understanding molecular mechanisms underlying many pathologies, such as cancer, viral infections, autoimmune diseases, neurological diseases, genetic disorders, etc. In most of these pathologies, RNAs play a role in the underlying mechanisms. Hence, there is a need for tools which would allow the study of the specific functions of the RNAs involved in the molecular mechanisms involving pathologies.

The present invention addresses the above problems, and is directed to tools which allow the modulation of the functionality of RNA fragments, such as, e.g., viral genome, which allow multiple applications. For instance, with the tools of the present invention (artificial RNAs) it is possible to study the structure-function relationship of a certain RNA fragment, or certain regions within a certain RNA fragment. In addition, with the RNAs of the present invention, it is possible to prevent and/or treat diseases where RNA fragments are involved, such as viral infections, cancer, immune diseases, genetic disorders, etc. For instance, with the artificial RNA of the present invention it is possible to study RBPs (ribosome binding proteins) that bind to double stranded RNA motifs.

### Summary of the invention

In a **first aspect**, the present invention relates to an artificial RNA suitable for disrupting by hybridization one or more target disruption structures of one or more RNA fragments, wherein the **artificial RNA** preferably comprises between 150 and 800 nucleotides, more preferably between 200 and 600 nucleotides;
(b) wherein the artificial RNA comprises at least one **hybridization region** which:
   (i) completely hybridizes with at least one target hybridization region comprised in the one or more target disruption structures of the one or more RNA fragments; and
   (ii) preferably has a total of between 7 and 100 nucleotides, more preferably between 10 and 50 nucleotides;
(c) wherein the one or more **target disruption structures:**
   (ii) comprises at least a a hairpin loop preceded or followed by a region of unpaired nucleotides; and
   (i) comprises at least one **target hybridization region** which comprises a single-stranded region of preferably at least 2 nucleotides, more preferably 3 nucleotides or more preceded or followed by a double-stranded region of preferably at least 5 nucleotides, more preferably 10 nucleotides or more, wherein the at least one target hybridization region completely hybridizes with the at least one hybridization region of the artificial RNA; and
(d) wherein the at least one **hybridization region** comprised in the artificial RNA is further characterized because, when hybridizing with the target hybridization region, the energy of the hybridization between the at least one hybridization region and the at least one target hybridization region is more negative than the energy of the target disruption region, thereby disrupting (partially or completely) the target disruption structure, and
wherein the energy of hybridization is preferably measured using the RNAcofold or the RNAstructure software.

Preferably, the artificial RNA is a circular RNA (circRNA).

Preferably, the artificial RNA of the present invention comprises 2 or more hybridization regions, more preferably between 6 and 20 hybridization regions.

In a preferred embodiment, the at least two, and preferably all, of the hybridization regions are capable of completely hybridizing with the same target hybridization region. In a further preferred embodiment, the at least two, and preferably all, of the hybridization regions are different from each other, i.e., they are all different amongst themselves.

Preferably, the one or more RNA fragments is selected from mRNA, tRNA, rRNA, non-coding RNA and viral genomic RNA, preferably the one or more RNA fragments are viral genomic RNA.

In a **second aspect,** the present invention relates to a composition comprising the artificial RNA of the invention.

In a **third aspect,** the present invention relates to a kit comprising the artificial RNA and/or the composition of the present invention.

In a **fourth aspect,** the present invention relates to the artificial RNA and/or the composition of the present invention for use as a medicament, preferably for use in a method of preventing and/or treating a viral infection

### Brief description of the drawings

Figure 1 represents schematically the intracellular production of antiviral circRNA. This figure describes how a circular RNA can be produced within the target cell. First, the figure shows a plasmid containing a CMV promoter, two complementary and repetitive regions surrounding the candidate circular RNA and flanking splicing acceptor and donor sites. This plasmid is transfected into the target cell. The cell then transcribes the linear RNA as shown, and the area between the splice donor and acceptor sites gets circularized through backsplicing.
Figure 2. Synthesis and circularization of RNA. Workflow composed of four major steps: (A) production of T7 promoter-flanked templates by PCR. (B) RNA preparation by *in vitro* transcription. (C) Generation of specific termini for ligation. *In vitro* transcribed RNAs have to be dephosphorylated prior *in vitro* circularization. (D) Ligation with T4 RNA Ligase 1 or circRNA Ligase. Circular or linear oligomers can be formed as side products.
Figure 3 represents schematically the mode of action.This figure shows the mode of action of a circular RNA as it hinders viral cycle. Viruses contain RNA structures in their genome that are vital for their life cycles. The circular RNA targets those structures by binding to them in a way that a conformational change occurs that hinders vital steps of the virus life cycle.

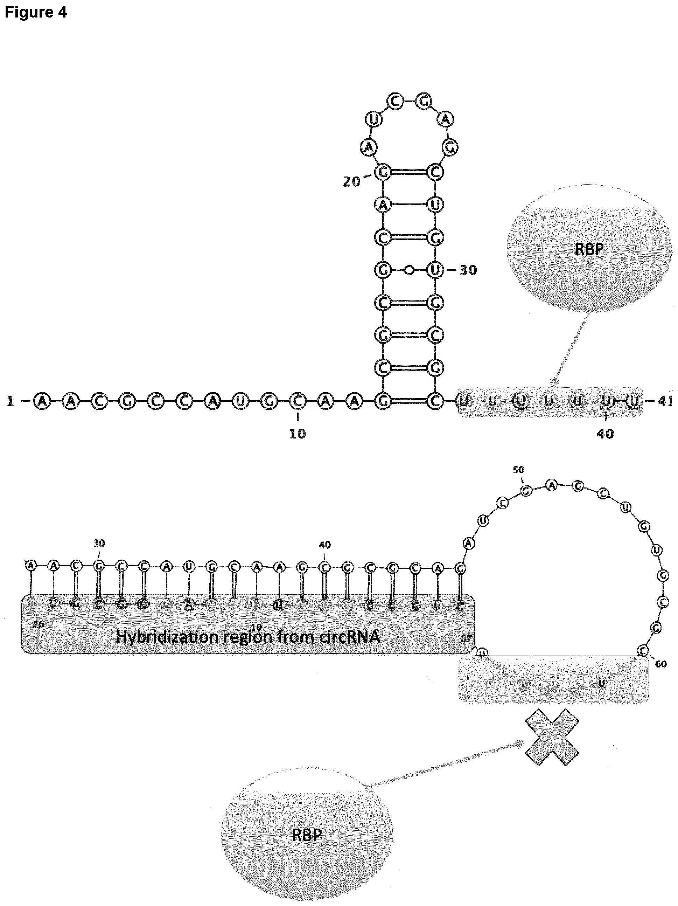

Figure 4 represents schematically an example of a relevant mode of action. This figure shows an example of how hybridization of the circular RNA can alter viral cycle. First, RNA binding proteins (RBPs) are necessary for many steps in the viral life cycle. These RBPs generally bind specific regions of the viral genome, where both sequence and structure (or structural context) are necessary. Similar to the figure, an example is the binding of PTB in the last domain of the IRES element of many picornaviruses, which binds to a box of single stranded pyrimidines immediately 3' of a stable haripin. As in the right drawing, when a circular RNA binds a certain structured region of the IRES and the single stranded region preceding this structured region, it changes the RNA structure in such a way that the box of pyrimidines is not in the same estructural context anymore, and therefore the RBP cannot bind to it.
Figure 5 represents an example of a circRNA that targets 3 different regions of the viral genome (or 3 different viral genomes) with 2 hybridization sequences per target region. Regions with the same color represent that they target the same viral region.
Figure 6 represents schematically the RNA secondary structure of the beginning and end of the HCV genome. Highlighted are the regions to which our circular RNAs are designed to target to.
Figure 7 represents schematically the results of 4 of the designed RNAs against HCV genome. The ids of the circRNAs correspond to the target regions shown in the previous figure. As it can be seen, infection is lowered up to 20% with respect to the control.
Figure 8 represents schematically the RNA secondary structure of the DENV genome. Highlighted are the regions to which our circular RNAs are designed to target to.
Figure 9 represents schematically the results of 3 of the designed RNAs against DENV genome. The ids of the circRNAs correspond to the target regions shown in the previous figure. As it can be seen, infection is lowered up to 40% with respect to the control.
Figure 10 represents the results of the 4 designed RNAs against CHIKV genome. As it can be seen, infection is lowered up to 50% with respect to the control.
Figure 11 represents the results of a broadspectrum circRNA designed against both DENV and HCV genomes when used to treat DENV infection. HCV_CDS2 is one of the circRNAs in example 1 and used here as negative control. DENV1_chp is one of the circRNAs in example 2 and used here as positive control.
Figure 12 represents the results of a broadspectrum circRNA designed against both DENV and HCV genomes when used to treat HCV infection. HCV_CDS2 is one of the circRNAs in example 1 and used here as positive control. DENV1_chp is one of the circRNAs in example 2 and used here as negative control.
Figure 13 represents the results of a second broadspectrum circRNA designed against both DENV and HCV genomes when used to treat DENV infection. HCV_CDS2 is one of the circRNAs in example 1 and used here as negative control. DENV1_chp is one of the circRNAs in example 2 and used here as positive control.
Figure 14 represents the results of a second broadspectrum circRNA designed against both DENV and HCV genomes when used to treat HCV infection. HCV_CDS2 is one of the circRNAs in example 1 and used here as positive control. DENV1_chp is one of the circRNAs in example 2 and used here as negative control.
Figure 15 represents the results of the two designed RNAs against West Nile virus (WNV) genome, circ wnv_slll 1 and circ wnv_slll 2. As it can be seen, infection is lowered with respect to the control.
Figure 16 represents the results of three broadspectrum circRNAs designed against both WNV and DENV (dchp_wsll_A, dchp_wsll_B and dchp_wsll_C) when used to treat WNV infection. Positive and negative controls from examples 2 and 5 were used. As it can be seen, all circRNA showed inhibition.
Figure 17 represents the results of three broadspectrum circRNAs designed against both WNV and DENV (dchp_wsll_A, dchp_wsll_B and dchp_wsll_C) when used to treat DENV infection. Positive and negative controls from examples 2 and 5 were used. As it can be seen, all circRNA showed inhibition.
Figure 18 represents the capacity of the designed RNA against HCV in inhibiting chronically infected cells with HCV. CircRNAs inhibit infectivity in HCV chronically infected cells. Huh7/Scr cells were infected with HCV and 48hpi transfected with circ_hcv_cds2. Two days later luciferase values were measured. Effects on infectivity are determined by changes in luciferase expression levels.
Figure 19 represents the result of the designed RNA against a region required for HCV RNA replication and its ability to inhibit HCV replication. Circ_hcv_cds2, which targets a region required for HCV RNA replication, inhibits HCV RNA replication. Circ_hcv_cds2 inhibited luciferase levels 48 hours post infection when HCV RNA is translated and replicated but not at 4 hours post infection when HCV RNA is solely translated. Effects on infectivity are determined by changes in luciferase expression levels. Statistical significance was calculated using a T-test (*represents *p*-value < 0.05).

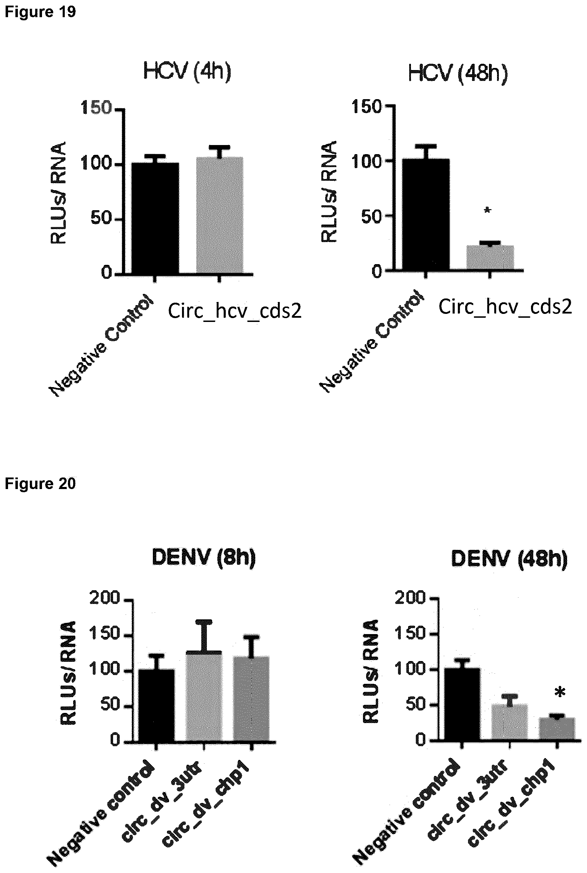

Figure 20 represents the result of the designed RNA against a region required for DENV RNA replication and its ability to inhibit DENV replication. Circ_dv_3utr and circ_dv_cHP_v1, designed to target structures within the DENV RNA genome directing RNA replication, inhibit DENV RNA replication. The circ_dv_3utr and circ_dv_cHP_v1 inhibit luciferase expression levels at 48 hours when the RNA genome is translated and replicated but not at 8 hours when is solely translated. All Results were obtained from at least three biological replicates. Statistical significance was calculated using a T-test (*represents p-value < 0.05).

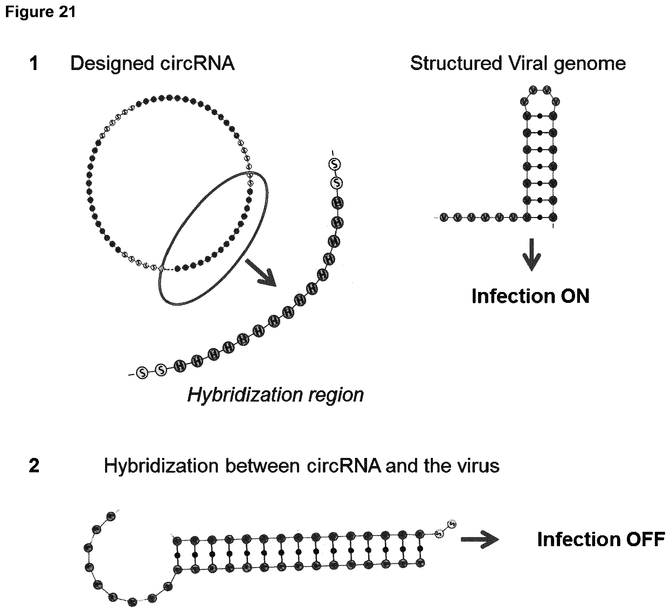

Figure 21. Artificial circRNA structure and mode of action. **1)** CircRNAs contain several different hybridization (brown regions H) and separation sequences (light grey regions S). Hybridization sequences target the viral RNA genome (dark grey regions) and separation sequences allow for structural flexibility and physical separation among the hybridization ones. Note that all H and S sequences are different among themselves. **2**) Hybridization regions are designed to target and disrupt a particular viral RNA genome structure, leading to a decrease in infectivity. The hybridization starts in a single stranded region, an external or hairpin loop or a pseudoknot and finish within a helix, so that the helix is disrupted.

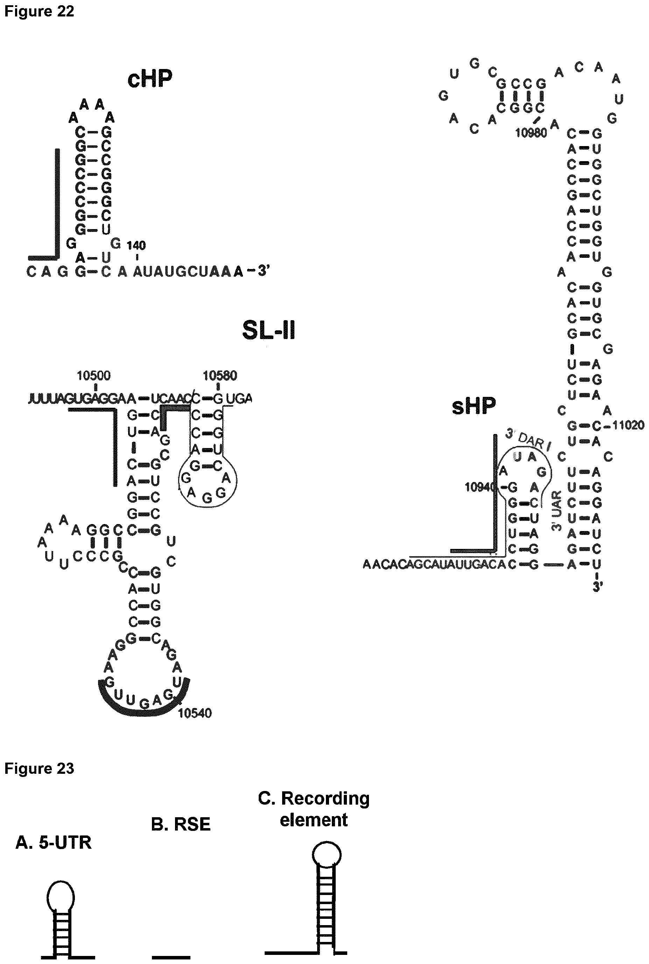

Figure 22. WNV structures used for the design of circWNV. Representation of the 5'-UTR (above) and 3'-UTR (below) from WNV genome. In red the hybridization regions used for the design of the circWNV. UTR: untranslated region [Adapted from Fernandez-Sanlés et al., 2017, Front. Microbiol. 8, 1-16].
Figure 23. Schematic CHIKV structures used for the design of circCHIKV. Representation of the predicted 5'-UTR (A), RSE (B) and Recording Element (C). In A and B all the structure is targeted by the circRNA in C, the hybridization region is depicted in red. UTR: untranslated region; RSE: repetitive sequence elements. [Adapted from Kendra et al., 2018, Virology 339, 200-212; and from Khan et al., 2002, J. Gen. Virol. 83, 3075-3084].

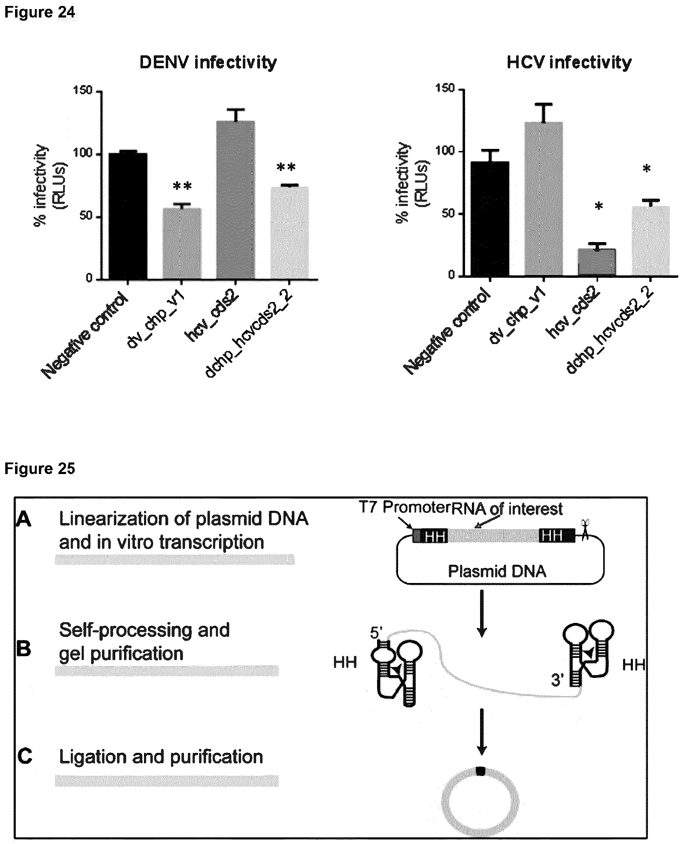

Figure 24. Designed broad-spectrum circRNAs impair both DENV and HCV infectivity. Cells were transfected with the circRNA dv_chp_v1, the circRNA hcv_cds2 or the broad-spectrum circRNA (circ dchp_hcv_cds2_2) containing hybridization sequences from circRNA dv_chp_v1 and circRNA hcv_cds2. Next, cells were infected either with DENV or HCV harbouring the luciferase reporter gene and 48h later the infectivity was measured. All results were obtained from at least three biological replicates. Effects on infectivity are determined by changes in luciferase expression levels. Statistical significance was calculated using a T-test (*represents *p*-value < 0.05).
Figure 25. Depicts the protocol to obtain circular RNAs *in vitro.*
Figure 26. Shows the results of both a circular RNA against DNV and WNV and a circular RNA against HCV generated *in vitro.*
Figure 27. Shows types of target disruption structures (inner line) and target hybridization regions (outer line).

### Detailed description of the invention

### Definitions

In describing the present disclosure, the following terms will be used and are defined as indicated below.

The forms "a", "an" and "the" include plural referents unless the context states otherwise.

The term "about" when referred to a given amount or quantity is meant to include deviations of plus or minus five percent.

The term "artificial circular RNAs" or "circular RNAs" or "circRNAs" is used herein to refer to non-coding RNAs forming covalently closed continuous loops which have the 3' and 5' ends joint together, thus they are missing the 5'-cap and in consequence the polyadenylated tail. Therefore, they are resistant to exonuclease-mediated degradation and to debranching enzymes, which confers them higher stability with respect to other RNAs.

The term "complementary" and "complementarity" are interchangeable and refer to the ability of polynucleotides to form base pairs with one another. Base pairs are typically formed by hydrogen bonds between nucleotide units in antiparallel polynucleotide strands or regions. Complementary polynucleotide strands or regions can base pair in the Watson-Crick manner (e.g., A to T, A to U, C to G). 100% (or total) complementary refers to the situation in which each nucleotide unit of one polynucleotide strand or region can hydrogen bond with each nucleotide unit of a second polynucleotide strand or region. Less than perfect (or partial) complementarity refers to the situation in which some, but not all, nucleotide units of two strands or two regions can hydrogen bond with each other and can be expressed as a percentage.

The term "hybridization" is used to refer to the structure formed by 2 independent strands of RNA that form a double stranded structure via base pairings from one strand to the other. These base pairs are considered to be G-C, A-U and G-U. (A - Adenine, C -Cytosin, G-Guanine, U - Uracil). As in the case of the complementarity, the hybridization can be total or partial.

The term "transfection or "transfect" is used to refer to the uptake of circular RNA by a cell. A cell has been "transfected" when the circular RNA has been introduced inside the cell membrane.

"Stem-loop intramolecular base pairing" ("stem-loops") is a pattern that can occur in single-stranded DNA or, more commonly, in RNA. The structure is also known as a "hairpin" or "hairpin loop". It occurs when two regions of the same strand, usually complementary in nucleotide sequence when read in opposite directions, base-pair to form a double helix that ends in an unpaired loop. The resulting structure is a key building block of many RNA secondary structures. The base-pairing of the helix need not be complete, there could be stretches of single stranded nucleotides, which are called "bulges" or "internal loops". As an important secondary structure of RNA, it can direct RNA folding, protect structural stability for messenger RNA (mRNA), provide recognition sites for RNA binding proteins, and serve as a substrate for enzymatic reactions.

"Internal-loops" (also termed "interior loops"), in RNA, are found where the double stranded RNA separates due to no Watson-Crick base pairing between the nucleotides. Internal-loops can be classified as either symmetrical or asymmetrical, with some asymmetrical internal-loops, also known as bulges. Internal-loops differ from stem-loops as they occur in the middle of a stretch of double stranded RNA.

"External-loops" are stretches of single-stranded nucleotides that separate hairpin loops or multi-loops.

"Multi-loops" are branching off of a double-stranded region into several hairpin loops.

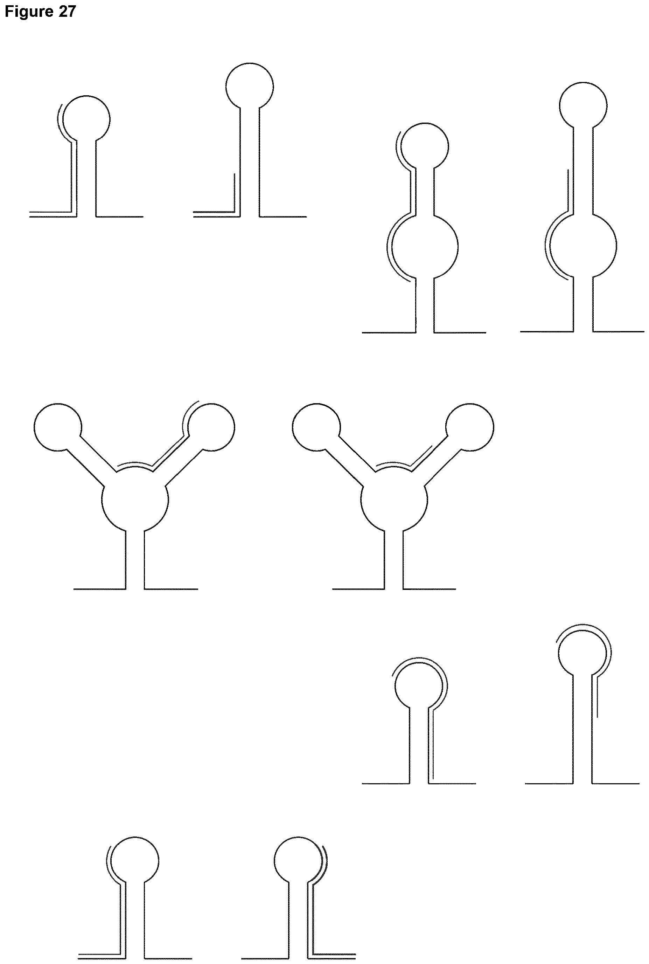

In the context of the present invention a "target disruption structure" is a stem loop (or hairpin loop), preferably preceded or followed by a stretch of unpaired nucleotides, (external loop, bulge or internal loop or multiloop, see figure 27).

In the context of the present invention, a "target hybridization region" is a region within a target disruption structure composed of a single stranded region preceded or followed by a double-stranded region. According to this definition, there are several types of target hybridization regions: external loop, bulge or internal loop, multiloop or stem loop (see Figure 27).

"Disruption by hybridization": given a target disruption structure, a target hybridization region and an artificial RNA that completely hybridizes with the target hybridization region, disruption by hybridization occurs when the energy of the hybridization is more favourable than the energy of the target disruption structure (more negative). Upon such hybridization, the structure of the target disruption structure changes, at least, completely for the overlap between the target disruption structure and the target hybridization region. Such energy calculations can be obtained in silico using well established software like RNAcofold (Lorenz, Ronny and Bernhart, Stephan H. and Höner zu Siederdissen, Christian and Tafer, Hakim and Flamm, Christoph and Stadler, Peter F. and Hofacker, Ivo L., ViennaRNA Package 2.0, Algorithms for Molecular Biology, 6:1 26, 2011, doi:10.1186/1748-7188-6-26; Reuter, J. S., & Mathews, D. H. (2010). RNAstructure: software for RNA secondary structure prediction and analysis. BMC Bioinformatics. 11,129; Mathews, D. H., et al., "Predicting oligonucleotide affinity to nucleic acid targets", RNA, 1999 5: 1458-1469), **RNAstructure** (https://rna.urmc.rochester.edu/RNAstructure.html), **Mfold** (http://unafold.rna.albany.edu/?q=mfold, M. Zuker, "Mfold web server for nucleic acid folding and hybridization prediction", Nucleic Acids Res. 31 (13), 3406-3415, 2003) or **Vienna package** (http://rna.tbi.univie.ac.at/; Lorenz, Ronny and Bernhart, Stephan H. and Höner zu Siederdissen, Christian and Tafer, Hakim and Flamm, Christoph and Stadler, Peter F. and Hofacker, Ivo L., ViennaRNA Package 2.0, Algorithms for Molecular Biology, 6:1 26, 2011, doi:10.1186/1748-7188-6-26).

"Viral conserved structure" refers to viral genomic RNA structures which are conserved among members of the same species, genus, family, etc. Those structures are sometimes characterized in the literature and have been experimentally validated. In the absence of such experimental proof, there exists software that can predict such structures in silico. For instance, all viral genomes of the same family can be aligned, using for example ClustalW, and then using RNAz (RNAz 2.0: Improved noncoding RNA detection, Gruber AR, Findeiß S, Washietl S, Hofacker IL, Stadler PF. Pac Symp Biocomput. 15:69-79, 2010; Nucleic Acids Res. 1994 Nov 11; 22(22): 4673-4680, doi: 10.1093/nar/22.22.4673, PMCID: PMC308517, PMID: 7984417; CLUSTAL W: Improving the sensitivity of progressive multiple sequence alignment through sequence weighting, position-specific gap penalties and weight matrix choice, J D Thompson, D G Higgins, and T J Gibson). RNAz predicts structured regions that are more thermodynamically stable than expected by comparison to random sequences of the same length and sequence composition (z-score), and additionally assesses regions by the support of compensatory and consistent mutations in the sequence alignment. Such conserved structures are believed to be so due to its functional relevance in the life-cycle of the virus.

The term 5'UTR refers to the region upstream of the coding region, immediately before the start codon.

The term "IRES" is defined as an internal ribosome entry site and is an RNA element that allows for translation initiation in a cap-independent manner, as part of the greater process of protein synthesis. In eukaryotic translation, initiation typically occurs at the 5' end of mRNA molecules, since 5' cap recognition is required for the assembly of the initiation complex. The location for IRES elements is often in the 5'UTR, but can also occur elsewhere in mRNAs.

The term CDS refers to the coding region of a messenger RNA or a viral genome. This is the region that codes for the corresponding protein.

The term 3'UTR refers to the region of the viral genome downstream of the CDS, immediately after the stop codon.

The term "cHP" means "capsid-coding region hairpin element" which is a known region of several flavivirus genomes found within the CDS.

The term "RSE" means "conserved repeated sequence element" and it is a known structure found in the 3'UTR of several alphavirus genomes.

"SRVVLC", structured region vital for the viral life cycle, are structured regions of the RNA viral genome which are vital in the viral replication, encapsidation and/or translation, i.e., if disrupted, the virus is less capable of performing essential functions of its life cycle and thus, the virus is less infective.

"Administering" an artificial RNA to a cell comprises transducing, transfecting, electroporating, translocating, fusing, phagocytosing, shooting or ballistic methods, etc., i.e., any means by which a nucleic acid can be transported across a cell membrane.

"Homology region" refers to regions in different virus strains/serotypes which share common structural and/or functional characteristics. Homologous structures do not imply sequence identity as a necessary condition. The skilled person is able to identify homologous regions in other viral strains/serotypes as the ones defined in the present application by conventional means.

The degree of identity between two sequences can be determined by conventional methods, for example, by means of standard sequence alignment algorithms known in the state of the art, such as, for example BLASTn (Altschul S.F. et al. Basic local alignment search tool. J Mol Biol. 1990 Oct 5; 215(3):403-10).

During the description of the claims, the word *"comprising",* and its variants does not intend to exclude other technical characteristics, additives, components or steps. In addition, the term *"comprising"* may also encompass the term "*consisting of*"*.*

### Description of the invention

In general, RNA fragments such as mRNA, tRNA, rRNA, non-coding RNA, viral RNA genomes, viral mRNAs, etc., contain highly structured regions (regions with secondary structure) which are either essential for their function or which, while not being essential, may play a more or less important role in the functionality of said RNA fragment. These highly structured regions comprise hairpin loops or at least portions of hairpin loops preceded or followed by a region of unpaired nucleotides. If the structure (such as the secondary structure) of these hairpin loops or portions of hairpin loops is altered, conformational changes within the RNA fragment take place. This would lead to changes in the functionality of the RNA fragment.

Biological RNA is single stranded and often forms complex and intricate base-pairing interactions due to its increased ability to form hydrogen bonds stemming from the extra hydroxyl group in the ribose sugar. The secondary structure of RNA consists of a single polynucleotide which is folded within the same molecule. Base pairing in RNA occurs when RNA folds between complementarity regions. Both single- and double-stranded regions are often found in RNA molecules. The antiparallel strands form a helical shape. The four basic elements in the secondary structure of RNA are helices, loops, bulges, and junctions. Stem-loop or hairpin loop is the most common element of RNA secondary structure. Stem-loop is formed when the RNA chains fold back on themselves to form a double helical tract called the stem, the unpaired nucleotides forms single stranded region called the loop.

The secondary structure of RNA can be predicted either computationally or with experimental methods. Computationally, RNA secondary structure can be predicted from one or several nucleic acid sequences using tools publically available to the skilled person, e.g., Vienna package, as described above.

As outlined above, RNA structure such as RNA secondary structure is important in many biological processes, including translation regulation in messenger RNA, replication of single-stranded RNA viruses, and the function of structural RNAs and RNA/protein complexes. In fact, for many RNA molecules, the secondary structure is highly important to the correct function of the RNA - often more so than the actual sequence.

Hence, changes in the secondary structure of the RNA fragments will inevitably lead to changes in the functionality of the RNA fragments.

The artificial RNAs of the present invention, preferably circRNAs, are able to change the secondary structure of the RNA fragments they target. Accordingly, with the artificial RNAs of the present invention it is possible to modulate the functionality of the target RNA fragments. For instance, within viral genomes, there are highly structured regions which are vital for the viral life cycle (SRVVLC). If these regions are altered in their conformation or secondary structure (e.g., disrupted), the virus would be less capable (and, preferably incapable) of performing essential functions of its life cycle. Accordingly, altering the conformation or secondary structure of these regions vital for the viral life cycle would lead the virus less infective, ideally totally ineffective. The same principle can be applied to other RNA fragments. For instance, a change in the conformation of a tRNA molecule can lead to a decrease (or increase) in the efficiency of translation of that specific tRNA. For instance, a change in the secondary structure of an mRNA molecule can lead to a decrease (or to an increase) in the binding affinity of that mRNA molecule and a certain protein or even the ribosome and thus affect its translation rate. Changes in the structure of an IRES element, both viral and cellular, can affect translation initiation. Changes in the structure of the UTRs of an mRNA can obscure miRNA binding sites, thus affecting translation regulation of such mRNA. Changes in the structure of intronic regions can affect splicing, etc.

In particular, the artificial RNAs of the present invention, preferably circRNAs, are able to hybridize with specific regions within the target RNA fragment. In doing so, the artificial RNAs of the present invention, preferably circRNAs, are able to disrupt these specific target regions within the target RNA fragment. These "specific regions within the target RNA fragment" are the so-called **"target disruption structures".**

The target disruption structures comprise at least a hairpin loop preceded or followed by a region of unpaired nucleotides, and also comprise the so-called "**target hybridization region(s)".** The target hybridization regions are regions within the target disruption structures which comprise a single-stranded region of at least 2 nucleotides, preceded or followed by a double-stranded region.

The artificial RNAs of the present invention, preferably circRNAs, hybridize with the target hybridization regions through the so-called **"hybridization regions"**. In other words, the artificial RNAs of the present invention, preferably circRNAs, comprise at least one **hybridization region,** which is a region of the artificial RNA which is able to completely hybridize with one or more target hybridization regions comprised in the target disruption regions of the target RNA fragment.

**Hybridization** (or **hybridisation**) is a phenomenon in which two RNA molecules anneal to each other via base pairing interactions. In the context of this invention only canonical base pairings are considered (C-G,A-U and G-U). By "complete hybridization" it is understood that all of the nucleotides of the hybridization region of the artificial RNA anneal with all of the nucleotides of the target hybridization region.

When the hybridization region completely hybridizes with the target hybridization region, the energy of the hybridization between the hybridization region and the target hybridization region is more negative than the energy of the target disruption region. When this happens, the target disruption structure is disrupted, i.e., the secondary structure of the target RNA fragment is altered. Hence, the functionality of the target RNA fragment is also altered: the functionality of the RNA fragment has been modulated by the artificial RNA of the present invention.

In a first aspect, the present invention relates to artificial RNAs, preferably circRNAs, suitable for disrupting by hybridization one or more target disruption structures of one or more RNA fragments.

By "disrupting by hybridization one or more target disruption structures of one or more RNA fragments" it is understood, in the context of the present invention, that the secondary structure of the one or more target disruption structures of one or more RNA fragments is altered when the artificial RNA of the present invention completely hybridizes with the target hybridization region of the target disruption structure.

The artificial RNA of the invention, preferably circRNA, can be of any length as long as it comprises at least one hybridization region as described in the present invention and is suitable for disrupting by hybridization one or more target disruption structures of one or more RNA fragments. Preferably, the RNA of the present invention, preferably circRNA, comprises between 100 and 1000 nucleotides, more preferably between 150 and 800 nucleotides, and even more preferably between 200 and 600 nucleotides.

As described above, the artificial RNA of the present invention, which is preferably a circular RNA, comprises at least one hybridization region. The at least one hybridization region is a region of the artificial RNA (i.e., a sequence of nucleotides) which completely hybridizes with at least one target hybridization region comprised in the one or more target disruption structures of the one or more RNA fragments. The number of nucleotides of the hybridization region is not limited as long as it is able to completely hybridize with at least one target hybridization region, although preferably it has the same length as the target hybridization region, comprised in the one or more target disruption structures and, in doing so, disrupts (i.e., alters the secondary structure) of the one or more target disruption structures.

In a preferred embodiment, the at least one hybridization region has a total of between 7 and 100 nucleotides, more preferably between 10 and 50 nucleotides, even more preferably between 15 and 35 nucleotides, such as between 15 and 25 nucleotides.

As described above, the hybridization region completely hybridizes with at least one target hybridization region comprised in the one or more target disruption structures of the one or more RNA fragments. The target disruption structures are regions of the RNA fragments which comprise at least a portion of a hairpin loop preceded or followed by a region of unpaired nucleotides. The target disruption structures comprise at least one target hybridization region. The target hybridization regions are regions (i.e., a sequence of nucleotides) which comprises a single-stranded region of at least 2 nucleotides preceded or followed by a double-stranded region. The length of the target hybridization region is not limited as long as it comprises a single-stranded region of at least 2 nucleotides preceded or followed by a double-stranded region and is able to completely hybridize with the at least one hybridization region of the artificial RNA. Preferably, the single-stranded region of the target hybridization region comprises 3 nucleotides or more, such as 3, 4, 5, 10, 15 nucleotides or more. Preferably the double-stranded region of the target hybridization region comprises 5 nucleotides or more, such as 5, 7, 10, 15, 20, 25 nucleotides or more.

Importantly, the hybridization region of the artificial RNA of the present invention, which is preferably a circular RNA, completely hybridizes with at least one target hybridization region comprised in the one or more target disruption structures of the one or more RNA fragments. Hence, the at least one hybridization region of the artificial RNA of the present invention has exaclty the same number of nucleotides than the at least one target hybridization region with which it hybridizes. Consequently, the at least one hybridization region of the artificial RNA of the present invention has a first region of a certain number of nucleotides which completely hybridizes with the single-stranded region of the target hybridization region, and a second region of a certain number of nucleotides which completely hybridizes with the double-stranded region of the target hybridization region.

In other words, in a first step, the single-stranded region of the target hybridization region anneals with certain nucleotides of the hybridization region of the artificial RNA, which is preferably a circular RNA. In a second step, the double-stranded region of the target hybridization region, which precedes or follows the single-stranded region, is disrupted, and new interactions are formed between one strand of the double-stranded region of the target hybridization region and certain nucleotides of the hybridization region of the artificial RNA, which anneal to each other. The disruption of the double-stranded region of the target hybridization region occurs when the energy of the hybridization between the at least one hybridization region and the at least one target hybridization region is more negative (more favourable) than the energy of the target disruption region.

As a result, at least the double-stranded region of the target hybridization region is disrupted and, hence, the target disruption structure is also disrupted. Hence, the structure of the target disruption structure changes, at least, completely for the overlap between the target disruption structure and the target hybridization region. Therefore, the secondary structure of the target RNA fragment is changed and, thus, its functionality is also altered.

The hybridization between the hybridization region of the artificial RNA and the target hybridization region of the target disruption structures of the RNA fragment can be followed *in vitro* or *in vivo.* The change in secondary structure of the RNA fragment can also be checked employing techniques well-known in the art such as **SHAPE** (Poulsen, Line Dahl et al. "SHAPE Selection (SHAPES) enrich for RNA structure signal in SHAPE sequencing-based probing data." RNA (New York, N.Y.) vol. 21,5 (2015): 1042-52. doi:10.1261/rna.047068.114) or **PARIS** (Lu Z, Gong J, Zhang QC. PARIS: Psoralen Analysis of RNA Interactions and Structures with High Throughput and Resolution. Methods Mol Biol. 2018; 1649:59-84. doi:10.1007/978-1-4939-7213-5_4).

Consequently, the at least one hybridization region comprised in the artificial RNA of the present invention is further characterized because, when hybridizing with the target hybridization region, the energy of the hybridization between the at least one hybridization region and the at least one target hybridization region is more negative than the energy of the target disruption region, thereby disrupting the target disruption structure. As discussed above, the skilled person is able to predict the energy of the hybridization between the at least one hybridization region and the at least one target hybridization region and the energy of the target disruption region. Available tools for this prediction are, e.g., well established software like **RNAcofold** (Lorenz, Ronny and Bernhart, Stephan H. and Höner zu Siederdissen, Christian and Tafer, Hakim and Flamm, Christoph and Stadler, Peter F. and Hofacker, Ivo L., ViennaRNA Package 2.0, Algorithms for Molecular Biology, 6:1 26, 2011, doi:10.1186/1748-7188-6-26; Reuter, J. S., & Mathews, D. H. (2010). RNAstructure: software for RNA secondary structure prediction and analysis. BMC Bioinformatics. 11,129, Mathews, D. H. et al., "Predicting oligonucleotide affinity to nucleic acid targets", RNA, 1999 5: 1458-1469), **RNAstructure** (https://rna.urmc.rochester.edu/RNAstructure.html), **Mfold** (http://unafold.rna.albany.edu/?q=mfold, M. Zuker, "Mfold web server for nucleic acid folding and hybridization prediction", Nucleic Acids Res. 31 (13), 3406-3415, 2003) or **Vienna package** (http://rna.tbi.univie.ac.at/), as described in detail above.

Preferably, the energy of the hybridization between the at least one hybridization region and the at least one target hybridization region and the energy of the target disruption region is calculated with **RNAcofold** software, with the settings as described in Mathews, D. H., et al., "Predicting oligonucleotide affinity to nucleic acid targets", RNA, 1999 5: 1458-1469.

For a given RNA fragment, the skilled person is able to identify potential target disruption structures, since these are regions of RNA which comprise at least a portion of a hairpin loop preceded or followed by a region of unpaired nucleotides. There are many RNA structure prediction software available to the skilled person and that predict the secondary structure of a given RNA sequence. For instance, we refer to Mathews, D. H., et al. "RNA secondary structure prediction." Current protocols in nucleic acid chemistry vol. Chapter 11 (2007): Unit 11.2. doi:10.1002/0471142700.nc1102s28.

In addition, the skilled person is able to identify one or more target hybridization regions within the target disruption structure, because the target hybridization region is comprised in the target disruption structure and comprises a single-stranded region of at least 2 nucleotides preceded or followed by a double-stranded region, as defined above.

Once a target hybridization region is identified, the skilled person is able to design one or more hybridization regions which would completely hybridize with the target hybridization region. Similarly as above, the skilled person is aware of several software for the design of RNA sequences which would completely hybridize with a given RNA sequence, e.g., **NUPACK** (http://www.nupack.org/design/new, see also B. R. Wolfe, N. J. Porubsky, J. N. Zadeh, R. M. Dirks, and N. A. Pierce, "Constrained multistate sequence design for nucleic acid reaction pathway engineering", J Am Chem Soc, 139:3134-3144, 2017; B. R. Wolfe and N. A. Pierce, "Sequence design for a test tube of interacting nucleic acid strands", ACS Synth Biol, 4:1086-1100, 2015; J. N. Zadeh, B. R. Wolfe, and N. A. Pierce, "Nucleic acid sequence design via efficient ensemble defect optimization" J Comput Chem, 32:439-452, 2011; and R. M. Dirks, M. Lin, E. Winfree, and N. A. Pierce, "Paradigms for computational nucleic acid design" Nucl Acids Res, 32:1392-1403, 2004.) or **RNAiFold** (https://bioinformatics.bc.edu/clotelab/RNAiFold/, see also Juan Antonio Garcia-Martin, Peter Clote, Ivan Dotu, "RNAiFold: A constraint programming algorithm for RNA inverse folding and molecular design, J Bioinform Comput Biol 11(2): 1350001, 2013; and Garcia-Martin JA, Dotu I, Clote P., "RNAiFold 2.0 A web server and software to design custom and Rfam-based RNA molecules", Nucleic Acids Research Web Server issue, 2015, doi: 10.1093/nar/gkv460, "RNAiFold 2.0 A web server and software to design custom and Rfam-based RNA molecules").

Once the one or more target hybridization regions is identified and the one or more hybridization regions is designed, the skilled person is able to predict whether the energy of the hybridization between the at least one hybridization region and the at least one target hybridization region is more negative than the energy of the target disruption region, as described above. Of course, it would also be possible to actually synthesize the artificial RNA comprising the hybridization region(s) and experimentally check whether it completely hybridizes with the target hybridization region, therefore disrupting the target disruption structure, as described above.

In a preferred embodiment, the artificial RNA of the invention is a circular RNA.

The present inventors have found that the use of artificial RNA-based therapies as disclosed herein, preferably circular RNAs, has fundamental advantages over other RNA molecules:
- *Stability.* On one hand, the circRNAs are extremely stable because of their unavailability to the cellular exonucleases. This stability would simplify their use in therapy, in contrast to the other current RNA-based therapies that require chemical modifications that are costly, unnatural, and could lead to toxicity concerns.
- *Resistance to the emergence and selection of escaping mutants.* The designed artificial RNAs, such as circRNAs, preferably contain (i) longer sequences whose hybridization to target hybridization regions will not be affected by single mutations and (ii) multiple hybridization regions capable of completely hybridizing to multiple target hybridization regions in the viral RNA genome, hampering the emergence of resistant mutants by selection of single mutation.
- *Ability to address multiple targets.* All current effective therapies involve molecules that target specific viral proteins and thus can cure only a single infection. By including in the designed artificial RNAs, such as circRNAs, hybridization regions able to hybridize with different target hybridization regions comprised in target disruption structures of different RNA fragments, such as different viral genomes, we will be able to target different RNA fragments, such as different viruses simultaneously. This broad-spectrum therapy is of great value (i) to simplify treatment of co-infections and (ii) to treat acute infections that share geographical locations and initial symptoms such as, for example, those caused by Dengue virus, Zika virus and Chikungynya virus, or those caused by SARS-CoV-2 and influenza. In acute infections early treatment is key to control the disease and epidemics. A broad-spectrum therapy will allow treating even before a final diagnosis is achieved. Moreover, during epidemics the use of such therapies as preventive treatments would be advisable.
- *Minimize the risk of drug resistance.* Moreover, to minimize further the risk of drug resistance, hybridization regions in the designed artificial RNAs, such as circRNAs, (e.g., see Fig. 21) that target the same viral target disruption structure are all different as the artificial RNAs of the present invention, such as circRNAs, may advantageously comprise G-U pairing. In contrast to DNA where complementarity is required, G-U pairing is a valid hybridization pair in RNA.

In a further embodiment, the artificial RNA of the present invention, which is preferably a circular RNA, comprises at least one hybridization region, wherein the at least one hybridization region is capable of hybridizing target hybridization regions from different target disruption structures within the same RNA fragment, or even from different RNA fragments.

In a preferred embodiment, the artificial RNA of the present invention, which is preferably a circular RNA, comprises 2 or more hybridization regions, preferably between 6 and 20 hybridization regions. In a further preferred embodiment, at least two, and preferably all, of the hybridization regions are capable of completely hybridizing with the same target hybridization region. Hence, the artificial RNA according to this preferred embodiment would have more probabilities of disrupting the target disruption structure of the RNA fragment, and would then be more effective in modulating the functionality of the RNA fragment, especially RNA fragments which are prone to mutations (e.g., viruses and/or tumors). Hence, having multiple hybridization regions impacts mainly the mutant escaping capabilities of the virus/tumor.

In another more preferred embodiment, the RNA of the present invention, which is preferably a circular RNA, comprises at least two hybridization regions, wherein at least two, and preferably all, of the hybridization regions have different nucleotide sequences, i.e., are different from each other in terms of nucleotide sequence. Hence, in this preferred embodiment, the at least two hybridization regions, preferably all of the hybridization regions of the artificial RNA, are different from each other, and they all target (are capable of hybridizing) the same target hybridization region ("many-to-one" approach). This way, the artificial RNA according to this preferred embodiment would have more probabilities of disrupting the target disruption structure of the RNA fragment, and would then be more effective in modulating the functionality of the RNA fragment. Alternatively, the at least two hybridization regions, preferably all of the hybridization regions of the artificial RNA, which are different from each other, target (are capable of hybridizing) different target hybridization regions from different target disruption structures, either within the same RNA fragment or even from different RNA fragments.

In a further preferred embodiment, the at least two, and preferably all, of the hybridization regions of the artificial RNA of the invention have a sequence identity of at least 95%, preferably of at least 98%, more preferably of at least 99% among each other.

In a further preferred embodiment, the 2 or more hybridization regions of the artificial RNA of the present invention are:
a) separated by non-hybridization regions of sizes up to 20 nucleotides; or
b) are not separated by non-hybridization regions; or
c) are overlapping.

Preferably, the one or more RNA fragments is selected from mRNA, tRNA, rRNA, non-coding RNA and viral genomic RNA. More preferably, the RNA fragment is viral genomic RNA. Even more preferably, the one or more RNA fragments is positive-sense single-stranded (ss) viral genomic RNA.

Viral RNA genomes and viral mRNAs contain highly structured regions ("target disruption structures") comprising at least a portion of a hairpin loop preceded or followed by a region of unpaired nucleotides which are essential for their function. These highly structured regions are preferably structured regions vital for the viral life cycle (SRVVLC). If these regions are disrupted, the virus would be less capable (and, preferably incapable) of performing essential functions of its life cycle. Accordingly, disrupting these regions would render the virus less infective, ideally totally ineffective.

The present inventors have designed artificial RNAs, preferably circular RNAs, comprising at least one (and preferably more than one) hybridization region that hybridizes to at least one target hybridization region (such as one, or two, or more) within at least one target disruption structure (such as one, or two, or more) present in the viral genomic RNA and disrupts it, consequently reducing or even inhibiting viral infection. As described above, in a preferred embodiment, the artificial RNAs of the present invention comprise at least two (and preferably more) hybridization regions, which are different from each other, and which are able to completely hybridize with one target hybridization region within one target disruption structure present in the viral genomic RNA and disrupt it, consequently reducing or even inhibiting viral infection. In this way ("many-to-one" approach) the efficiency of the disruption is increased.

In one embodiment, the one or more target disruption structures is comprised in the IRES element of the viral genome. Preferably, the one or more target disruption structures comprised in the IRES element of the viral genome is a structured region vital for the viral life cycle (SRVVLC).

In another embodiment, the one or more target disruption structures is comprised in the 5'UTR and/or in the 3'UTR of the viral genome. Preferably, the one or more target disruption structures comprised in the 5'UTR and/or in the 3'UTR of the viral genome is a structured region vital for the viral life cycle (SRVVLC).

In a further embodiment, the one or more target disruption structures is comprised in the CDS of the viral genome. Preferably, the one or more target disruption structures comprised in the CDS of the viral genome is a structured region vital for the viral life cycle (SRVVLC).

In a further embodiment, the at least two hybridization region of the artificial RNA are capable of completely hybridizing with at least one target hybridization region comprised in at least one disruption structure present in any combination of two of the following locations: the IRES element, the region of the 5'UTR, the region of the CDS and/or the region of the 3'UTR of the viral genome.

In a further embodiment, the artificial RNA of the present invention, which is preferably a circular RNA, is able to disrupt by hybridization one or more target disruption structures comprised in at least two viral genomic RNAs. Preferably, the artificial RNA of the present invention is able to disrupt by hybridization one or more structured regions vital for the viral life cycle comprised in at least two viral genomic RNAs.

In a preferred embodiment, the disruption of the one or more structured regions vital for the viral life cycle renders the virus less active, more preferably the disruption of the one or more structured regions vital for the viral life cycle renders the virus completely inactive.

The viral genome as target RNA fragment (viral genomic RNA) is not limited. Any viral genome with target disruption structures (i.e., regions comprising at least a hairpin loop preceded or followed by a region of unpaired nucleotides) may be a suitable target RNA fragment for the artificial RNA of the present invention. Examples of viral genomes which may be target RNA fragments according to the present invention are the following: Hepatitis-C-Virus (HCV), Hepatitis-A-Virus (HAV), Poliovirus, Coxsackie B virus, Coronavirus, Rhinovirus (common cold), Dengue virus , Zika virus, Chikungunya virus, West Nile virus and Yellow Fever virus.

In another preferred embodiment, the target disruption structures that the artificial RNA of the present invention, preferably in the artificial circular RNA of the present invention, is able to disrupt is a target disruption structure comprised in the 5'UTR of the viral genome In a preferred embodiment, the target disruption structure of the 5'UTR region of the DENV (Dengue virus) genome is cHP (capsid coding hairpin region).

In another preferred embodiment, the target disruption structures that the artificial RNA of the present invention, preferably in the artificial circular RNA of the present invention, is able to disrupt is a target disruption structure comprised in the IRES element of the viral genome. In a preferred embodiment, the target disruption structure of the IRES element of the HCV (Hepatitis-C virus) viral genome is IRES1 and/or IRES2.

In another preferred embodiment, the target disruption structures that the artificial RNA of the present invention, preferably in the artificial circular RNA of the present invention, is able to disrupt is a target disruption structure comprised in the CDS of the viral genome. In a preferred embodiment, the target disruption structure of the CDS of the viral genome is CDS1 and/or CDS2. Preferably, the target disruption structure is selected from regions SL388, SL427, SL588 and/or SL750 of the genome of the HCV. In a preferred embodiment, the target disruption structure is region SL427 and/or region SL588 of the genome of the HCV (SL stands for Stem Loop).

In another preferred embodiment, the target disruption structures that the artificial RNA of the present invention, preferably in the artificial circular RNA of the present invention, is able to disrupt is a target disruption structure comprised in the 3'UTR of the viral genome. In a preferred embodiment, the target disruption structure of the 3'UTR region of the DENV genome is sHP (short Stem Loop or short Hairpin region). In a further preferred embodiment, the target disruption structure of the 3'UTR region of the WNV (West Nile virus) genome is SL_II.

In another preferred embodiment, in the circular RNA according to any of the preceding embodiments, the target disruption structures are found in a combination of two or more of the IRES element, the structured region of the 5'UTR, the structured region of the CDS or the structured region of the 3'UTR of the viral genome.

In another preferred embodiment, the target disruption structure of the CHIKV (Chikungunya virus) genome is the RSE region and/or the Recoding Element (RE).

In a further preferred embodiment, the one or more target hybridization regions to which the at least one hybridization region of the artificial RNA according to the present invention, preferably of the artificial circular RNA of the present invention, completely hybridizes is comprised in SEQ ID NO.: 1. In particular, the target hybridization region comprises the nucleotide sequence as defined in one or more of SEQ ID NO.: 25 to 28, or a nucleotide sequence with at least 70% identity to the nucleotide sequence as defined in SEQ ID NO.: 25 to 28, preferably with at least 80%, more preferably with at least 90%, even more preferably with at least 95% identity to the nucleotide sequence as defined in SEQ ID NO.: 25 to 28, or the homologe regions in another HCV strain/serotype.

In a preferred embodiment, the one or more target hybridization regions to which the at least one hybridization region of the artificial RNA according to the present invention, preferably of the artificial circular RNA of the present invention, completely hybridizes comprises the nucleotide sequence as defined in SEQ ID NO.: 25 to 27 (combination circ_hcv_ires1, circ HCV2 and circ_hcv_cds1, see Table 1 below) or a nucleotide sequence with at least 70% identity to the nucleotide sequence as defined in SEQ ID NO.: 25 to 27, preferably with at least 80%, more preferably with at least 90%, even more preferably with at least 95% identity to the nucleotide sequence as defined in SEQ ID NO.: 25 to 27, or the homologe regions in another HCV strain/serotype.

The degree of identity between two sequences can be determined by conventional methods, for example, by means of standard sequence alignment algorithms known in the state of the art, such as, for example BLASTn (Altschul S.F. et al. Basic local alignment search tool. J Mol Biol. 1990 Oct 5; 215(3):403-10).

"Homology region" refers to regions in different virus strains/serotypes which share common structural and/or functional characteristics. Homologous structures do not imply sequence identity as a necessary condition. The skilled person is able to identify homologe regions in other viral strains/serotypes as the ones defined in the present application by conventional means.

**Table 1.**

| **Name of the artificial circular RNA** | **Target disruption structure comprised in** | **Target hybridization region** | **SEQ ID NO.:** | **Hybridization regions of the artificial RNA** |
|---|---|---|---|---|
| circ_hcv_ires1 | IRES 1 | CUCCGCCAUGA AUCACUCCCCU GUGAGGAACUA SEQ ID NO.: 25 | SEQ ID NO.:2 | 7 |
| Circ HCV2 | IRES 2 | UCUCGUAGACC GUGCACCAUGA GC SEQ ID NO.: 26 | SEQ ID NO.:3 | 11 |
| circ_hcv_cds1 | CDS1 (target disruption structure: cHP) | CCAAAAGAAAC ACCAACCGUCG CCCAGA SEQ ID NO.: 27 | SEQ ID NO.:4 | 8 |
| circ_hcv_combo1 | IRES- CDS | Combination circ_hcv_ires1, circ HCV2 and circ_hcv_cds1 | SEQ ID NO.:5 | 3 xtarget |
| circ_hcv_cds2 | CDS2 | GGGGCCCCAGG UUGGG SEQ ID NO.: 28 | SEQ ID NO.:6 | 12 |
| | | | SEQ ID NO,: 64 | |

**Table 1. CircRNAs designed against HCV:** The different circRNAs designed against HCV are classified in the table with information of the target disruption structures which they disrupt by hybridization, the sequence of the target hybridization region which completely hybridizes with the hybridization region of the artificial RNA, the sequence of the whole circRNA and the number of hybridization regions present in each candidate (each artificial circular RNA). IRES: internal ribosomal entry site; CDS: coding sequence; cHP: capsid hairpin.

In a further preferred embodiment, the one or more target hybridization regions to which the at least one hybridization region of the artificial RNA according to the present invention, preferably of the artificial circular RNA of the present invention, completely hybridizes is comprised in SEQ ID NO.: 7. In particular, the target hybridization region comprises the nucleotide sequence as defined in one or more of SEQ ID NO.: 29 to 30 or a nucleotide sequence with at least 70% identity to the nucleotide sequence as defined in SEQ ID NO.: 29 to 30, preferably with at least 80%, more preferably with at least 90%, even more preferably with at least 95% identity to the nucleotide sequence as defined in SEQ ID NO.: 29 to 30, or the homologe regions in another DENV strain/serotype.

**Table 2:**

| **Name of the artificial circular RNA** | **Target disruption structure comprised in** | **Target hybridization region** | **SEQ ID NO.:** | **Hybridization regions of the artificial RNA** |
|---|---|---|---|---|
| circ_dv_3utr | 3' UTR (target disruption structure: sHP) | AACAGCAUAU UGACGCUGGG AAAGACCAGA GA SEQ ID NO.: 29 | SEQ ID NO.:8 | 7 |
| circ_dv_cHP_v1 | CDS (target disruption structure: eHP1) | ACGGAAAAAG GCGAAAAACA CGCCUUUCAA UAU SEQ ID NO.: 30 | SEQ ID NO.:9 | 7 |
| circ_dv_cHP_v2 | CDS (target disruption structure: cHP2) | SEQ ID NO.: 30 | SEQ ID NO.:1O | 7 |

**Table 2. CircRNAs designed against DENV:** The different circRNAs designed against DENV are classified in the table with information of the target disruption structures which they disrupt by hybridization, the sequence of the target hybridization region which completely hybridizes with the hybridization region of the artificial RNA, the sequence of the whole circRNA and the number of hybridization regions present in each candidate (each artificial circular RNA). UTR: untranslated region cHP: capsid region hairpin

In a further preferred embodiment, the one or more target hybridization regions to which the at least one hybridization region of the artificial RNA according to the present invention, preferably of the artificial circular RNA of the present invention, completely hybridizes is comprised in one or more of SEQ ID NO.: 1 and SEQ ID NO.: 7. In particular, the target hybridization region comprises the nucleotide sequence as defined in one or more of SEQ ID NO.: 30 and SEQ ID NO.: 28 or a nucleotide sequence with at least 70% identity to the nucleotide sequence as defined in SEQ ID NO.: 30 and SEQ ID NO.: 28, preferably with at least 80%, more preferably with at least 90%, even more preferably with at least 95% identity to the nucleotide sequence as defined in SEQ ID NO.: 30 and SEQ ID NO.: 28, or the homologe regions in another DENV/HCV strain/serotype.

**Table 3:**

| **Name of the artificial circular RNA** | **Target disruption structure comprised in** | **Target hybridization region** | **SEQ ID NO.:** | **Hybridization regions of the artificial RNA** |
|---|---|---|---|---|
| circ_dv_cHP_v 1_ | DENV: CDS (target disruption structure: cHP) HCV: CDS | ACGGAAAAAGGCGAAA AACACGCCUUUCAAUA U SEQ ID NO.: 30 | SEQ ID NO: 32 | 4 x target |
| circ_hcv_cds2_ 2 | | GGGGCCCCAGGUUGGG SEQ ID NO.: 28 | | |
| DENV cHP_HCV CDS2_T | DENV: CDS (target disruption structure: cHP) HCV: CDS2 | SEQ ID NO.: 30 | SEQ ID NO: 16 | 19 |
| | | SEQ ID NO.: 28 | | |
| DENV1cHP_H CV CDS2_1 | DENV: CDS (target disruption structure: cHP) HCV: CDS2 | SEQ ID NO.: 30 | SEQ ID NO: 17 | 4xtarget |
| | | SEQ ID NO.: 28 | | |

**Table 3. Broad-spectrum DENV-HCV circRNA.** Information for circ_dv_cHP_v1-circ_hcv_cds2 of the two target disruption structures (DENV cHP and HCV CDS), the target hybridization regions, the sequence of the complete circRNA and the number of hybridization regions in each circRNA. cHP: capsid region hairpin CDS: coding sequence.

In a further preferred embodiment, the one or more target hybridization regions to which the at least one hybridization region of the artificial RNA according to the present invention, preferably of the artificial circular RNA of the present invention, completely hybridizes is comprised in SEQ ID NO. 11. In particular, the target hybridization region comprises the nucleotide sequence as defined in one or more of SEQ ID NO.: 31, 33 and 35 or a nucleotide sequence with at least 70% identity to the nucleotide sequence as defined in SEQ ID NO.: 31, 33 and 35, preferably with at least 80%, more preferably with at least 90%, even more preferably with at least 95% identity to the nucleotide sequence as defined in SEQ ID NO.: 31, 33 and 35, or the homologe regions in another CHIKV strain/serotype.

**Table 4:**

| **Name of the artificial circular RNA** | **Target disruption structure comprised in** | **Target hybridization region** | **SEQ ID NO.:** | **Hybridization regions of the artificial RNA** |
|---|---|---|---|---|
| chikv5utr | 5' UTR | ACACACGUAGC CUACCAGUUUC UUACUGCUCUA CU SEQ ID NO.: 33 | SEQ ID NO.: 12 | 6 |
| chikv5utr | 5'UTR | SEQ ID NO.: 33 | SEQ ID NO.: 13 | 7 |
| chikvRSE | 3' UTR (target disruption structure: Repetitive Sequence Element (RSE)) | AGCAAAUAAUC UAUAGAUCAAA GGGCUACGCAA SEQ ID NO.: 35 | SEQ ID NO.: 14 | 6 |
| Chikv_ re | Recoding Element (CDS) | GCUGCUGUAAA ACGUUGGCUUU UUUAGCCGUAA U SEQ ID NO.: 31 | SEQ ID NO.:39 | 6 |
| chikvRSE | 3' UTR (target disruption structure: RSE) | SEQ ID NO.: 35 | SEQ ID NO.:15 | 6 |

**Table 4. CircRNAs designed against CHIKV:** The different circRNAs designed against CHIKV are classified in the table with information of the target disruption structure, the sequence of the target hybridization region, the sequence of the whole circRNA and the number of hybridization regions present in each candidate (in each artificial circRNA). UTR: untranslated region CDS: coding sequence.

In a further preferred embodiment, the one or more target hybridization regions to which the at least one hybridization region of the artificial RNA according to the present invention, preferably of the artificial circular RNA of the present invention, completely hybridizes is comprised in SEQ ID NO. 20. In particular, the target hybridization region comprises the nucleotide sequence as defined in SEQ ID NO.: 37 or a nucleotide sequence with at least 70% identity to the nucleotide sequence as defined in SEQ ID NO.: 37, preferably with at least 80%, more preferably with at least 90%, even more preferably with at least 95% identity to the nucleotide sequence as defined in SEQ ID NO.: 37, or the homologe regions in another WNV strain/serotype.

**Table 5:**

| **Name of the artificial circular RNA** | **Target disruption structure comprised in** | **Target hybridization region** | **SEQ ID NO.:** | **Hybridization regions of the artificial RNA** |
|---|---|---|---|---|
| circ_wnv_slII_1 | 3' UTR (target disruption structure SL III) | UUUUGAGGAGA AAGUCAGGCCG GGAAGUU SEQ ID NO.: 37 | SEQ ID NO.:24 | 7 |
| circ wnv_siII_2 | 3' UTR (target disruption structure SL III) | UUUUGAGGAGA AAGUCAGGCCG GGAAGUU SEQ ID NO.: 37 | SEQ ID NO.: 19 | 7 |

**Table 5. CircRNAs designed against WNV:** The different circRNAs designed against WNV are classified in the table with information of the target disruption structures, the sequence of the target hybridization region, the sequence of the complete circRNA and the number of hybridization regions present in each candidate (in each artificial circRNA). SLIII: Stem Loop III. UTR: untranslated region.

In a further preferred embodiment, the one or more target hybridization regions to which the at least one hybridization region of the artificial RNA according to the present invention, preferably of the artificial circular RNA of the present invention, completely hybridizes is comprised in SEQ ID NO.: 20 and SEQ ID NO.: 7. In particular, the target hybridization region comprises the nucleotide sequence as defined in one or more of SEQ ID NO.: 30 and 37 or a nucleotide sequence with at least 70% identity to the nucleotide sequence as defined in SEQ ID NO.: 30 and 37, preferably with at least 80%, more preferably with at least 90%, even more preferably with at least 95% identity to the nucleotide sequence as defined in SEQ ID NO.: 30 and 37, or the homologe regions in another DENV/WNV strain/serotype.

**Table 6:**

| **Name of the artificial circular RNA** | **Target disruption structure comprised in** | **Target hybridization region** | **SEQ ID NO.:** | **Hybridization regions of the artificial RNA** |
|---|---|---|---|---|
| dchp wslI_A | CDS and 3' UTR (target disruption structures: cHP and SLIII) | SEQ ID NO.: 30 and 37 | SEQ ID NO.:21 | 6 |
| | | | SEQ ID NO: 63 | |
| dchp_wslI_B | CDS and 3' UTR (target disruption structures: cHP and SLIII) | SEQ ID NO.: 30 and 37 | SEQ ID NO.:22 | 6 |
| dchp_wslI_C | CDS and 3' UTR (target disruption structures: cHP and SLIII) | SEQ ID NO.: 30 and 37 | SEQ ID NO.:23 | 6 |

**Table 6. CircRNAs designed against WNV and DENV:** The different circRNAs designed against WNV and DENV are classified in the table with information of the target disruption structures, the sequence of the target hybridization region, the sequence of the complete circRNA and the number of hybridization regions present in each candidate (in each artificial circRNA). SLI: Stem Loop I. cHP: capsid region hairpin

In a further preferred embodiment, the one or more target hybridization regions to which the at least one hybridization region of the artificial RNA according to the present invention, preferably of the artificial circular RNA of the present invention, completely hybridizes is comprised in one or more of SEQ ID NO.: 1, SEQ ID NO.: 7, SEQ ID NO.: 11 and/or SEQ ID NO. 20. In particular, the target hybridization region comprises the nucleotide sequence as defined in one or more of SEQ ID NO.: 25 to 31, 33 and 35-37 or a nucleotide sequence with at least 70% identity to the nucleotide sequence as defined in SEQ ID NO.: 25 to 31, 33 and 35-37, preferably with at least 80%, more preferably with at least 90%, even more preferably with at least 95% identity to the nucleotide sequence as defined in SEQ ID NO.: 25 to 31, 33 and 35-37.

In another preferred embodiment, the target disruption structures in the HCV viral genome are found in the IRES and/or CDS region and/or a combination of them.

In another preferred embodiment, the target disruption structures in the Dengue viral genome are found in the 3'UTR and/or is the cHP and/or a combination of them.

In another preferred embodiment, the target disruption structures in the Zika viral genome are found in the 5'UTR and/or are the RSE and/or a combination of them.

In another preferred embodiment, the target disruption structures in the Chikungunya viral genome is found in the 5'UTR and/or is the RSE and/or a combination of them.

In another preferred embodiment, the target disruption structure in the West Nile viral genome is the stem-loop III (SLIII).

In another preferred embodiment, the hybridization regions of the artificial RNA of the present invention, which is preferably a circular RNA, target (i.e., are able to completely hybridize with target hybridization regions present in) more than one the target disruption structures present in one viral genome.

In a particular embodiment, the artificial RNA of the present invention, preferably the artificial circular RNA of the present invention, has a broad spectrum activity against a RNA viral genome, preferably against HCV, Dengue, Zika, Chikungunya, West Nile and Yellow Fever viral genome. In the present invention "a broad spectrum activity" related to the artificial RNA, preferably the circular RNA of the present invention means that said artificial RNA is effective against a wide range of RNA viruses, preferably against HCV, Dengue, Zika, Chikungunya, West Nile and Yellow Fever.

In a further preferred embodiment, the one or more target hybridization regions to which the at least one hybridization region of the artificial RNA according to the present invention, preferably of the artificial circular RNA of the present invention, completely hybridizes is comprised in SEQ ID NO. 34. In particular, the target hybridization region comprises the nucleotide sequence as defined in one or more of SEQ ID NO.: 58-62 or a nucleotide sequence with at least 70% identity to the nucleotide sequence as defined in SEQ ID NO.: 58-62, preferably with at least 80%, more preferably with at least 90%, even more preferably with at least 95% identity to the nucleotide sequence as defined in SEQ ID NO.: 58-62, or the homologe regions in another SARS-CoV-2 strain/serotype.

**Table 7:**

| **Name of the artificial circular RNA** | **Target disruption structure comprised in** | **Target hybridization region** | **SEQ ID NO.:** | **Hybridization regions of the artificial RNA** |
|---|---|---|---|---|
| Artificial circular ARN 1; target disruption structure comprised in SARS-CoV-2 3'UTR replication site | 3'UTR replication site | CAGAAUGAAU UCUCGUAACU ACAUAGCACA AGUAG SEQ ID NO.: 62 | SEQ ID NO.:36 | 6 |
| Artificial circular ARN 2; target disruption structure comprised in SARS-CoV-2 3'UTR | 3'UTR replication site | CAGAAUGAAU UCUCGUAACU ACAUAGCACA AGUAG SEQ ID NO.: 62 | SEQ ID NO.:38 | 6 |
| Artificial circular RNA 3; target disruption structure comprised in SARS-CoV-2 3'UTR replication site | 3'UTR replication site | CAGAAUGAAU UCUCGUAACU ACAUAGCACA AGUAG SEQ ID NO.: 62 | SEQ ID NO.:40 | 6 |
| Artificial circular RNA 4; target disruption structure comprised in SARS-CoV-2 3'UTR replication site | 3'UTR replication site | CAGAAUGAAU UCUCGUAACU ACAUAGCACA AGUAG SEQ ID NO.: 62 | SEQ ID NO.:41 | 6 |
| Artificial circular RNA 5; target disruption structure comprised in SARS-CoV-2 3'UTR replication site | 3'UTR replication site | CAGAAUGAAU UCUCGUAACU ACAUAGCACA AGUAG SEQ ID NO.: 62 | SEQ ID NO.:42 | 6 |
| Artificial circular RNA 1; target disruption structure comprised in SARS-CoV-2 5'UTR (SIII) | 5'UTR (target disruption structure: SIII) | AACCAACUUU CGAUCUCUUG UAGAUCU SEQ ID NO.: 58 | SEQ ID NO.:43 | 7 |
| Artificial circular RNA 2; target disruption structure comprised in SARS-CoV-2 5'UTR (SIII) | 5'UTR (target disruption structure: SIII) | AACCAACUUU CGAUCUCUUG UAGAUCU SEQ ID NO.: 58 | SEQ ID NO.:44 | 7 |
| Artificial circular RNA 3; target disruption structure comprised in SARS-CoV-2 5'UTR (SIII) | 5'UTR (target disruption structure: SIII) | AACCAACUUU CGAUCUCUUG UAGAUCU SEQ ID NO.: 58 | SEQ ID NO.:45 | 7 |
| Artificial circular RNA 1; target hybridization region: SARS-CoV-2 Target A | Target hybridization region: SARS-CoV-2 Target A | UUUAAGUUUA GAAUAGACGG UGACAUGGUA CCA SEQ ID NO.: 59 | SEQ ID NO.:46 | 6 |
| Artificial circular RNA 2; target hybridization region: SARS-CoV-2 Target A | Target hybridization region: SARS-CoV-2 Target A | UUUAAGUUUA GAAUAGACGG UGACAUGGUA CCA SEQ ID NO.: 59 | SEQ ID NO.:47 | 6 |
| Artificial circular RNA 3; target hybridization region: SARS-CoV-2 Target A | Target hybridization region: SARS-CoV-2 Target A | UUUAAGUUUA GAAUAGACGG UGACAUGGUA CCA SEQ ID NO.: 59 | SEQ ID NO.:48 | 6 |
| Artificial circular RNA 4; target hybridization region: SARS-CoV-2 Target A | Target hybridization region: SARS-CoV-2 Target A | UUUAAGUUUA GAAUAGACGG UGACAUGGUA CCA SEQ ID NO.: 59 | SEQ ID NO.:49 | 6 |
| Artificial circular RNA 1; target hybridization region: SARS-CoV-2 Target C | Target hybridization region: SARS-CoV-2 Target C | UCACUAAGAA AUCUGCUGCU GAGGCUUCUA | SEQ ID NO.:50 | 7 |
| | | SEQ ID NO.: 60 | | |
| Artificial circular RNA 2; target hybridization region: SARS-CoV-2 Target C | Target hybridization region: SARS-CoV-2 Target C | UCACUAAGAA AUCUGCUGCU GAGGCUUCUA SEQ ID NO.: 60 | SEQ ID NO.:51 | 7 |
| Artificial circular RNA 3; target hybridization region: SARS-CoV-2 Target C | Target hybridization region: SARS-CoV-2 Target C | UCACUAAGAA AUCUGCUGCU GAGGCUUCUA SEQ ID NO.: 60 | SEQ ID NO.:52 | 7 |
| Artificial circular RNA 4; target hybridization region: SARS-CoV-2 Target C | Target hybridization region: SARS-CoV-2 Target C | UCACUAAGAA AUCUGCUGCU GAGGCUUCUA SEQ ID NO.: 60 | SEQ ID NO.:53 | 7 |
| Artificial circular RNA 1; target hybridization region: SARS-CoV-2 Target D | Target hybridization region: SARS-CoV-2 Target D | UCGUCUAUCU UCUGCAGGCU GCUUACGGUU U SEQ ID NO.: 61 | SEQ ID NO.:54 | 6 |
| Artificial circular RNA 2; target hybridization region: SARS-CoV-2 Target D | Target hybridization region: SARS-CoV-2 Target D | UCGUCUAUCU UCUGCAGGCU GCUUACGGUU U SEQ ID NO.: 61 | SEQ ID NO.:55 | 6 |
| Artificial circular RNA 3; target hybridization region: SARS-CoV-2 Target D | Target hybridization region: SARS-CoV-2 Target D | UCGUCUAUCU UCUGCAGGCU GCUUACGGUU U SEQ ID NO.: 61 | SEQ ID NO.:56 | 6 |
| Artificial circular RNA 4; target hybridization region: SARS-CoV-2 Target D | Target hybridization region: SARS-CoV-2 Target D | UCGUCUAUCU UCUGCAGGCU GCUUACGGUU | SEQ ID NO.:57 | 6 |
| | | U SEQ ID NO.: 61 | | |

**Table 7. CircRNAs designed against severe acute respiratory syndrome coronavirus 2 (SARS-CoV-2) isolate Wuhan-Hu-1 (complete genome: SEQ ID NO.: 34):** The different circRNAs designed against SARS-CoV-2 are classified in the table with information of the target disruption structures, the sequence of the target hybridization region, the sequence of the complete circRNA and the number of hybridization regions present in each candidate (in each artificial circRNA).

In a **second aspect**, the present invention provides a composition comprising the artificial RNA of the present invention, in any of the embodiments, alone or in combination, disclosed herein.

Preferably, the composition is a pharmaceutical composition, and preferably comprises the artificial RNA of the present invention, in any of the embodiments, and one or more pharmaceutically acceptable carriers.

Where clinical applications are contemplated, pharmaceutical compositions will be prepared in a form appropriate for the intended application. Generally, this will entail preparing compositions that are essentially free of pyrogens, as well as other impurities that could be harmful to humans or animals.

Colloidal dispersion systems, such as macromolecule complexes, nanocapsules, microspheres, beads, and lipid-based systems including oil-in-water emulsions, micelles, mixed micelles, and liposomes, may be used as delivery vehicles for artificial RNAs, such as circular RNAs. Commercially available fat emulsions that are suitable for delivering the nucleic acids of the disclosure to tissues include Intralipid, Liposyn, Liposyn II, Liposyn III, Nutrilipid, and other similar lipid emulsions. A colloidal system for use as a delivery vehicle in vivo is a liposome (i.e., an artificial membrane vesicle). The preparation and use of such systems is well known in the art. Exemplary formulations are also disclosed in U.S. Pat. No. 5,981,505; U.S. Pat. No. 6,217,900; U.S. Pat. No. 6,383,512; U.S. Pat. No. 5,783,565; U.S. Pat. No. 7,202,227; U.S. Pat. No. 6,379,965; U.S. Pat. No. 6,127,170; U.S. Pat. No. 5,837,533; U.S. Pat. No. 6,747,014; and WO 03/093449. In particular, cationic liposome formulations comprising lipofectamine can be used for delivery. Lipofectamine may be formulated with a neutral co-lipid or helper lipid. See e.g., U. S. Patent No. 7,479,573, Dalby et al. (2004) Science Direct, Methods 33:95-103, Hawley-Nelson et al. (1993) Focus 15 :73-79.

One will generally desire to employ appropriate salts and buffers to render delivery vehicles stable and allow for uptake by target cells. Buffers also will be employed when recombinant cells (e.g., transfected *ex vivo* with an artificial RNA, such as a circular RNA) are introduced into a patient. Aqueous compositions of the present disclosure comprise an effective amount of the delivery vehicle, dissolved or dispersed in a pharmaceutically acceptable carrier or aqueous medium.

The phrases "pharmaceutically acceptable" or "pharmacologically acceptable" refers to molecular entities and compositions that do not produce adverse, allergic, or other untoward reactions when administered to an animal or a human. As used herein, "pharmaceutically acceptable carrier" includes solvents, buffers, solutions, dispersion media, coatings, antibacterial and antifungal agents, isotonic and absorption delaying agents and the like acceptable for use in formulating pharmaceuticals, such as pharmaceuticals suitable for administration to humans. The use of such media and agents for pharmaceutically active substances is well known in the art.

Except insofar as any conventional media or agent is incompatible with the active ingredients of the present disclosure, its use in therapeutic compositions is contemplated. Supplementary active ingredients also can be incorporated into the compositions, provided they do not inactivate the nucleic acids of the compositions.

The pharmaceutical forms suitable for injectable use or catheter delivery include, for example, sterile aqueous solutions or dispersions and sterile powders for the extemporaneous preparation of sterile injectable solutions or dispersions. Generally, these preparations are sterile and fluid to the extent that easy injectability exists. Preparations should be stable under the conditions of manufacture and storage and should be preserved against the contaminating action of microorganisms, such as bacteria and fungi. Appropriate solvents or dispersion media may contain, for example, water, ethanol, polyol (for example, glycerol, propylene glycol, and liquid polyethylene glycol, and the like), suitable mixtures thereof, and vegetable oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions may be prepared by incorporating the active compounds in an appropriate amount into a solvent along with any other ingredients (for example as enumerated above) as desired, followed by filtered sterilization. Generally, dispersions are prepared by incorporating the various sterilized active ingredients into a sterile vehicle which contains the basic dispersion medium and the desired other ingredients, e.g., as enumerated above. In the case of sterile powders for the preparation of sterile injectable solutions, the preferred methods of preparation include vacuum drying and freeze-drying techniques which yield a powder of the active ingredient(s) plus any additional desired ingredient from a previously sterile-filtered solution thereof.

The compositions of the present invention generally may be formulated in a neutral or salt form. Pharmaceutically-acceptable salts include, for example, acid addition salts (formed with the free amino groups of the protein) derived from inorganic acids (e.g., hydrochloric or phosphoric acids, or from organic acids (e.g., acetic, oxalic, tartaric, mandelic, and the like). Salts formed with the free carboxyl groups of the protein can also be derived from inorganic bases (e.g., sodium, potassium, ammonium, calcium, or ferric hydroxides) or from organic bases (e.g., isopropylamine, trimethylamine, histidine, procaine and the like).

Upon formulation, solutions are preferably administered in a manner compatible with the dosage formulation and in such amount as is therapeutically effective. The formulations may easily be administered in a variety of dosage forms such as injectable solutions, drug release capsules and the like. For parenteral administration in an aqueous solution, for example, the solution generally is suitably buffered and the liquid diluent first rendered isotonic for example with sufficient saline or glucose. Such aqueous solutions may be used, for example, for intravenous, intramuscular, subcutaneous and intraperitoneal administration. Preferably, sterile aqueous media are employed as is known to those of skill in the art, particularly in light of the present disclosure. By way of illustration, a single dose may be dissolved in 1 ml of isotonic NaCl solution and either added to 1000 ml of hypodermoclysis fluid or injected at the proposed site of infusion, (see for example, "Remington's Pharmaceutical Sciences" 15th Edition, pages 1035-1038 and 1570-1580). Some variation in dosage will necessarily occur depending on the condition of the subject being treated. The person responsible for administration will, in any event, determine the appropriate dose for the individual subject. Moreover, for human administration, preparations should meet sterility, pyrogenicity, and general safety and purity standards as required by FDA Office of Biologies standards.

The pharmaceutical compositions of the present invention can also be housed in a syringe, an implantation device, or the like, depending upon the intended mode of delivery and use. Preferably, the compositions comprising artificial RNAs, such as artificial circular RNAs, prepared as described herein, are in unit dosage form, meaning an amount of a composition appropriate for a single dose, in a premeasured or pre-packaged form.

As for the administration, at least one therapeutically effective cycle of treatment with an artificial RNA, such as artificial circular RNAs, may be administered to a subject for treatment of a viral infection caused by, e.g., HCV, Dengue virus, Zika virus, Chikungunya virus, West Nile virus, Yellow Fever virus or coronavirus, such as SARS and/or MERS, preferably SARS-CoV-2.

By "therapeutically effective dose or amount" of a composition comprising an artificial RNA, such as artificial circular RNAs, is intended an amount that, when administered as described herein, brings about a positive therapeutic response, such as improved recovery from the treated condition, such as for example the viral infection, the cancer or the genetic disorder.

Multiple therapeutically effective doses of compositions comprising artificial RNAs of the present invention, such as artificial circular RNAs of the present invention, and/or one or more other therapeutic agents, will be administered. The compositions of the present invention are typically, although not necessarily, administered via injection (subcutaneously, intravenously, intra-arterially, or intramuscularly), by infusion, or locally. Additional modes of administration are also contemplated, such as intraperitoneal, intrathecal, intratumor, intralymphatic, intravascular, intralesion, transdermal, and so forth. In some embodiments, the pharmaceutical composition comprising the artificial RNA of the present invention, , such as artificial circular RNAs, is administered locally. The pharmaceutical compositions comprising the artificial RNAs of the present invention, such as artificial circular RNAs, and other agents may be administered using the same or different routes of administration in accordance with any medically acceptable method known in the art.

The pharmaceutical compositions comprising the artificial RNAs of the present invention, such as artificial circular RNAs, may also be administered prophylactically, e.g., to prevent the viral infection and/or cancer and/or genetic disorder.

The pharmaceutical compositions comprising the artificial RNAs of the present invention, such as artificial circular RNAs, and/or other agents are in a sustained-release formulation, or a formulation that is administered using a sustained-release device. Such devices are well known in the art, and include, for example, miniature implantable pumps that can provide for delivery over time in a continuous, steady-state fashion at a variety of doses to achieve a sustained-release effect with a non-sustained-release pharmaceutical composition.

Those of ordinary skill in the art will appreciate which conditions compositions comprising artificial RNAs of the present invention can effectively treat and/or prevent. The actual dose to be administered will vary depending upon the age, weight, and general condition of the subject as well as the severity of the condition being treated, the judgment of the health care professional, and conjugate being administered.

Therapeutically effective amounts can be determined by those skilled in the art, and are adjusted to the particular requirements of each particular case. Compositions comprising the artificial RNAs of the present invention, such as artificial circular RNAs, can be administered alone or in combination with one or more other therapeutic agents. The specific dosing schedule will be known by those of ordinary skill in the art or can be determined experimentally using routine methods. Exemplary dosing schedules include, without limitation, administration five times a day, four times a day, three times a day, twice daily, once daily, three times weekly, twice weekly, once weekly, twice monthly, once monthly, and any combination thereof. Preferred compositions are those requiring dosing no more than once a day.

Compositions comprising the artificial RNAs of the present invention, such as artificial circular RNAs, can be administered prior to, concurrent with, or subsequent to other agents. If provided at the same time as other agents, the artificial RNAs of the present invention can be provided in the same or in a different composition. Thus, artificial RNAs and one or more other agents can be presented to the individual by way of concurrent therapy.

By "concurrent therapy" is intended administration to a subject such that the therapeutic effect of the combination of the substances is caused in the subject undergoing therapy. For example, concurrent therapy may be achieved by administering a dose of a pharmaceutical composition comprising an artificial RNA according to the present invention, such as artificial circular RNAs, and a dose of a pharmaceutical composition comprising at least one other agent, which in combination comprise a therapeutically effective dose, according to a particular dosing regimen. Similarly, an artificial RNA of the present invention and one or more other therapeutic agents can be administered in at least one therapeutic dose.

Administration of the separate pharmaceutical compositions can be performed simultaneously or at different times (i.e., sequentially, in either order, on the same day, or on different days), as long as the therapeutic effect of the combination of these substances is caused in the subject undergoing therapy.

In a **third aspect,** the present invention provides a kit comprising the artificial RNA of the present invention, in any of its embodiments, or comprising the composition of the present invention, and instructions for using the artificial RNA or composition.

Hence, any of the artificial RNAs and/or compositions described herein may be included in a kit. For example circular RNAs as disclosed herein may be included in a kit. The kit may also include one or more transfection reagents to facilitate delivery of the artificial RNAs to cells. Such kits may also include components that preserve the polynucleotides or that protect against their degradation. Such components may be RNAse-free or protect against RNAses.

Such kits generally will comprise, in suitable means, distinct containers for each individual reagent or solution. The kit may comprise one or more containers holding the artificial RNAs and other agents. Suitable containers for the compositions include, for example, bottles, vials, syringes, and test tubes. Containers can be formed from a variety of materials, including glass or plastic. A container may have a sterile access port (for example, the container may be a vial having a stopper pierceable by a hypodermic injection needle).

The kit can further comprise a container comprising a pharmaceutically acceptable buffer, such as phosphate-buffered saline, Ringer's solution, or dextrose solution. It can also contain other materials useful to the end-user, including other pharmaceutically acceptable formulating solutions such as buffers, diluents, filters, needles, and syringes or other delivery devices. The delivery device may be pre-filled with the compositions.

The kit can also comprise a package insert containing written instructions for methods of treating the viral infections with the artificial RNAs of the present invention. The package insert can be an unapproved draft package insert or can be a package insert approved by the Food and Drug Administration (FDA) or other regulatory body.

As described above, preferably, the artificial RNA of the present invention, in any of its embodiments, which may be comprised in the composition or kit of the present invention, comprises the nucleotide sequence as defined in SEQ ID NO: 2, 3, 4, 5, 6 (for HCV); 8, 9, 10 (for Dengue virus); 12, 13, 14, 15, 39 (for Chikungunya virus); 16 and 17 (Broadspectrum activity for both HCV and Dengue Virus); 24 and 19 (for West Nile Virus); 21, 22 and 23 (Broad spectrum activity for both Dengue and West Nile Viruses); 32 (Broad spectrum activity for both HCV and Dengue Virus). These are the target hybridization regions which completely hybridize with the hybridization regions comprised in the artificial RNAs described in the examples below.

Preferably, the the one or more target hybridization regions which completely hybridize with the at least one hybridization region comprised in the artificial RNA of the present invention, in any of its embodiments, which may also be comprised in the composition and/or kit of the present invention is comprised in SEQ ID NO.: 1, SEQ ID NO.: 7, SEQ ID NO.: 11, SEQ ID NO.: 20 and/or SEQ ID NO.: 34.

In a **fourth aspect,** the present invention provides the artificial RNA of the present invention, in any of its embodiments, or the composition or the kit of the present invention for use as a medicament. Preferably, the present invention provides the artificial RNA of the present invention, in any of its embodiments, or the composition or the kit of the present invention for use in a method of preventing and/or treating a viral infection. Preferably, the artificial RNA of the present invention has a broad spectrum activity against two or more RNA viruses.

In a further embodiment, the present invention provides the artificial RNA of the present invention, in any of its embodiments, or the composition or the kit of the present invention for use in a method of preventing and/or treating cancer.

In a further embodiment, the present invention provides the artificial RNA of the present invention, in any of its embodiments, or the composition or the kit of the present invention for use in a method of preventing and/or treating genetic disorders.

In the context of the present invention, a genetic disorder refers to a health problem caused by one or more abnormalities in the genome. It can be caused by a mutation in a single gene (monogenic) or multiple genes (polygenic) or by a chromosomal abnormality. Examples of genetic disorders are the following: Familial hypercholesterolemia, Polycystic kidney disease, Neurofibromatosis type I, Hereditary spherocytosis, Marfan syndrome, Huntington's disease, Sickle cell anaemia, Cystic fibrosis, Tay-Sachs disease, Phenylketonuria, Mucopolysaccharidoses, Lysosomal acid lipase deficiency, Glycogen storage diseases, Galactosemia, Duchenne muscular dystrophy or Hemophilia.

Hence, in the fourth aspect, the present invention provides the use of the artificial RNA of the present invention, in any of its embodiments, or the composition or the kit of the present invention for the manufacture of a medicament for preventing and/or treating a viral infection and/or cancer and/or a genetic disorder.

In a preferred embodiment, the viral infection is caused by HCV, Dengue, Zika, Chikungunya, West Nile, Yellow Fever virus or coronavirus, such as SARS and/or MERS, preferably SARS-CoV-2.

As it is well known in the art (see for example WO 2017/222911), any known method of nucleic acid delivery may be used for administration of the artificial RNAs as disclosed herein (e.g., immunogenic or non-immunogenic) to a subject. For example, an artificial RNA may be administered by transfection *in vivo.* Alternatively, the artificial RNA may be administered by transfection *ex vivo,* and subsequent transfer of a cell transfected with the artificial RNA to the subject.

An artificial RNA, such as a circular RNA, can be delivered with a nucleic acid carrier (e.g. cationic carrier) or a nanoparticle (e.g., lipid nanoparticle, polymeric nanoparticle, nanoparticle comprising a combination of polymers and peptides, or an electrostatic complex). The artificial RNA may be delivered to a subject using a recombinant virus, an exosome, liposome, or other lipid vesicle, or a cell engineered to secrete an artificial RNA, such as a circular RNA.

The artificial RNA, such as a circular RNA, can also be conjugated to a targeting ligand (e.g., small molecule, peptide or protein) for localized delivery to a particular site (e.g., cells, tissue, or organ) in the subject. The artificial RNA can be even linked to an internalization sequence, a protein transduction domain, or a cell penetrating peptide to facilitate entry into a cell.

In a **fifth aspect,** the present invention provides a method for producing the artificial RNA of the invention, preferably the artificial circular RNA of the present invention, wherein in the method comprises the step of:
a) synthesizing the artificial RNA of the invention;
b) screening the artificial RNA of step a) for artificial RNAs which are capable of disrupting the structure one or more target disruption structures of one or more RNA fragments, preferably which are able of disrupting the SRVVLC and/or rendering the virus less capable (ideally incapable) of performing essential functions of its life cycle, i.e., artificial RNAs which are capable of yielding conformational changes in the target RNA fragment, e.g., yielding conformational changes in the RNA of the virus which impede essential functions of the life cycle of the virus.

### Production of circRNAs

Artificial RNAs, such as circRNAs, are designed using an in-house software tool. This tool allows for several design constraints to be taken into account, for instance GC content range of the sequence. Once the artificial RNAs, such as circRNAs RNAs, are designed, they can be produced intracellularly or by *in vitro* transcription.

### (1) Intracellular production of artificial RNAs, such as circRNAs.

The corresponding artificial RNAs sequence, such as circRNAs sequence, in DNA form is commercially obtained and cloned to be later transformed into cells using the following steps.

First, the designed artificial RNA sequence is cloned into a plasmid (por example a pcDNA3-CIRS7 plasmid) between two sequences that mediate splicing and circularization, SA (splicing acceptor) and SD (splicing donor) (clone gently provided by Dr. Thomas B. Hansen from the University of Aarhus (Denmark) (Patent application: WO 2014/082644 A1). This plasmid contains a gene conferring resistance to the antibiotic ampicillin. The derived plasmid is then transformed into XL1-Blue competent cells (Sigma). The transformed cells are grown in LB media with ampicillin (LB-Amp) for selection. One of the selected colonies is then grown in LB-Amp liquid medium for 36h for amplification. After this time, the culture is centrifuged to obtain the bacteria cells and the plasmid purified using NucleoSpin^{®} Plasmid kit (Macherey-Nagel).

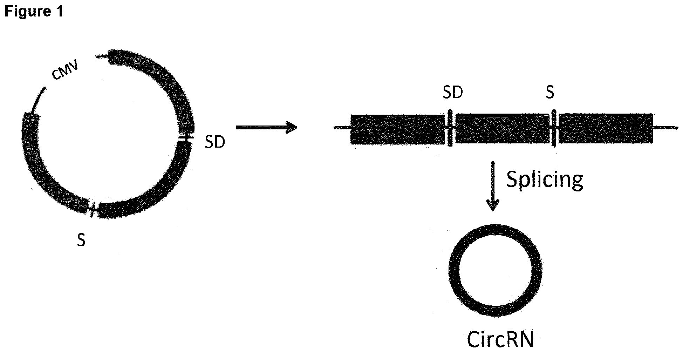

When these derived plasmids are introduced into human cells, the transcription promoter CMV is recognized by the cellular transcription machinery and a mRNA is expressed using the host transcription machinery. For the production of circRNAs, the resultant RNA is circularized by the host splicing machinery generating the designed circRNAs (see figure 1).

### In vitro production of artificial circRNAs.

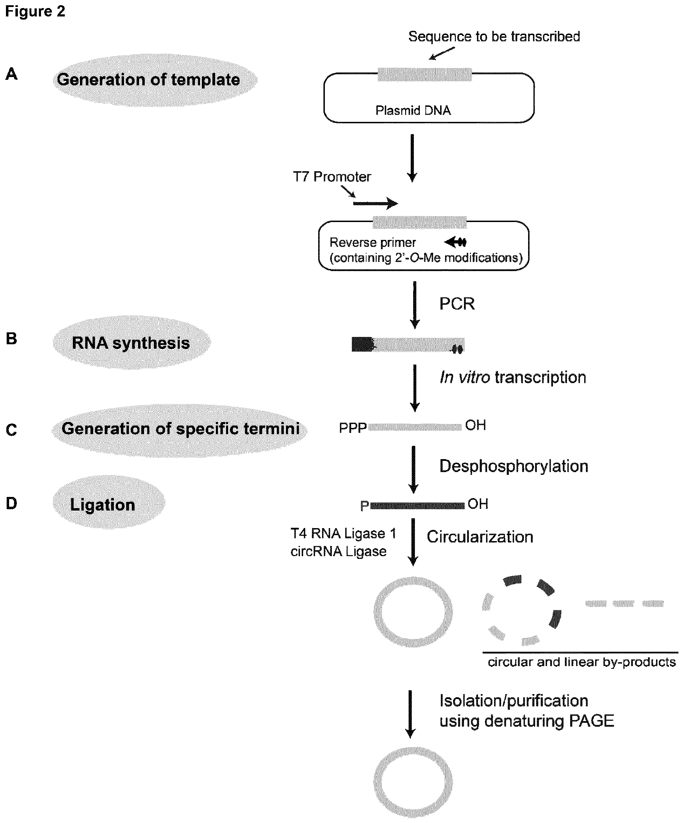

The production of circRNAs can be also achieved *in vitro* by obtaining the corresponding linear RNA, through chemical synthesis or *in vitro* transcription, and then circularize it with RNA ligases (see figure 2).

A more efficient approach is to produce the circRNAs in vitro from T7 promoter-flanked templates. This plasmid contains self-cleavage ribozymes that after the in vitro transcription will be cleaved generating the ends that will allow the circularization and generation of circRNAs. For each candidate, the T7 promoter plasmid was linearized using HindIII (FastDigest) during 30 min at 37°C. The purified linearized molecule was in vitro transcribed and purified from a polyacrylamide gel. Afterwards, the RNAs will be circularized using RtcB (NEB) and the circularized molecules were selected after RNAse R (Lucigen) treatment during 30' at 37°C (See figure 25).

### Mode of action

The artificial RNAs, such as circRNAs, are designed so that they hybridize with selected regions (target hybridization regions) present in target disruption structures of one or more RNA fragments, such as an RNA viral genome. These target disruption structures are relevant to the functionality of the RNA fragments, as described above. For instance, if the RNA fragment is RNA viral genome, the target disruption structures may be relevant for multiple functions of the viral RNA and infectivity such as translation, replication, localization or/and encapsidation. These signals can be found in the 5' and 3'untranslated regions and in the coding sequence region (CDS) of the viral RNA genomes. In one embodiment, these target disruption structures can be located within the IRES element (Internal Ribosomal Entry Site), which is responsible for sequestering the host ribosomes to initiate translation of the viral proteins for some viral RNA genomes.

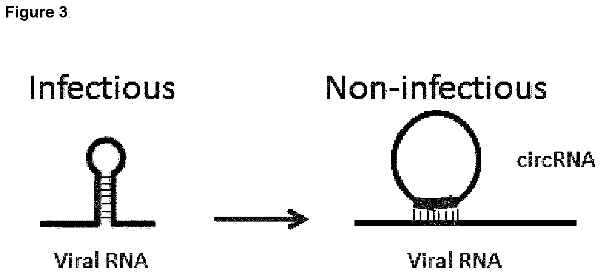

The mechanism of action is **NOT** just the hybridization between the hybridization regions of the artificial RNA and the target hybridization regions of the target disruption structures, in contrast to the state of the art, but the structural changes triggered upon this hybridization will alter the secondary structure of the RNA fragment, ultimately affecting its function. For instance, viral RNA genomes and viral mRNAs contain highly structured regions essential for their function. Binding of the designed artificial RNAs to target disruption structures in the viral RNA genome will lead to the unfolding of the structure (changes in the secondary structure) and consequently to the reduction or even inhibition of the infectivity (see, e.g., figure 3).

For instance, the disruption of the target disruption structures in the RNA viral genome can disturb the accessibility of RNA binding proteins (RBP) to their target viral RNA sequences. Note that this strategy differs from that whose aim is hybridizing the exact binding site of an RBP (see, e.g., figure 4).

Sometimes, RBPs bind single stranded RNA regions that require to be found in a specific structural context, for instance, immediately 3' of a stem loop (see, e.g., figure 4, left side). In our case, hybridizing with a different target hybridization region might disrupt this stem loop (which is part of a target disruption structure) and, although the binding site remains accessible, the structural change renders RBP binding ineffective (see, e.g., figure 4, right side).

### Structure

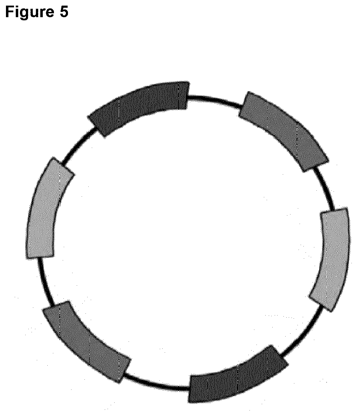

The structure of the designed artificial RNAs, such as circRNAs, is very flexible. Generally, it contains at least one, preferably several hybridization regions that hybridize with at least one, preferably several target hybridization regions present in at least one, preferably several target disruption structures of one or more RNA fragments, preferably in the viral genome. These preferably several hybridization regions in the artificial RNA are preferably separated by sequences that are quasi-random, in the sense that they do not perform any functions other than separate the preferably several hybridization regions, and to allow for the observance of the design constraints (low secondary structure, specific GC content, etc.). For instance, an example of an artificial RNA, which is a circRNA, that targets 3 different target disruption structures of the viral genome with 2 hybridization regions per target disruption structure is depicted in figure 5. Regions with the same color represent the fact that they target the same viral target disruption structure.

However, as explained above, these preferably several hybridization regions of the artificial RNA, such as circRNA, that target the same target hybridization region within a certain target disruption structure are preferably designed to be different from each other. This is possible since RNA allows for three different base-pairing types: G-C, A-U and G-U. This fact also entails that, e.g., viral escape (through mutations) from the artificial RNAs is more difficult since it will need to escape all the different hybridization regions at the same time. Note that the hybridization requirement is different from the anti-sense or complementarity requirement.

It is also possible to design the hybridization regions of a single artificial RNA in order to target different regions of different RNA fragments, such as different regions of different RNA viruses to obtain broad-spectrum artificial RNAs.

The present invention is now further illustrated by reference to the following examples which do not intend to limit the scope of the present invention.

### EXAMPLES

### Example 1:

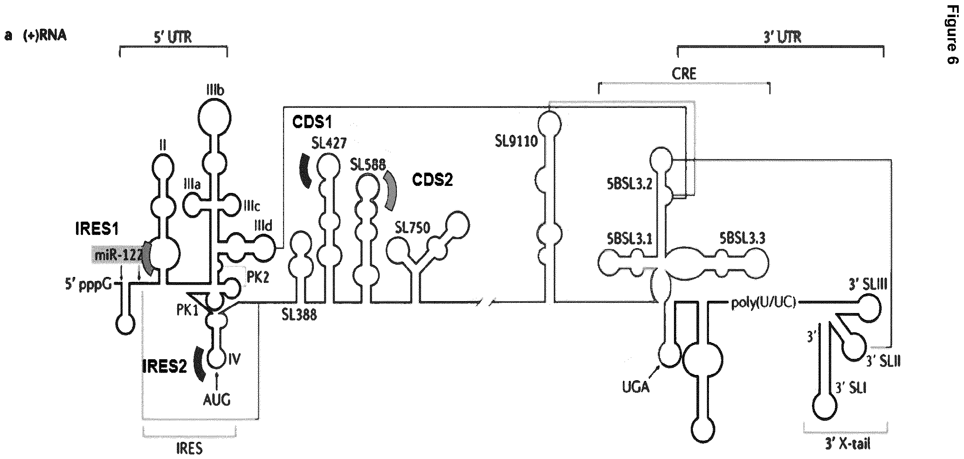

We have designed and tested four circRNAs (circ_hcv_ires1 (SEQ ID NO.: 2), circ_hcv_cds1 (SEQ ID NO.: 4), circ_hcv_cds2 (SEQ ID NO.: 6) y circ_hcv_combo1 (SEQ ID NO.: 5) against two different regions of HCV genome (see SEQ ID NO.: 1) as previously disclosed in the section Production of circRNAs. Circ_hcv_ires1 contains 7 hybridization sites of length 33 nucleotides that target a sequence in the IRES element. Circ_hcv_cds1 contains 8 hybridization sites of length 28 nucleotides that target a sequence in the coding region. Circ_hcv_cds2 contains 12 hybridization sites that target a sequence in the coding region. Circ_hcv_combo1 contains 3 hybridization sites per target region (IRES1 (SEQ ID NO.: 25), IRES2 (SEQ ID NO.: 26) and cHP (CDS1) (SEQ ID NO.: 27)). The target regions in the HCV genome are depicted in Figure 6 and SEQ ID NO.: 1.

### Cell cultures and transfection

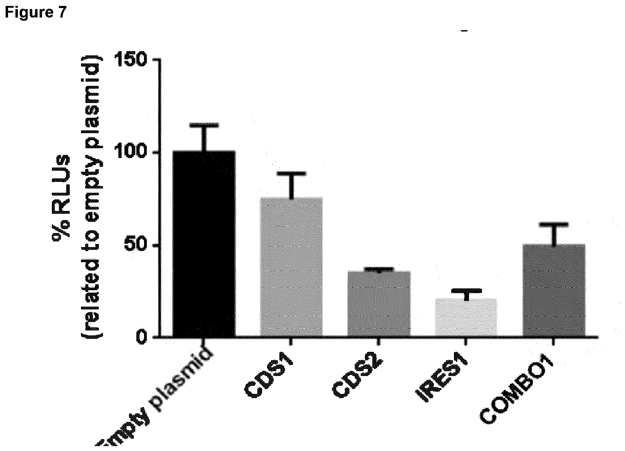

We transfected each circRNA in Huh7 cells and 24h post-transfection we infected the cells with an HCV derivative that expresses luciferase. After 48 hours post-infection, we measured the luciferase levels and compared the values to a control (mock transfected). We carried out five biological replicates for each condition. All circRNAs tested inhibit HCV infection by a significant amount. As shown in figure 7 all circRNAs designed are capable of lowering the infection with different efficiencies that range between 30% and 70% with respect to the control.

### Example 2:

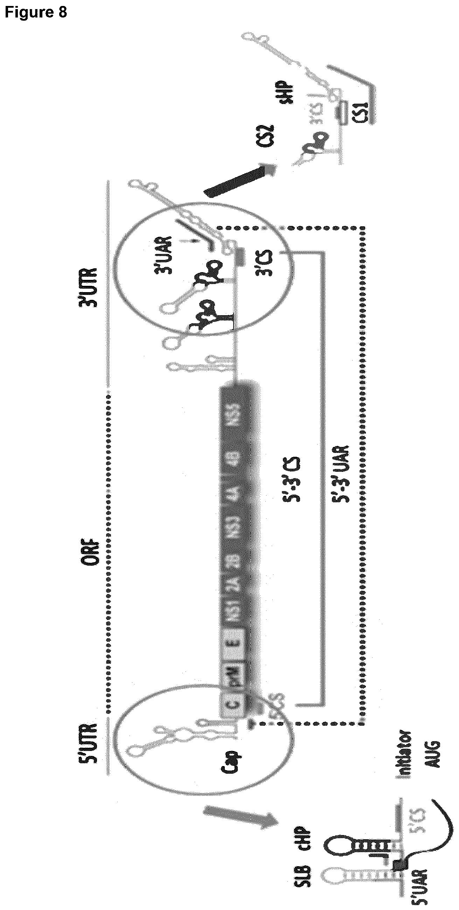

We have designed and tested three circRNAs (circ_dv_3utr (SEQ ID NO.: 8), circ_dv_cHP_v1 (SEQ ID NO.: 9) and circ_dv_cHP_v2 (SEQ ID NO.: 10)) against two different regions of DENV (Dengue) genome (SEQ ID NO.: 7) as previously disclosed in the section Production of circRNAs. Plasmid to generate DENV (pFK-DVs-R2A) carrying the Renilla luciferase reporter gene has been previously described (Scaturro, P. et al. Characterization of the mode of action of a potent dengue virus capsid inhibitor. J. Virol. 88, 11540-55 (2014)). All three circRNAs contain 7 hybridization regions that target the corresponding regions in the DENV genome (See figure 8 and SEQ ID NO.: 7).

### Cell cultures and transfection

The human embryonic kidney cell line HEK293 was maintained in Dulbecco's modified Eagle's medium (DMEM, Invitrogen, Carlsbad, CA) supplemented with 10% heat inactivated fetal bovine serum (FBS) and 10% non-essential amino acids. Cells were grown in an incubator with 5% CO2 at 37 °C. 1 10⁵ HEK293 cells/well were seeded in 24-well plates the day before transfection. 2 micrograms of each plasmid containing the cirRNAs or the empty plasmid were transfected using Lipofectamine 2000 (Invitrogen) following manufacturer's instructions. After overnight incubation, DMEM medium was removed and cells were washed with 1X PBS. Cells were inoculated with Dengue Virus for 4h at 37 °C. Finally, the virus containing media was replaced with fresh media. Forty-eight hours post infection, Luciferase activity was assayed. Cells were washed with 1X PBS, lysed in 150 µl of Renilla lysis buffer and frozen. Upon thawing, lysates were resuspended by pipetting. 4 µl of the lysates were mixed with 20 µl of Renila Luciferase Assay Buffer and 1/200 of substrate from the Renilla Luciferase assay system (Promega) and measured immediately in a luminometer for 2 s. Mean relative light units (RLU) were plotted as percentage relative to control infections (cells transfected with the circoVIR plasmid).

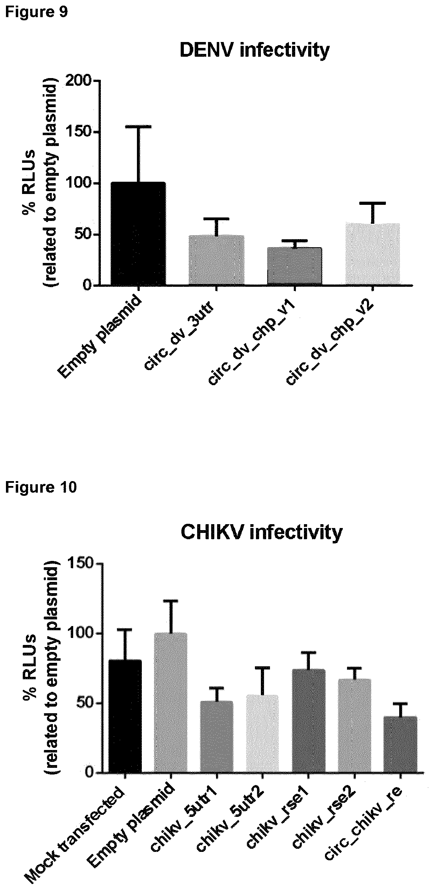

Results are shown in Figure 9 and depict the significant inhibition of DENV infection with respect to the control (empty plasmid).

### Example 3:

We have designed and tested five circRNAs (chikv_5utr1 (SEQ ID NO.: 12), chikv_5utr2 (SEQ ID NO.: 13), chikv_RSE1 (SEQ ID NO.: 14), chikv_RSE2 (SEQ ID NO.: 15) and chikv_RE (SEQ ID NO.: 39) against three different regions of CHIKV (Chikungunya) genome (SEQ ID NO.: 11) as previously disclosed in the section Production of circRNAs. The plasmid to generate CHIKV carrying the reporter gene of Gaussian luciferase (kindly provided by Dr. Merits, University of Tartu, Estonia) is based directly on the viral sequence isolated from a human patient from La Reunion (isolate LR2006_OPY1 (DQ443544). All five circRNAs contain 6 hybridization regions that target the corresponding regions in the CHIKV genome (SEQ ID NO.: 11).

### Cell cultures and transfection

The human embryonic kidney cell line HEK293 was maintained in Dulbecco's modified Eagle's medium (DMEM, Invitrogen, Carlsbad, CA) supplemented with 10% heat inactivated fetal bovine serum (FBS) and 10% non-essential amino acids. Cells were grown in an incubator with 5% CO2 at 37 °C. 1 10⁵ HEK293 cells/well were seeded in 24-well plates the day before transfection. 2 micrograms of each plasmid containing the cirRNAs or the empty plasmid were transfected using Lipofectamine 2000 (Invitrogen) following manufacturer's instructions. After overnight incubation, DMEM medium was removed and cells were washed with 1X PBS. Cells were inoculated with Chikungunya Virus for 1 h at 37 °C. Finally, the virus containing media was replaced with fresh media. Sixteen hours post infection, Luciferase activity was assayed. The supernatant of the cells was collected and inactivated with UV for 10 minutes. 4 µl of the supernatant were mixed with 20 µl of Renila Luciferase Assay Buffer and 1/200 of substrate from the Renilla Luciferase assay system (Promega) and measured immediately in a luminometer for 2 s. Mean relative light units (RLU) were plotted as percentage relative to control infections (cells transfected with the circoVIR plasmid).

Results are shown in Figure 10 and depict the significant inhibition of CHIKV infection with respect to the control (empty plasmid).

### Example 4:

We have designed and tested three circRNA (DENV1 cHP_ HCV CDS2_1 (SEQ ID NO.: 17), DENV cHP_ HCV CDS2_2 (circ_dv_cHP_v1_circ_hcv_cds2_2, SEQ ID NO.: 32), and (DENV1 cHP_ HCV CDS2_T (SEQ ID NO.: 16) against one region of DENV (Dengue) genome (SEQ ID NO.: 7) and one region of HCV genome (SEQ ID NO.: 1) as previously disclosed in the section Production of circRNAs. Plasmid to generate HCV (pFK-Luc-Jc1) carrying the Firefly luciferase reporter gene has been previously described (Wakita T et al., Production of infectious hepatitis C virus in tissue culture from a cloned viral genome, Nat Med, 2005;11:791-796). Plasmid to generate DENV (pFK-DVs-R2A) carrying the Renilla luciferase reporter gene has been previously described (Scaturro, P. et al., Characterization of the mode of action of a potent dengue virus capsid inhibitor, J. Virol. 88, 11540-55 (2014)).

The first 2 circRNAs contain 6 hybridization regions that target the corresponding regions in the DENV genome and the HCV genome, and the last one contains 7 against DENV and 12 against HCV.

### Cell cultures and transfection

The human embryonic kidney cell line HEK293 was maintained in Dulbecco's modified Eagle's medium (DMEM, Invitrogen, Carlsbad, CA) supplemented with 10% heat inactivated fetal bovine serum (FBS) and 10% non-essential amino acids. Cells were grown in an incubator with 5% CO2 at 37 °C. 1 10⁵ HEK293 cells/well were seeded in 24-well plates the day before transfection. 2 micrograms of each plasmid containing the cirRNAs or the empty plasmid were transfected using Lipofectamine 2000 (Invitrogen) following manufacturer's instructions. After overnight incubation, DMEM medium was removed and cells were washed with 1X PBS. Cells were inoculated with Dengue Virus for 4h at 37 °C. Finally, the virus containing media was replaced with fresh media. Forty-eight hours post infection, Luciferase activity was assayed. Cells were washed with 1X PBS, lysed in 150 µl of Renilla lysis buffer and frozen. Upon thawing, lysates were resuspended by pipetting. 4 µl of the lysates were mixed with 20 µl of Renila Luciferase Assay Buffer and 1/200 of substrate from the Renilla Luciferase assay system (Promega) and measured immediately in a luminometer for 2 s. Mean relative light units (RLU) were plotted as percentage relative to control infections (cells transfected with the circoVIR plasmid).

The human hepatocarcinoma cell line Huh7/Scr was maintained in Dulbecco's modified Eagle's medium (DMEM, Invitrogen, Carlsbad, CA) supplemented with 10% heat inactivated fetal bovine serum (FBS) and 10% non-essential amino acids. Cells were grown in an incubator with 5% CO2 at 37 °C. 2.5 10⁴ Huh7/Scr cells/well were seeded in 24-well plates the day before transfection. 2 micrograms of each plasmid containing the cirRNAs or the empty plasmid were transfected using Lipofectamine 2000 (Invitrogen) following manufacturer's instructions.

After overnight incubation, DMEM medium was removed and cells were washed with 1X PBS. Cells were inoculated with the different viruses for 4h at 37 °C. Finally, the virus containing media was replaced with fresh media. Forty-eight hours post infection, Luciferase activity was assayed. Cells were washed with 1X PBS, lysed in 150 µl of Passive lysis buffer and frozen. Upon thawing, lysates were resuspended by pipetting. 50 µl of the lysates were mixed with 25 µl of Luciferase Assay Reagent (Promega) and incubated 5 minutes at room temperature. Afterwards the luciferase activity was measured in a luminometer for 2 s. Mean relative light units (RLU) were plotted as percentage relative to control infections (cells transfected with the circoVIR plasmid).

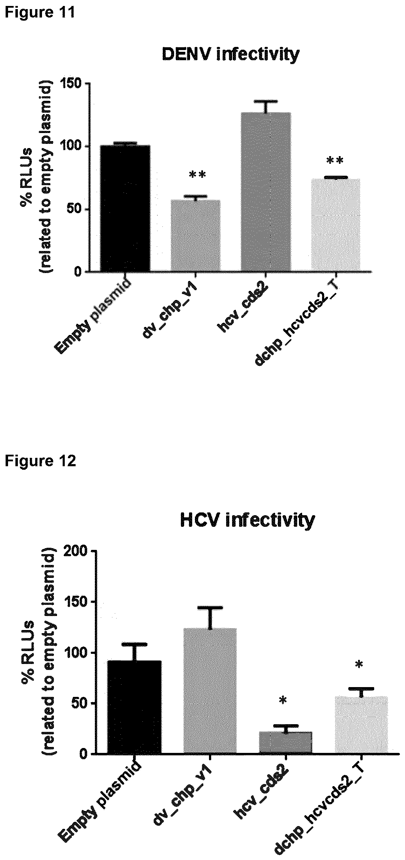

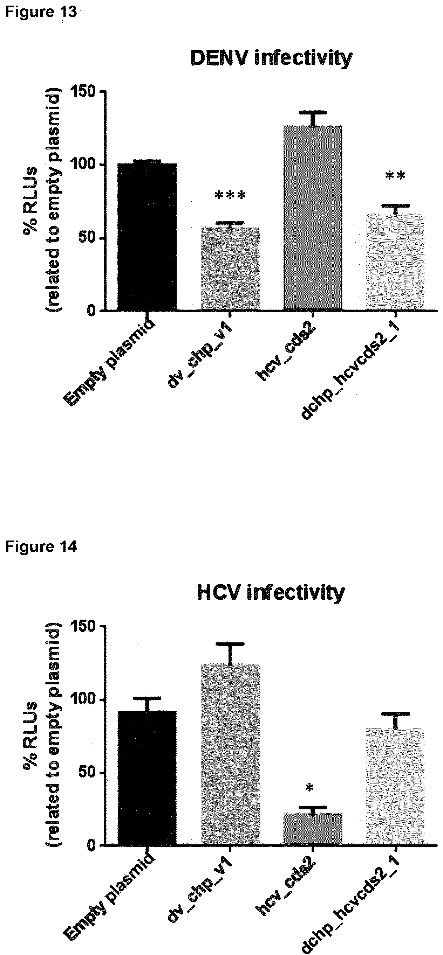

Results for each circRNA against both DENV and HCV along with the corresponding positive and negative controls from examples 1 and 2 are shown in figures 11, 12, 13 14 and 24. Inhibition is shown for all circRNAs in both viral infections, demonstrating the broadspectrum capabilities of the design circRNAs.

### Example 5:

We have designed and tested two circRNAs (circ_wnv_slll_1 (SEQ ID NO.: 24), circ_wnv_slll_2 (SEQ ID NO.: 19)) against one region of WNV (West Nile Virus) genome (SEQ ID NO.: 20) as previously disclosed in the section Production of circRNAs. Plasmid to generate WNV carrying the Nanoluc luciferase reporter gene was kindly provided by Dr. Merits, University of Tartu, Estonia. Both circRNAs contain 7 hybridization regions that target the corresponding region in the WNV genome.

### Cell cultures and transfection

The human embryonic kidney cell line HEK293 was maintained in Dulbecco's modified Eagle's medium (DMEM, Invitrogen, Carlsbad, CA) supplemented with 10% heat inactivated fetal bovine serum (FBS) and 10% non-essential amino acids. Cells were grown in an incubator with 5% CO2 at 37 °C. 1 10⁵ HEK293 cells/well were seeded in 24-well plates the day before transfection. 2 micrograms of each plasmid containing the circRNAs or the empty plasmid were transfected using Lipofectamine 2000 (Invitrogen) following manufacturer's instructions. After overnight incubation, DMEM medium was removed and cells were washed with 1X PBS. Cells were inoculated with WNV for 4h at 37 °C. Finally, the virus containing media was replaced with fresh media. Forty-eight hours post infection, Luciferase activity was assayed. Cells were washed with 1X PBS, lysed in 150 µl of Renilla lysis buffer and frozen. Upon thawing, lysates were resuspended by pipetting. 4 µl of the lysates were mixed with 20 µl of Renila Luciferase Assay Buffer and 1/200 of substrate from the Renilla Luciferase assay system (Promega) and measured immediately in a luminometer for 2 s. Mean relative light units (RLU) were plotted as percentage relative to control infections (cells transfected with the circoVIR plasmid).

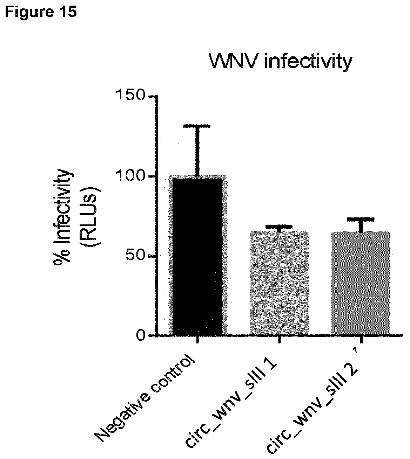

Results are shown in Figure 15 and depict the significant inhibition of WNV infection with respect to the control (empty plasmid).

### Example 6:

We have designed and tested three circRNA (dchp_wslll_A (SEQ ID NO.: 21), dchp_wslll_B (SEQ ID NO.: 22) and dchp_wslll_C (SEQ ID NO.: 23) against one region of DENV (Dengue) genome (SEQ ID NO: 7) and one region of WNV genome (SEQ ID NO.: 20) as previously disclosed in the section Production of circRNAs. Plasmid to generate DENV (pFK-DVs-R2A) carrying the Renilla luciferase reporter gene has been previously described (Scaturro, P. et al., Characterization of the mode of action of a potent dengue virus capsid inhibitor, J. Virol. 88, 11540-55 (2014)). Plasmid to generate WNV carrying the Nanoluc luciferase reporter gene was kindly provided by Dr. Merits, University of Tartu, Estonia. The circRNAs contain 6 hybridization regions that target the corresponding regions in the DENV genome and the WNV genome.

### Cell cultures and transfection

The human embryonic kidney cell line HEK293 was maintained in Dulbecco's modified Eagle's medium (DMEM, Invitrogen, Carlsbad, CA) supplemented with 10% heat inactivated fetal bovine serum (FBS) and 10% non-essential amino acids. Cells were grown in an incubator with 5% CO2 at 37 °C. 1x10⁵ HEK293 cells/well and were seeded in 24-well plates the day before transfection. 2 micrograms of each plasmid containing the circRNAs or the empty plasmid were transfected using Lipofectamine 2000 (Invitrogen) following manufacturer's instructions. After overnight incubation, DMEM medium was removed and cells were washed with 1X PBS. Cells were inoculated with Dengue Virus or West Nile Virus for 4 h at 37 °C. Finally, the virus containing media was replaced with fresh media. Forty-eight hours post infection, Luciferase activity was assayed. Cells were washed with 1X PBS, lysed in 150 µl of Renilla lysis buffer and frozen. Upon thawing, lysates were resuspended by pipetting. 4 µl of the lysates were mixed with 20 µl of Renila Luciferase Assay Buffer and 1/200 of substrate from the Renilla Luciferase assay system (Promega) and measured immediately in a luminometer for 2 s. Mean relative light units (RLU) were plotted as percentage relative to control infections (cells transfected with the circoVIR plasmid).

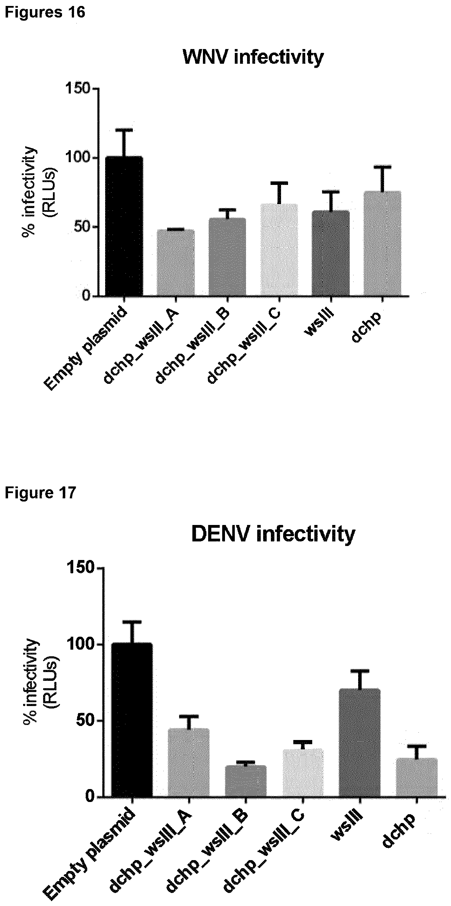

Results for each circRNA against both DENV and WNV along with the corresponding positive and negative controls from examples 2 and 5 are shown in figures 16 and 17. Inhibition is shown for the circRNAs in both viral infections, demonstrating the broadspectrum capabilities of the designed circRNAs.

### Example 7:

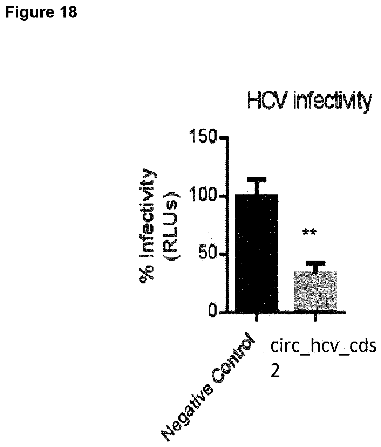

We tested whether circ_hcv_cds2 (SEQ ID NO.: 6) may inhibit infections already established. For this, Huh7/Scr cells were infected with HCVJc1-luc and 48 hours later transfected with circ_hcv_cds2 (SEQ ID NO.: 6). Luciferase values were measured 24 hours post-transfection. Importantly, circ_hcv_cds2 inhibited infectivity with similar efficiency as when cells were expressing the circ_hcv_cds2 before infection (Fig. 18).

### Example 8:

Next, we examine whether the circ_hcv_cds2 (SEQ ID NO.: 6) is indeed inhibiting the function described for the target sequence, viral RNA replication. For this, we used HCV RNA replicons that harbour a luciferase reporter gene but not the viral structural genes required for encapsidation. Thus, these replicons allow efficient translation and replication of the viral RNA genome but not virion production. Huh7/Scr cells were transfected with circ_hcv_cds2 (SEQ ID NO.: 6) or the corresponding empty plasmid and the next day, transfected with the HCV replicon. Luciferase values were measured at 4 hours and 48 hours post-HCV replicon transfection. These times were selected because already established kinetics prove that luciferase production derived at 4 hours is solely from HCV RNA translation and at 48 hours from both translation and replication. Indeed, a decrease in viral infectivity was observed at 48 but not at 4 hours post-transfection (Fig. 19) indicating that circ_hcv_cds2 impairs viral RNA replication.

### Example 9:

Circ_dv_3utr (SEQ ID NO.: 8) and circ_dv_cHP_v1 (SEQ ID NO.: 9), designed to target structures within the DENV RNA genome directing RNA replication, were tested to see if they inhibit DENV RNA replication following the same procedure as in Example 8. The results show that circ_dv_3utr and circ_dv_cHP_v1 inhibit luciferase expression levels at 48 hours when the RNA genome is translated and replicated but not at 8 hours when is solely translated (Fig. 20).

### Example 10:

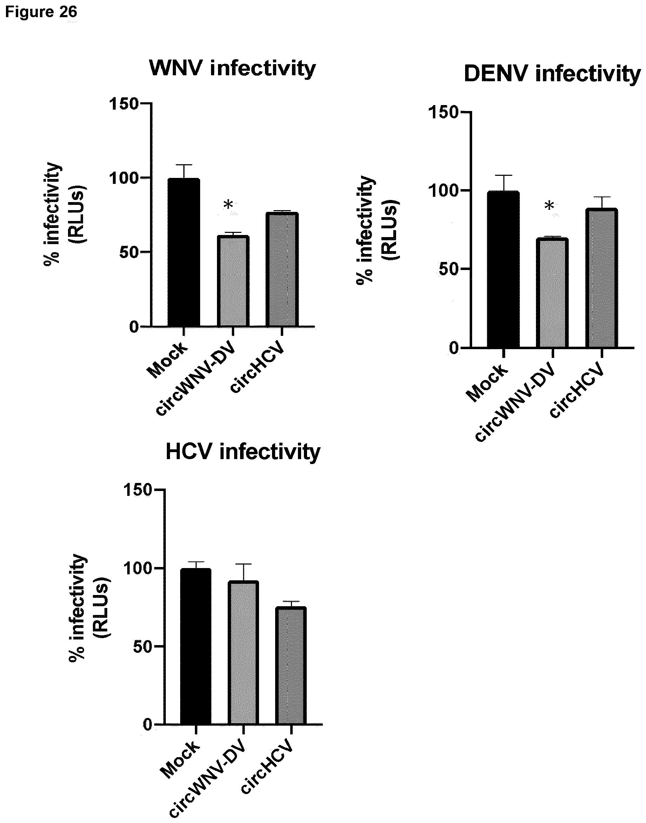

The human embryonic kidney cell line HEK293 and the Hepatocarcinoma cell line Huh7 were maintained in Dulbecco's modified Eagle's medium (DMEM, Invitrogen, Carlsbad, CA) supplemented with 10% heat inactivated fetal bovine serum (FBS) and 10% non-essential amino acids. Cells were grown in an incubator with 5% CO2 at 37 °C. 1x10⁵ HEK293 cells/well or 4x10⁴ Huh7/cells and were seeded in 24-well plates the day before transfection. 100 nanograms of circular RNAs against WNV-DENV (circWD1-*in vitro* SEQ ID NO.: 63) or circular RNA against HCV (circHCV-*in vitro* SEQ ID NO.: 64) were transfected using Lipofectamine 2000 (Invitrogen) following manufacturer's instructions. After overnight incubation, DMEM medium was removed and cells were washed with 1X PBS. HEK293 cells were inoculated with Dengue Virus or West Nile Virus for 4h at 37 °C. In parallel, Huh7 cells were inoculated with Hepatitis C virus for 4h at 37°C. Finally, the virus containing media was replaced with fresh media. Forty-eight hours post infection, Luciferase activity was assayed. Cells were washed with 1X PBS, lysed in 150 µl of Renilla lysis buffer (HEK293) or 150 µl of Passive lysis buffer and frozen. Upon thawing, lysates were resuspended by pipetting. In the case of DENV and WNV, 4 µl of the lysates were mixed with 20 µl of Renila Luciferase Assay Buffer and 1/200 of substrate from the Renilla Luciferase assay system (Promega) and measured immediately in a luminometer for 2 s. In the case of HCV, 50 µl of the lysates were mixed with 25 µl of Luciferase Assay Reagent (LARII) for 5 minutes and measured immediately in a luminometer for 2 s. Mean relative light units (RLU) were plotted as percentage relative to control infections (cells transfected with the circoVIR plasmid) in Figure 26.

### Further items of the invention

The present invention also comprises the following items:
1. An artificial circular RNA between 200 and 600 nucleotides having 6 to 20 hybridization regions of sizes between 10 and 50 nucleotides against one or more structured regions of one or more RNA viral genomes, wherein such hybridization regions have sequences that are different among each other, wherein the one or more structured regions are structured regions vital for the viral life cycle (SRVVLC) preceded by a single stranded region, and wherein the artificial circular RNA is capable of disrupting the structure of the one or more SRVVLC, rendering the virus less infective.
2. The artificial circular RNA of item 1, wherein the hybridization regions are:
   a) separated by non-hybridization regions of sizes up to 20 nucleotides; or
   b) are not separated by non-hybridization regions; or
   c) are overlapping.
3. The artificial circular RNA according to any of the preceding items, wherein the target of the hybridization regions is a SRVVLC of the IRES element of the viral genome.
4. The artificial circular RNA according to any of the items 1 o 2, wherein the target of the hybridization regions is a SRVVLC of the 5'UTR of the viral genome.
5. The artificial circular RNA according to any of the items 1 or 2, wherein the target of the hybridization regions is a SRVVLC of the CDS of the viral genome.
6. The artificial circular RNA according to items 1 or 2, wherein the target of the hybridization regions is a SRVVLC of the 3'UTR of the viral genome.
7. The artificial circular RNA, according to any of items 1 to 6, wherein the target of the hybridization regions is a combination of two or more of the IRES element, the region of the 5'UTR, the region of the CDS or the region of the 3'UTR of the viral genome.
8. The artificial circular RNA according to item 3 or 7, wherein said viral genome is selected from HCV, HAV, Poliovirus, Coxsackie B virus and rhinovirus (common cold).
9. The artificial circular RNA according to any of items 4 to 7, wherein said viral genome is selected from HCV, Dengue, Zika, Chikungunya, West Nile and Yellow Fever virus.
10. The artificial circular RNA according to any of items 1 to 9, wherein said circular RNA has a broad spectrum activity against two or more RNA viral genome.
11. A composition comprising the artificial circular RNA according to any of the previous items.
12. A kit comprising the artificial circular RNA according to any of items 1 to 10 or the composition according to item 11, and instructions for using said circular RNA or composition.
13. The artificial circular RNA according to any of items 1 to 10, or the composition according to item 11 or the kit according to items 12, wherein said circular RNA comprises the sequence selected from SEQ ID NO: 2, 4, 5, 6 (for HCV); 8, 9, 10 (for Dengue virus); 12, 13, 14, 15, 39 (for Chikungunya virus); 16 and 17 (Broadspectrum activity for both HCV and Dengue Virus); 24 and 19 (for West Nile Virus); 21, 22 and 23 (Broad spectrum activity for both Dengue and West Nile Viruses); 32 (Broad spectrum activity for both HCV and Dengue Virus), whrerin the one or more targets of the 6 to 20 hybridization regions is comprised in SEQ ID NO.: 1, SEQ ID NO.: 7, SEQ ID NO.: 11 and/or SEQ ID NO.: 20.
14. The artificial circular RNA according to any of items 1 to 10 or 13, or the composition according to item 11 or 13 or the kit according to item 12 or 13 for use in preventing and/or treating a viral infection.
15. The artificial circular RNA or the composition or the kit for use according to item 14, wherein said viral infection is caused by HCV, HAV, Poliovirus, Coxsackie B virus, rhinovirus (common cold), Dengue, Zika, Chikungunya, West Nile or Yellow Fever virus.

## Claims

1. An artificial RNA suitable for disrupting by hybridization one or more target disruption structures of one or more RNA fragments,
(a) wherein the **artificial RNA** comprises between 150 and 800 nucleotides, preferably between 200 and 600 nucleotides;
(b) wherein the artificial RNA comprises at least one **hybridization region** which:
(i) completely hybridizes with at least one target hybridization region comprised in the one or more target disruption structures of the one or more RNA fragments; and
(ii) has a total of between 7 and 100 nucleotides, preferably between 10 and 50 nucleotides;
(c) wherein the one or more **target disruption structures:**
(ii) comprises at least a hairpin loop preceded or followed by a region of unpaired nucleotides; and
(i) comprises at least one **target hybridization region** which comprises a single-stranded region of at least 2 nucleotides, preferably 3 nucleotides or more preceded or followed by a double-stranded region of at least 5 nucleotides, preferably 10 nucleotides or more, wherein the at least one target hybridization region completely hybridizes with the at least one hybridization region of the artificial RNA; and
(d) wherein the at least one **hybridization region** comprised in the artificial RNA is further characterized because, when hybridizing with the target hybridization region, the energy of the hybridization between the at least one hybridization region and the at least one target hybridization region is more negative than the energy of the target disruption region, thereby disrupting the target disruption structure, and wherein the energy of hybridization is measured using the RNAcofold software.

2. The artificial RNA of claim 1 wherein the artificial RNA is a circular RNA.

3. The artificial RNA of any of the preceding claims wherein the artificial RNA comprises 2 or more hybridization regions, preferably between 6 and 20 hybridization regions.

4. The artificial RNA of claim 3 wherein at least two, and preferably all, of the hybridization regions are capable of completely hybridizing with the same target hybridization region.

5. The artificial RNA of any one of claims 3-4 wherein at least two, and preferably all, of the hybridization regions have different nucleotide sequences.

6. The artificial RNA of claim 5, wherein at least two, and preferably all, of the hybridization regions have a sequence identity of at least 95%, preferably of at least 98%, more preferably of at least 99%.

7. The artificial RNA of any one of claims 3-6, wherein the 2 or more hybridization regions are:
a) separated by non-hybridization regions of sizes up to 20 nucleotides; or
b) are not separated by non-hybridization regions; or
c) are overlapping.

8. The artificial RNA of any one of the preceding claims, wherein the one or more RNA fragments is selected from mRNA, tRNA, rRNA, non-coding RNA and viral genomic RNA.

9. The artificial RNA of claim 9, wherein the one or more RNA fragments is viral genomic RNA.

10. The artificial RNA of claim 9, wherein the one or more RNA fragments is positive-sense single-stranded viral genomic RNA.

11. The artificial RNA of any of claims 9-10, wherein the one or more target disruption structures is comprised in the IRES element of the viral genome, preferably wherein the one or more target disruption structures comprised in the IRES element of the viral genome is a structured region vital for the viral life cycle (SRVVLC).

12. The artificial RNA of any of claims 9-10, wherein the one or more target disruption structures is comprised in the 5'UTR and/or in the 3'UTR of the viral genome, preferably wherein the one or more target disruption structures comprised in the 5'UTR and/or in the 3'UTR of the viral genome is a structured region vital for the viral life cycle (SRVVLC).

13. The artificial RNA of any of claims 9-10, wherein the one or more target disruption structures is comprised in the CDS of the viral genome, preferably wherein the one or more target disruption structures comprised in the CDS of the viral genome is a structured region vital for the viral life cycle (SRVVLC).

14. The artificial RNA of any of claims 9-10, wherein the at least two hybridization regions of the artificial RNA is capable of completely hybridizing with at least one target hybridization region, preferably two target hybridization regions, comprised in at least one disruption structure, preferably at least two target disruption structure, present in any combination of two of the following locations: the IRES element, the region of the 5'UTR, the region of the CDS and/or the region of the 3'UTR of the viral genome.

15. The artificial RNA of any of claims 11-14, wherein the disruption of the one or more structured regions vital for the viral life cycle renders the virus less active.

16. The artificial RNA of any of claims 9-15, wherein the viral genomic RNA is selected from HCV, HAV, Poliovirus, Coxsackie B virus, Coronavirus and Rhinovirus (common cold).

17. The artificial RNA of any of claims 9-15, wherein the viral genomic RNA is selected from HCV, Dengue, Zika, Chikungunya, West Nile and Yellow Fever virus.

18. The artificial RNA of any of claims 9-17, wherein the artificial RNA is able to disrupt by hybridization one or more target disruption structures comprised in at least two viral genomic RNAs, preferably wherein the artificial RNA is able to disrupt by hybridization one or more structured region vital for the viral life cycle comprised in at least two viral genomic

19. A composition comprising the artificial RNA as defined in any of the previous claims.

20. A kit comprising the artificial RNA as defined in any of claims 1 to 18 or comprising the composition as defined in claim 19, and instructions for using the artificial RNA or composition.

21. The artificial RNA of any of claims 1 to 18, or the composition of claim 19, or the kit of claim 20, wherein the sequence of the artificial RNA comprises, or preferably, consists of, the following nucleotides defined in: SEQ ID NO: 2, 3, 4, 5, 6 (for HCV); 8, 9, 10 (for Dengue virus); 12, 13, 14, 15, 39 (for Chikungunya virus); 16 and 17 (broad spectrum activity for both HCV and Dengue Virus); 24 and 19 (for West Nile Virus); 21, 22 and 23 (broad spectrum activity for both Dengue and West Nile Viruses); 32 (broad spectrum activity for both HCV and Dengue Virus); 36, 38, 40, 41, 42, 43, 44, 45, 46, 47, 48, 49, 50, 51, 52, 53, 54, 55, 56 and 57 (for SARS-CoV-2).

22. The artificial RNA of claim 21 or the composition of claim 19, or the kit of claim 20, wherein the one or more target hybridization region of the artificial RNA which completely hybridize with the at least one hybridization region is comprised in the artificial RNA defined in SEQ ID NO.: 1, SEQ ID NO.: 7, SEQ ID NO.: 11 SEQ ID NO.: 34 and/or SEQ ID NO.: 20.

23. The artificial RNA of any one of claims 1 to 18 and 21 to 22, or the composition of any of claims 19-22, or the kit of any one of claims 20-22 for use as a medicament.

24. The artificial RNA of any one of claims 1 to 18 and 21 to 23, or the composition of any of claims 19-23, or the kit of any one of claims 20-23 for use in a method of preventing and/or treating a viral infection.

25. The artificial RNA of any one of claims 1 to 18 and 21 to 23, or the composition of any of claims 1923, or the kit of any one of claims 20-23 for use according to claim 24, wherein the viral infection is caused by HCV, HAV, Poliovirus, Coxsackie B virus, rhinovirus (common cold), Dengue, Zika, Chikungunya, West Nile, Yellow Fever virus or coronavirus, such as SARS and/or MERS, preferably SARS-CoV-2.


SEQUENCE LISTING

<110> Universitat Pompeu Fabra

<120> Artificial RNAs for modulating RNA fragments

<130> 905243

<160> 64

<170> BiSSAP 1.3.6

<210> 1
<211> 9678
<212> RNA
<213> Hepatitis C virus

<220>
<221> misc_feature
<222> 24..56
<223> /note="target region of the 5'UTR"

<220>
<221> misc_feature
<222> 323..346
<223> /note="target region of the 5'UTR"

<220>
<221> misc_feature
<222> 372..399
<223> /note="target region of the CDS"

<220>
<221> misc_feature
<222> 459..474
<223> /note="target region of the CDS"

<220>
<221> misc_feature
<222> 651..668
<223> /note="target region of the CDS"

<220>
<221> misc_feature
<222> 9567..9600
<223> /note="target region of the 3'UTR"

<400> 1
accugccccu aauaggggcg acacuccgcc augaaucacu ccccugugag gaacuacugu 60

cuucacgcag aaagcgccua gccauggcgu uaguaugagu gucguacagc cuccaggccc 120

cccccucccg ggagagccau aguggucugc ggaaccggug aguacaccgg aauugccggg 180

aagacugggu ccuuucuugg auaaacccac ucuaugcccg gccauuuggg cgugcccccg 240

caagacugcu agccgaguag cguuggguug cgaaaggccu ugugguacug ccugauaggg 300

cgcuugcgag ugccccggga ggucucguag accgugcacc augagcacaa auccuaaacc 360

ucaaagaaaa accaaaagaa acaccaaccg ucgcccagaa gacguuaagu ucccgggcgg 420

cggccagauc guuggcggag uauacuuguu gccgcgcagg ggccccaggu ugggugugcg 480

cacgacaagg aaaacuucgg agcgguccca gccacguggg agacgccagc ccauccccaa 540

agaucggcgc uccacuggca aggccugggg aaaaccaggu cgccccuggc cccuauaugg 600

gaaugaggga cucggcuggg caggauggcu ccuguccccc cgaggcucuc gccccuccug 660

gggccccacu gacccccggc auaggucgcg caacgugggu aaagucaucg acacccuaac 720

guguggcuuu gccgaccuca ugggguacau ccccgucgua ggcgccccgc uuaguggcgc 780

cgccagagcu gucgcgcacg gcgugagagu ccuggaggac gggguuaauu augcaacagg 840

gaaccuaccc gguuuccccu uuucuaucuu cuugcuggcc cuguuguccu gcaucaccgu 900

uccggucucu gcugcccagg ugaagaauac caguagcagc uacaugguga ccaaugacug 960

cuccaaugac agcaucacuu ggcagcucga ggcugcgguu cuccacgucc ccgggugcgu 1020

cccgugcgag agagugggga auacgucacg guguugggug ccagucucgc caaacauggc 1080

ugugcggcag cccggugccc ucacgcaggg ucugcggacg cacaucgaua ugguugugau 1140

guccgccacc uucugcucug cucucuacgu gggggaccuc uguggcgggg ugaugcucgc 1200

ggcccaggug uucaucgucu cgccgcagua ccacugguuu gugcaagaau gcaauugcuc 1260

caucuacccu ggcaccauca cuggacaccg cauggcaugg gacaugauga ugaacugguc 1320

gcccacggcc accaugaucc uggcguacgu gaugcgcguc cccgagguca ucauagacau 1380

cguuagcggg gcucacuggg gcgucauguu cggcuuggcc uacuucucua ugcagggagc 1440

gugggcgaag gucauuguca uccuucugcu ggccgcuggg guggacgcgg gcaccaccac 1500

cguuggaggc gcuguugcac guuccaccaa cgugauugcc ggcguguuca gccauggccc 1560

ucagcagaac auucagcuca uuaacaccaa cggcaguugg cacaucaacc guacugccuu 1620

gaauugcaau gacuccuuga acaccggcuu ucucgcggcc uuguucuaca ccaaccgcuu 1680

uaacucguca ggguguccag ggcgccuguc cgccugccgc aacaucgagg cuuuccggau 1740

aggguggggc acccuacagu acgaggauaa ugucaccaau ccagaggaua ugaggccgua 1800

cugcuggcac uaccccccaa agccgugugg cguagucccc gcgaggucug uguguggccc 1860

aguguacugu uucaccccca gcccgguagu agugggcacg accgacagac guggagugcc 1920

caccuacaca uggggagaga augagacaga ugucuuccua cugaacagca cccgaccgcc 1980

gcagggcuca ugguucggcu gcacguggau gaacuccacu gguuucacca agacuugugg 2040

cgcgccaccu ugccgcacca gagcugacuu caacgccagc acggacuugu ugugcccuac 2100

ggauuguuuu aggaagcauc cugaugccac uuauauuaag ugugguucug ggcccuggcu 2160

cacaccaaag ugccuggucc acuacccuua cagacucugg cauuaccccu gcacagucaa 2220

uuuuaccauc uucaagauaa gaauguaugu aggggggguu gagcacaggc ucacggccgc 2280

augcaacuuc acucgugggg aucgcugcga cuuggaggac agggacagga gucagcuguc 2340

uccucuguug cacucuacca cggaaugggc cauccugccc ugcaccuacu cagacuuacc 2400

cgcuuuguca acuggucuuc uccaccuuca ccagaacauc guggacguac aauacaugua 2460

uggccucuca ccugcuauca caaaauacgu cguucgaugg gagugggugg uacucuuauu 2520

ccugcucuua gcggacgcca gagucugcgc cugcuugugg augcucaucu uguuggccca 2580

ggccgaagca gcauuggaga aguuggucgu cuugcacgcu gcgagugcgg cuaacugcca 2640

uggccuccua uauuuugcca ucuucuucgu ggcagcuugg cacaucaggg gucggguggu 2700

ccccuugacc accuauugcc ucacuggccu auggcccuuc ugccuacugc ucauggcacu 2760

gccccggcag gcuuaugccu augacgcacc ugugcacgga cagauaggcg uggguuuguu 2820

gauauugauc acccucuuca cacucacccc gggguauaag acccuccucg gccagugucu 2880

guggugguug ugcuaucucc ugacccuggg ggaagccaug auucaggagu ggguaccacc 2940

caugcaggug cgcggcggcc gcgauggcau cgcgugggcc gucacuauau ucugcccggg 3000

ugugguguuu gacauuacca aauggcuuuu ggcguugcuu gggccugcuu accucuuaag 3060

ggccgcuuug acacaugugc cguacuucgu cagagcucac gcucugauaa ggguaugcgc 3120

uuuggugaag cagcucgcgg gggguaggua uguucaggug gcgcuauugg cccuuggcag 3180

guggacuggc accuacaucu augaccaccu cacaccuaug ucggacuggg ccgcuagcgg 3240

ccugcgcgac uuagcggucg ccguggaacc caucaucuuc aguccgaugg agaagaaggu 3300

caucgucugg ggagcggaga cggcugcaug uggggacauu cuacauggac uucccguguc 3360

cgcccgacuc ggccaggaga uccuccucgg cccagcugau ggcuacaccu ccaaggggug 3420

gaagcuccuu gcucccauca cugcuuaugc ccagcaaaca cgaggccucc ugggcgccau 3480

aguggugagu augacggggc gugacaggac agaacaggcc ggggaagucc aaauccuguc 3540

cacagucucu caguccuucc ucggaacaac caucucgggg guuuugugga cuguuuacca 3600

cggagcuggc aacaagacuc uagccggcuu acgggguccg gucacgcaga uguacucgag 3660

ugcugagggg gacuugguag gcuggcccag ccccccuggg accaagucuu uggagccgug 3720

caagugugga gccgucgacc uauaucuggu cacgcggaac gcugauguca ucccggcucg 3780

gagacgcggg gacaagcggg gagcauugcu cuccccgaga cccauuucga ccuugaaggg 3840

guccucgggg gggccggugc ucugcccuag gggccacguc guugggcucu uccgagcagc 3900

ugugugcucu cggggcgugg ccaaauccau cgauuucauc cccguugaga cacucgacgu 3960

uguuacaagg ucucccacuu ucagugacaa cagcacgcca ccggcugugc cccagaccua 4020

ucaggucggg uacuugcaug cuccaacugg caguggaaag agcaccaagg ucccugucgc 4080

guaugccgcc cagggguaca aaguacuagu gcuuaacccc ucgguagcug ccacccuggg 4140

guuuggggcg uaccuaucca aggcacaugg caucaauccc aacauuagga cuggagucag 4200

gaccgugaug accggggagg ccaucacgua cuccacauau ggcaaauuuc ucgccgaugg 4260

gggcugcgcu agcggcgccu augacaucau cauaugcgau gaaugccacg cuguggaugc 4320

uaccuccauu cucggcaucg gaacgguccu ugaucaagca gagacagccg gggucagacu 4380

aacugugcug gcuacggcca caccccccgg gucagugaca accccccauc ccgauauaga 4440

agagguaggc cucgggcggg agggugagau ccccuucuau gggagggcga uuccccuauc 4500

cugcaucaag ggagggagac accugauuuu cugccacuca aagaaaaagu gugacgagcu 4560

cgcggcggcc cuucggggca ugggcuugaa ugccguggca uacuauagag gguuggacgu 4620

cuccauaaua ccagcucagg gagauguggu ggucgucgcc accgacgccc ucaugacggg 4680

guacacugga gacuuugacu ccgugaucga cugcaaugua gcggucaccc aagcugucga 4740

cuucagccug gaccccaccu ucacuauaac cacacagacu gucccacaag acgcugucuc 4800

acgcagucag cgccgcgggc gcacagguag aggaagacag ggcacuuaua gguauguuuc 4860

cacuggugaa cgagccucag gaauguuuga caguguagug cuuugugagu gcuacgacgc 4920

aggggcugcg ugguacgauc ucacaccagc ggagaccacc gucaggcuua gagcguauuu 4980

caacacgccc ggccuacccg ugugucaaga ccaucuugaa uuuugggagg caguuuucac 5040

cggccucaca cacauagacg cccacuuccu cucccaaaca aagcaagcgg gggagaacuu 5100

cgcguaccua guagccuacc aagcuacggu gugcgccaga gccaaggccc cucccccguc 5160

cugggacgcc auguggaagu gccuggcccg acucaagccu acgcuugcgg gccccacacc 5220

ucuccuguac cguuugggcc cuauuaccaa ugaggucacc cucacacacc cugggacgaa 5280

guacaucgcc acaugcaugc aagcugaccu ugaggucaug accagcacgu ggguccuagc 5340

uggaggaguc cuggcagccg ucgccgcaua uugccuggcg acuggaugcg uuuccaucau 5400

cggccgcuug cacgucaacc agcgagucgu cguugcgccg gauaaggagg uccuguauga 5460

ggcuuuugau gagauggagg aaugcgccuc uagggcggcu cucaucgaag aggggcagcg 5520

gauagccgag auguugaagu ccaagaucca aggcuugcug cagcaggccu cuaagcaggc 5580

ccaggacaua caacccgcua ugcaggcuuc auggcccaaa guggaacaau uuugggccag 5640

acacaugugg aacuucauua gcggcaucca auaccucgca ggauugucaa cacugccagg 5700

gaaccccgcg guggcuucca ugauggcauu cagugccgcc cucaccaguc cguugucgac 5760

caguaccacc auccuucuca acaucauggg aggcugguua gcgucccaga ucgcaccacc 5820

cgcgggggcc accggcuuug ucgucagugg ccuggugggg gcugccgugg gcagcauagg 5880

ccuggguaag gugcuggugg acauccuggc aggauauggu gcgggcauuu cgggggcccu 5940

cgucgcauuc aagaucaugu cuggcgagaa gcccucuaug gaagauguca ucaaucuacu 6000

gccugggauc cugucuccgg gagcccuggu gguggggguc aucugcgcgg ccauucugcg 6060

ccgccacgug ggaccggggg agggcgcggu ccaauggaug aacaggcuua uugccuuugc 6120

uuccagagga aaccacgucg ccccuacuca cuacgugacg gagucggaug cgucgcagcg 6180

ugugacccaa cuacuuggcu cucuuacuau aaccagccua cucagaagac uccacaauug 6240

gauaacugag gacugcccca ucccaugcuc cggauccugg cuccgcgacg ugugggacug 6300

gguuugcacc aucuugacag acuucaaaaa uuggcugacc ucuaaauugu uccccaagcu 6360

gcccggccuc cccuucaucu cuugucaaaa gggguacaag gguguguggg ccggcacugg 6420

caucaugacc acgcgcugcc cuugcggcgc caacaucucu ggcaaugucc gccugggcuc 6480

uaugaggauc acagggccua aaaccugcau gaacaccugg caggggaccu uuccuaucaa 6540

uugcuacacg gagggccagu gcgcgccgaa accccccacg aacuacaaga ccgccaucug 6600

gaggguggcg gccucggagu acgcggaggu gacgcagcau gggucguacu ccuauguaac 6660

aggacugacc acugacaauc ugaaaauucc uugccaacua ccuucuccag aguuuuucuc 6720

cuggguggac ggugugcaga uccauagguu ugcacccaca ccaaagccgu uuuuccggga 6780

ugaggucucg uucugcguug ggcuuaauuc cuaugcuguc gggucccagc uucccuguga 6840

accugagccc gacgcagacg uauugagguc caugcuaaca gauccgcccc acaucacggc 6900

ggagacugcg gcgcggcgcu uggcacgggg aucaccucca ucugaggcga gcuccucagu 6960

gagccagcua ucagcaccgu cgcugcgggc caccugcacc acccacagca acaccuauga 7020

cguggacaug gucgaugcca accugcucau ggagggcggu guggcucaga cagagccuga 7080

guccagggug cccguucugg acuuucucga gccaauggcc gaggaagaga gcgaccuuga 7140

gcccucaaua ccaucggagu gcaugcuccc caggagcggg uuuccacggg ccuuaccggc 7200

uugggcacgg ccugacuaca acccgccgcu cguggaaucg uggaggaggc cagauuacca 7260

accgcccacc guugcugguu gugcucuccc cccccccaag aaggccccga cgccuccccc 7320

aaggagacgc cggacagugg gucugagcga gagcaccaua ucagaagccc uccagcaacu 7380

ggccaucaag accuuuggcc agccccccuc gagcggugau gcaggcucgu ccacgggggc 7440

gggcgccgcc gaauccggcg guccgacguc cccuggugag ccggcccccu cagagacagg 7500

uuccgccucc ucuaugcccc cccucgaggg ggagccugga gauccggacc uggagucuga 7560

ucagguagag cuucaaccuc ccccccaggg ggggggggua gcucccgguu cgggcucggg 7620

gucuuggucu acuugcuccg aggaggacga uaccaccgug ugcugcucca ugucauacuc 7680

cuggaccggg gcucuaauaa cucccuguag ccccgaagag gaaaaguugc caaucaaccc 7740

uuugaguaac ucgcuguugc gauaccauaa caagguguac uguacaacau caaagagcgc 7800

cucacagagg gcuaaaaagg uaacuuuuga caggacgcaa gugcucgacg cccauuauga 7860

cucagucuua aaggacauca agcuagcggc uuccaagguc agcgcaaggc uccucaccuu 7920

ggaggaggcg ugccaguuga cuccacccca uucugcaaga uccaaguaug gauucggggc 7980

caaggagguc cgcagcuugu ccgggagggc cguuaaccac aucaaguccg uguggaagga 8040

ccuccuggaa gacccacaaa caccaauucc cacaaccauc auggccaaaa augagguguu 8100

cugcguggac cccgccaagg gggguaagaa accagcucgc cucaucguuu acccugaccu 8160

cggcguccgg gucugcgaga aaauggcccu cuaugacauu acacaaaagc uuccucaggc 8220

gguaauggga gcuuccuaug gcuuccagua cuccccugcc caacgggugg aguaucucuu 8280

gaaagcaugg gcggaaaaga aggaccccau ggguuuuucg uaugauaccc gaugcuucga 8340

cucaaccguc acugagagag acaucaggac cgaggagucc auauaccagg ccugcucccu 8400

gcccgaggag gcccgcacug ccauacacuc gcugacugag agacuuuacg uaggagggcc 8460

cauguucaac agcaaggguc aaaccugcgg uuacagacgu ugccgcgcca gcggggugcu 8520

aaccacuagc auggguaaca ccaucacaug cuaugugaaa gcccuagcgg ccugcaaggc 8580

ugcggggaua guugcgccca caaugcuggu augcggcgau gaccuaguag ucaucucaga 8640

aagccagggg acugaggagg acgagcggaa ccugagagcc uucacggagg ccaugaccag 8700

guacucugcc ccuccuggug auccccccag accggaauau gaccuggagc uaauaacauc 8760

cuguuccuca aaugugucug uggcguuggg cccgcggggc cgccgcagau acuaccugac 8820

cagagaccca accacuccac ucgcccgggc ugccugggaa acaguuagac acuccccuau 8880

caauucaugg cugggaaaca ucauccagua ugcuccaacc auauggguuc gcaugguccu 8940

aaugacacac uucuucucca uucucauggu ccaagacacc cuggaccaga accucaacuu 9000

ugagauguau ggaucaguau acuccgugaa uccuuuggac cuuccagcca uaauugagag 9060

guuacacggg cuugacgccu uuucuaugca cacauacucu caccacgaac ugacgcgggu 9120

ggcuucagcc cucagaaaac uuggggcgcc accccucagg guguggaaga gucgggcucg 9180

cgcagucagg gcgucccuca ucucccgugg agggaaagcg gccguuugcg gccgauaucu 9240

cuucaauugg gcggugaaga ccaagcucaa acucacucca uugccggagg cgcgccuacu 9300

ggacuuaucc aguugguuca ccgucggcgc cggcgggggc gacauuuuuc acagcguguc 9360

gcgcgcccga ccccgcucau uacucuucgg ccuacuccua cuuuucguag ggguaggccu 9420

cuuccuacuc cccgcucggu agagcggcac acacuaggua cacuccauag cuaacuguuc 9480

cuuuuuuuuu uuuuuuuuuu uuuuuuuuuu uuuuuuuuuu uuuucuuuuu uuuuuuuuuc 9540

ccucuuucuu cccuucucau cuuauucuac uuucuuucuu gguggcucca ucuuagcccu 9600

agucacggcu agcugugaaa gguccgugag ccgcaugacu gcagagagug ccguaacugg 9660

ucucucugca gaucaugu 9678

<210> 2
<211> 294
<212> RNA
<213> Artificial sequence

<220>
<223> Circular Artificial sequence to IRES1 (hepatitis C virus)
      (circ_hcv_ires1)

<220>
<221> misc_feature
<222> 1..33
<223> /note="hybridization site to IRES (hepatitis c virus)"

<220>
<221> misc_feature
<222> 1..33
<223> /note="hybridization site to IRES (hepatitis c virus)"

<220>
<221> misc_feature
<222> 43..75
<223> /note="hybridization site to IRES (hepatitis c virus)"

<220>
<221> misc_feature
<222> 85..117
<223> /note="hybridization site to IRES (hepatitis c virus)"

<220>
<221> misc_feature
<222> 127..159
<223> /note="hybridization site to IRES (hepatitis c virus)"

<220>
<221> misc_feature
<222> 169..201
<223> /note="hybridization site to IRES (hepatitis c virus)"

<220>
<221> misc_feature
<222> 169..201
<223> /note="hybridization site to IRES (hepatitis c virus)"

<220>
<221> misc_feature
<222> 211..243
<223> /note="hybridization site to IRES (hepatitis c virus)"

<220>
<221> misc_feature
<222> 253..285
<223> /note="hybridization site to IRES (hepatitis c virus)"

<400> 2
uaguuccuca cgggggagug guucguggug gagcggcgcc aaugguuuuu ugcgggggag      60

ugguucgugg cggggguaac cccuuaguuc uucauggggg ggugguucau gguggggaaa      120

cuaccguagu uuuucacagg ggggugguuc auggcggaga gaagcgcuua guuucuuaug      180

ggggagugau ucauggcgga guguggggca ugguuuuucg caggggagug auucguggug      240

gggagaguuu ugugguucuu uguagggggg ugauucgugg uggggacauc auug      294

<210> 3
<211> 373
<212> RNA
<213> Artificial sequence

<220>
<223> Circular Artificial sequence to IRES2 (hepatitis C virus)

<220>
<221> misc_feature
<222> 11..34
<223> /note="hybridization site to IRES (hepatitis c virus)"

<220>
<221> misc_feature
<222> 44..67
<223> /note="hybridization site to IRES (hepatitis c virus)"

<220>
<221> misc_feature
<222> 77..100
<223> /note="hybridization site to IRES (hepatitis c virus)"

<220>
<221> misc_feature
<222> 110..123
<223> /note="hybridization site to IRES (hepatitis c virus)"

<220>
<221> misc_feature
<222> 133..166
<223> /note="hybridization site to IRES (hepatitis c virus)"

<220>
<221> misc_feature
<222> 176..199
<223> /note="hybridization site to IRES (hepatitis c virus)"

<220>
<221> misc_feature
<222> 209..232
<223> /note="hybridization site to IRES (hepatitis c virus)"

<220>
<221> misc_feature
<222> 242..265
<223> /note="hybridization site to IRES (hepatitis c virus)"

<220>
<221> misc_feature
<222> 275..298
<223> /note="hybridization site to IRES (hepatitis c virus)"

<220>
<221> misc_feature
<222> 308..331
<223> /note="hybridization site to IRES (hepatitis c virus)"

<220>
<221> misc_feature
<222> 341..373
<223> /note="hybridization site to IRES (hepatitis c virus)"

<400> 3
ugccgaaaau gcuuauggug cacgguuugc gagagcaaga agagcuugug gugcaugguu      60

ugcggggaca guaaaggcuc auggugcaug gucuacgagg ugagauagcg uuuauggugu     120

acggucugug ggaaauuccg guguucgugg ugugcggucu gugggauauu agaucguuca     180

ugguguacgg uuugugggga uaauagcggc uuauggugca cggucuaugg gacgcgagaa     240

ggcucauggu gugugguuug ugaggcagug ugcgguucau ggugcacggu cugcgagaca     300

ccaggcggcu cguggugcac ggucuauggg gugucgucgu gcuuguggug ugcggucuau     360

gagggcggcu gaa                                                        373

<210> 4
<211> 346
<212> RNA
<213> Artificial sequence

<220>
<223> Circular Artificial sequence to CDS1 (hepatitis C virus)
      (circ_hcv_cds1)

<220>
<221> misc_feature
<222> 11..38
<223> /note="hybridization site to CDS1 (hepatitis C virus)"

<220>
<221> misc_feature
<222> 53..80
<223> /note="hybridization site to CDS1 (hepatitis C virus)"

<220>
<221> misc_feature
<222> 95..122
<223> /note="hybridization site to CDS1 (hepatitis C virus)"

<220>
<221> misc_feature
<222> 137..164
<223> /note="hybridization site to CDS1 (hepatitis C virus)"

<220>
<221> misc_feature
<222> 179..206
<223> /note="hybridization site to CDS1 (hepatitis C virus)"

<220>
<221> misc_feature
<222> 221..248
<223> /note="hybridization site to CDS1 (hepatitis C virus)"

<220>
<221> misc_feature
<222> 263..290
<223> /note="hybridization site to CDS1 (hepatitis C virus)"

<220>

<221> misc_feature
<222> 305..332
<223> /note="hybridization site to CDS1 (hepatitis C virus)"

<400> 4
cugcacuggg uuugggcgac gguugguguu uuuuuuggac gagauuucuu gcucugggcg      60

acgguuggug uuucuuuugg guguuagugc guacucuggg cgaugguugg uguuucuuuu     120

gguaauuauu acuuucuuug ggcgauggu ggguguuuuu uuggugggu gaaaagaguu     180

ugggcggugg uugguguuuc uuuuggcgug gugaguaaca uuuggguggc gguugguguu     240

uuuuuuggcu gcaacgcacc gguuugggcg acgguuggug uuucuuuugg gggucaaccg     300

ggaaucuggg uggcgguugg uguuuuuuuu ggugauggcc gaggua                   346

<210> 5
<211> 355
<212> RNA
<213> Artificial sequence

<220>
<223> Circular Artificial sequence to IRES1, IRES2 and CDS1 (hepatitis
      C virus) (circ_hcv_combo1)

<220>
<221> misc_feature
<222> 11..43
<223> /note="hybridization site to IRES1 (hepatitis C virus)"

<220>
<221> misc_feature
<222> 54..77
<223> /note="hybridization site to IRES2 (hepatitis C virus)"

<220>
<221> misc_feature
<222> 88..115
<223> /note="hybridization site to CDS1 (hepatitis C virus)"

<220>
<221> misc_feature
<222> 126..158
<223> /note="hybridization site to IRES1 (hepatitis C virus)"

<220>
<221> misc_feature
<222> 169..192
<223> /note="hybridization site to IRES2 (hepatitis C virus)"

<220>
<221> misc_feature
<222> 203..230
<223> /note="hybridization site to CDS1 (hepatitis C virus)"

<220>
<221> misc_feature
<222> 241..273
<223> /note="hybridization site to IRES1 (hepatitis C virus)"

<220>
<221> misc_feature
<222> 284..307
<223> /note="hybridization site to IRES2 (hepatitis C virus)"

<220>
<221> misc_feature
<222> 318..345
<223> /note="hybridization site to IRES2 (hepatitis C virus)"

<400> 5
   gcgccaagua uaguuccuca caggggagug auuuauggug gagaccccua aacgcuugug      60
   gugcacgguc uacgggguac cgagaaguuu gggcgguggu uggguguuuu uuuggcgcuu     120
   gugggugguu uuuugcaggg gagugauuua ugguggaggc aagaguuugc uuauggugua     180
   cggucuguga gaugacauca gguuugggcg gcgguuggug uuuuuuuugg augaggaacc     240
   uaguucuuca uaggggagug auuuguggug gagacagcga guuguuugug gugcacgguu     300
   uacgagaaga augaagaucu ggguggcggu ugguguuucu uuugguagca caugu        355


<210> 6
<211> 288
<212> RNA
<213> Artificial sequence


<220>
<223> Circular Artificial sequence to CDS2 (hepatitis C virus)
      (circ_hcv_cds2)

<220>
<221> misc_feature
<222> 4..14
<223> /note="hybridization site to CDS2 (hepatitis C virus)"

<220>
<221> misc_feature
<222> 28..38
<223> /note="hybridization site to CDS2 (hepatitis C virus)"

<220>
<221> misc_feature
<222> 52..62
<223> /note="hybridization site to CDS2 (hepatitis C virus)"

<220>
<221> misc_feature
<222> 76..86
<223> /note="hybridization site to CDS2 (hepatitis C virus)"

<220>
<221> misc_feature
<222> 100..110
<223> /note="hybridization site to CDS2 (hepatitis C virus)"

<220>
<221> misc_feature
<222> 124..134
<223> /note="hybridization site to CDS2 (hepatitis C virus)"

<220>
<221> misc_feature
<222> 148..158
<223> /note="hybridization site to CDS2 (hepatitis C virus)"

<220>
<221> misc_feature
<222> 172..182
<223> /note="hybridization site to CDS2 (hepatitis C virus)"

<220>
<221> misc_feature
<222> 196..206
<223> /note="hybridization site to CDS2 (hepatitis C virus)"

<220>
<221> misc_feature
<222> 220..230
<223> /note="hybridization site to CDS2 (hepatitis C virus)"

<220>
<221> misc_feature
<222> 244..254
<223> /note="hybridization site to CDS2 (hepatitis C virus)"

<220>
<221> misc_feature
<222> 266..278
<223> /note="hybridization site to CDS2 (hepatitis C virus)"

<400> 6
cccccugggg cucugaugag gaaccucucu ggggucccca cagcgagucu ccuuggggcc     60

ccuagaauga aguucuuugg gguuucauag cacgguucuc cugggguuuu cauacgauug    120

ucucuugggg ucuugcgugu uuacccuuuu gggguccugc uaagggggcu uucuggggcc    180

uuucgaauaa gucuuuuugg ggccucguuu uaucacucuu cugggguucc cagccuuucc    240

cuucuugggg cuccuccgac caugccccuu gggguucuac ccuguaug                 288


<210> 7
<211> 10722
<212> RNA
<213> Dengue virus


<220>
<221> misc_feature
<222> 108..140
<223> /note="target region of cHP"

<220>
<221> misc_feature
<222> 10615..10646
<223> /note="target region of 3UTR"

<400> 7
aguuguuagu cuacguggac cgacaaagac agauucuuug agggagcuaa gcucaacgua      60

guucuaacag uuuuuuaauu agagagcaga ucucugauga auaaccaacg gaaaaaggcg      120

aaaaacacgc cuuucaauau gcugaaacgc gagagaaacc gcgugucgac ugugcaacag 180

cugacaaaga gauucucacu uggaaugcug cagggacgag gaccauuaaa acuguucaug 240

gcccuggugg cguuccuucg uuuccuaaca aucccaccaa cagcagggau auugaagaga 300

uggggaacaa uuaaaaaauc aaaagcuauu aauguuuuga gaggguucag gaaagagauu 360

ggaaggaugc ugaacaucuu gaauaggaga cgcagaucug caggcaugau cauuaugcug 420

auuccaacag ugauggcguu ccauuuaacc acacguaacg gagaaccaca caugaucguc 480

agcagacaag agaaagggaa aagucuucug uuuaaaacag aggauggcgu gaacaugugu 540

acccucaugg ccauggaccu uggugaauug ugugaagaca caaucacgua caaguguccc 600

cuucucaggc agaaugagcc agaagacaua gacuguuggu gcaacucuac guccacgugg 660

guaacuuaug ggacguguac caccauggga gaacauagaa gagaaaaaag aucaguggca 720

cucguuccac augugggaau gggacuggag acacgaacug aaacauggau gucaucagaa 780

ggggccugga aacaugucca gagaauugaa acuuggaucu ugagacaucc aggcuucacc 840

augauggcag caauccuggc auacaccaua ggaacgacac auuuccaaag agcccugauu 900

uucaucuuac ugacagcugu cacuccuuca augacaaugc guugcauagg aaugucaaau 960

agagacuuug uggaaggggu uucaggagga agcuggguug acauagucuu agaacaugga 1020

agcuguguga cgacgauggc aaaaaacaaa ccaacauugg auuuugaacu gauaaaaaca 1080

gaagccaaac agccugccac ccuaaggaag uacuguauag aggcaaagcu aaccaacaca 1140

acaacagaau cucgcugccc aacacaaggg gaacccagcc uaaaugaaga gcaggacaaa 1200

agguucgucu gcaaacacuc caugguagac agaggauggg gaaauggaug uggacuauuu 1260

ggaaagggag gcauugugac cugugcuaug uucagaugca aaaagaacau ggaaggaaaa 1320

guugugcaac cagaaaacuu ggaauacacc auugugauaa caccucacuc aggggaagag 1380

caugcagucg gaaaugacac aggaaaacau ggcaaggaaa ucaaaauaac accacagagu 1440

uccaucacag aagcagaauu gacagguuau ggcacuguca caauggagug cucuccaaga 1500

acgggccucg acuucaauga gaugguguug cugcagaugg aaaauaaagc uuggcuggug 1560

cacaggcaau gguuccuaga ccugccguua ccaugguugc ccggagcgga cacacaaggg 1620

ucaaauugga uacagaaaga gacauugguc acuuucaaaa auccccaugc gaagaaacag 1680

gauguuguug uuuuaggauc ccaagaaggg gccaugcaca cagcacuuac aggggccaca 1740

gaaauccaaa ugucaucagg aaacuuacuc uucacaggac aucucaagug caggcugaga 1800

auggacaagc uacagcucaa aggaauguca uacucuaugu gcacaggaaa guuuaaaguu 1860

gugaaggaaa uagcagaaac acaacaugga acaauaguua ucagagugca auaugaaggg 1920

gacggcucuc caugcaagau cccuuuugag auaauggauu uggaaaaaag acaugucuua 1980

ggucgccuga uuacagucaa cccaauugug acagaaaaag auagcccagu caacauagaa 2040

gcagaaccuc cauucggaga cagcuacauc aucauaggag uagagccggg acaacugaag 2100

cucaacuggu uuaagaaagg aaguucuauc ggccaaaugu uugagacaac aaugaggggg 2160

gcgaagagaa uggccauuuu aggugacaca gccugggauu uuggauccuu gggaggagug 2220

uuuacaucua uaggaaaggc ucuccaccaa gucuuuggag caaucuaugg agcugccuuc 2280

agugggguuu cauggacuau gaaaauccuc auaggaguca uuaucacaug gauaggaaug 2340

aauucacgca gcaccucacu gucugugaca cuaguauugg ugggaauugu gacacuguau 2400

uugggaguca uggugcaggc cgauaguggu ugcguuguga gcuggaaaaa caaagaacug 2460

aaauguggca gugggauuuu caucacagac aacgugcaca cauggacaga acaauacaag 2520

uuccaaccag aauccccuuc aaaacuagcu ucagcuaucc agaaagccca ugaagagggc 2580

auuuguggaa uccgcucagu aacaagacug gagaaucuga uguggaaaca aauaacacca 2640

gaauugaauc acauucuauc agaaaaugag gugaaguuaa cuauuaugac aggagacauc 2700

aaaggaauca ugcaggcagg aaaacgaucu cugcggccuc agcccacuga gcugaaguau 2760

ucauggaaaa cauggggcaa agcaaaaaug cucucuacag agucucauaa ccagaccuuu 2820

cucauugaug gccccgaaac agcagaaugc cccaacacaa auagagcuug gaauucguug 2880

gaaguugaag acuauggcuu uggaguauuc accaccaaua uauggcuaaa auugaaagaa 2940

aaacaggaug uauucugcga cucaaaacuc augucagcgg ccauaaaaga caacagagcc 3000

guccaugccg auauggguua uuggauagaa agugcacuca augacacaug gaagauagag 3060

aaagccucuu ucauugaagu uaaaaacugc cacuggccaa aaucacacac ccucuggagc 3120

aauggagugc uagaaaguga gaugauaauu ccaaagaauc ucgcuggacc agugucucaa 3180

cacaacuaua gaccaggcua ccauacacaa auaacaggac cauggcaucu agguaagcuu 3240

gagauggacu uugauuucug ugauggaaca acagugguag ugacugagga cugcggaaau 3300

agaggacccu cuuugagaac aaccacugcc ucuggaaaac ucauaacaga auggugcugc 3360

cgaucuugca cauuaccacc gcuaagauac agaggugagg augggugcug guacgggaug 3420

gaaaucagac cauugaagga gaaagaagag aauuugguca acuccuuggu cacagcugga 3480

caugggcagg ucgacaacuu uucacuagga gucuugggaa uggcauuguu ccuggaggaa 3540

augcuuagga cccgaguagg aacgaaacau gcaauacuac uaguugcagu uucuuuugug 3600

acauugauca cagggaacau guccuuuaga gaccugggaa gagugauggu uaugguaggc 3660

gccacuauga cggaugacau agguaugggc gugacuuauc uugcccuacu agcagccuuc 3720

aaagucagac caacuuuugc agcuggacua cucuugagaa agcugaccuc caaggaauug 3780

augaugacua cuauaggaau uguacuccuc ucccagagca ccauaccaga gaccauucuu 3840

gaguugacug augcguuagc cuuaggcaug augguccuca aaauggugag aaauauggaa 3900

aaguaucaau uggcagugac uaucauggcu aucuugugcg ucccaaacgc agugauauua 3960

caaaacgcau ggaaagugag uugcacaaua uuggcagugg uguccguuuc cccacugcuc 4020

uuaacauccu cacagcaaaa aacagauugg auaccauuag cauugacgau caaaggucuc 4080

aauccaacag cuauuuuucu aacaacccuc ucaagaacca gcaagaaaag gagcuggcca 4140

uuaaaugagg cuaucauggc agucgggaug gugagcauuu uagccaguuc ucuccuaaaa 4200

aaugauauuc ccaugacagg accauuagug gcuggagggc uccucacugu gugcuacgug 4260

cucacuggac gaucggccga uuuggaacug gagagagcag ccgaugucaa augggaagac 4320

caggcagaga uaucaggaag caguccaauc cugucaauaa caauaucaga agaugguagc 4380

augucgauaa aaaaugaaga ggaagaacaa acacugacca uacucauuag aacaggauug 4440

cuggugaucu caggacuuuu uccuguauca auaccaauca cggcagcagc augguaccug 4500

ugggaaguga agaaacaacg ggccggagua uugugggaug uuccuucacc cccacccaug 4560

ggaaaggcug aacuggaaga uggagccuau agaauuaagc aaaaagggau ucuuggauau 4620

ucccagaucg gagccggagu uuacaaagaa ggaacauucc auacaaugug gcaugucaca 4680

cguggcgcug uucuaaugca uaaaggaaag aggauugaac caucaugggc ggacgucaag 4740

aaagaccuaa uaucauaugg aggaggcugg aaguuagaag gagaauggaa ggaaggagaa 4800

gaaguccagg uauuggcacu ggagccugga aaaaauccaa gagccgucca aacgaaaccu 4860

ggucuuuuca aaaccaacgc cggaacaaua ggugcuguau cucuggacuu uucuccugga 4920

acgucaggau cuccaauuau cgacaaaaaa ggaaaaguug ugggucuuua ugguaauggu 4980

guuguuacaa ggaguggagc auaugugagu gcuauagccc agacugaaaa aagcauugaa 5040

gacaacccag agaucgaaga ugacauuuuc cgaaagagaa gacugaccau cauggaccuc 5100

cacccaggag cgggaaagac gaagagauac cuuccggcca uagucagaga agcuauaaaa 5160

cgggguuuga gaacauuaau cuuggccccc acuagaguug uggcagcuga aauggaggaa 5220

gcccuuagag gacuuccaau aagauaccag accccagcca ucagagcuga gcacaccggg 5280

cgggagauug uggaccuaau gugucaugcc acauuuacca ugaggcugcu aucaccaguu 5340

agagugccaa acuacaaccu gauuaucaug gacgaagccc auuucacaga cccagcaagu 5400

auagcagcua gaggauacau cucaacucga guggagaugg gugaggcagc ugggauuuuu 5460

augacagcca cucccccggg aagcagagac ccauuuccuc agagcaaugc accaaucaua 5520

gaugaagaaa gagaaauccc ugaacguucg uggaauuccg gacaugaaug ggucacggau 5580

uuuaaaggga agacuguuug guucguucca aguauaaaag caggaaauga uauagcagcu 5640

ugccugagga aaaauggaaa gaaagugaua caacucagua ggaagaccuu ugauucugag 5700

uaugucaaga cuagaaccaa ugauugggac uucgugguua caacugacau uucagaaaug 5760

ggugccaauu ucaaggcuga gaggguuaua gaccccagac gcugcaugaa accagucaua 5820

cuaacagaug gugaagagcg ggugauucug gcaggaccua ugccagugac ccacucuagu 5880

gcagcacaaa gaagagggag aauaggaaga aauccaaaaa augagaauga ccaguacaua 5940

uacauggggg aaccucugga aaaugaugaa gacugugcac acuggaaaga agcuaaaaug 6000

cuccuagaua acaucaacac gccagaagga aucauuccua gcauguucga accagagcgu 6060

gaaaaggugg augccauuga uggcgaauac cgcuugagag gagaagcaag gaaaaccuuu 6120

guagacuuaa ugagaagagg agaccuacca gucugguugg ccuacagagu ggcagcugaa 6180

ggcaucaacu acgcagacag aagguggugu uuugauggag ucaagaacaa ccaaauccua 6240

gaagaaaacg uggaaguuga aaucuggaca aaagaagggg aaaggaagaa auugaaaccc 6300

agaugguugg augcuaggau cuauucugac ccacuggcgc uaaaagaauu uaaggaauuu 6360

gcagccggaa gaaagucucu gacccugaac cuaaucacag aaauggguag gcucccaacc 6420

uucaugacuc agaaggcaag agacgcacug gacaacuuag cagugcugca cacggcugag 6480

gcagguggaa gggcguacaa ccaugcucuc agugaacugc cggagacccu ggagacauug 6540

cuuuuacuga cacuucuggc uacagucacg ggagggaucu uuuuauucuu gaugagcgga 6600

aggggcauag ggaagaugac ccugggaaug ugcugcauaa ucacggcuag cauccuccua 6660

ugguacgcac aaauacagcc acacuggaua gcagcuucaa uaauacugga guuuuuucuc 6720

uaguuuugcu uauuccagaa ccugaaaaac agagaacacc ccaagacaac caacugaccu 6780

acguugucau agccauccuc acaguggugg ccgcaaccau ggcaaacgag augguuuucc 6840

uagaaaaaac gaagaaagau cucggauugg gaagcauugc aacccagcaa cccgagagca 6900

acauccugga cauagaucua cguccugcau cagcauggac gcuguaugcc guggccacaa 6960

cauuuguuac accaauguug agacauagca uugaaaauuc cucagugaau gugucccuaa 7020

cagcuauagc caaccaagcc acaguguuaa ugggucucgg gaaaggaugg ccauugucaa 7080

agauggacau cggaguuccc cuucucgcca uuggaugcua cucacaaguc aaccccauaa 7140

cucucacagc agcucuuuuc uuauugguag cacauuaugc caucauaggg ccaggacucc 7200

aagcaaaagc aaccagagaa gcucagaaaa gagcagcggc gggcaucaug aaaaacccaa 7260

cugucgaugg aauaacagug auugaccuag auccaauacc uuaugaucca aaguuugaaa 7320

agcaguuggg acaaguaaug cuccuagucc ucugcgugac ucaaguauug augaugagga 7380

cuacaugggc ucugugugag gcuuuaaccu uagcuaccgg gcccaucucc acauuguggg 7440

aaggaaaucc agggagguuu uggaacacua ccauugcggu gucaauggcu aacauuuuua 7500

gagggaguua cuuggccgga gcuggacuuc ucuuuucuau uaugaagaac acaaccaaca 7560

caagaagggg aacuggcaac auaggagaga cgcuuggaga gaaauggaaa agccgauuga 7620

acgcauuggg aaaaagugaa uuccagaucu acaagaaaag uggaauccag gaaguggaua 7680

gaaccuuagc aaaagaaggc auuaaaagag gagaaacgga ccaucacgcu gugucgcgag 7740

gcucagcaaa acugagaugg uucguugaga gaaacauggu cacaccagaa gggaaaguag 7800

uggaccucgg uuguggcaga ggaggcuggu cauacuauug uggaggacua aagaauguaa 7860

gagaagucaa aggccuaaca aaaggaggac caggacacga agaacccauc cccaugucaa 7920

cauaugggug gaaucuagug cgucuucaaa guggaguuga cguuuucuuc aucccgccag 7980

aaaaguguga cacauuauug ugugacauag gggagucauc accaaauccc acaguggaag 8040

caggacgaac acucagaguc cuuaacuuag uagaaaauug guugaacaac aacacucaau 8100

uuugcauaaa gguucucaac ccauauaugc ccucagucau agaaaaaaug gaagcacuac 8160

aaaggaaaua uggaggagcc uuagugagga auccacucuc acgaaacucc acacaugaga 8220

uguacugggu auccaaugcu uccgggaaca uagugucauc agugaacaug auuucaagga 8280

uguugaucaa cagauuuaca augagauaca agaaagccac uuacgagccg gauguugacc 8340

ucggaagcgg aacccguaac aucgggauug aaagugagau accaaaccua gauauaauug 8400

ggaaaagaau agaaaaaaua aagcaagagc augaaacauc auggcacuau gaccaagacc 8460

acccauacaa aacgugggca uaccauggua gcuaugaaac aaaacagacu ggaucagcau 8520

cauccauggu caacggagug gucaggcugc ugacaaaacc uugggacguc guccccaugg 8580

ugacacagau ggcaaugaca gacacgacuc cauuuggaca acagcgcguu uuuaaagaga 8640

aaguggacac gagaacccaa gaaccgaaag aaggcacgaa gaaacuaaug aaaauaacag 8700

cagaguggcu uuggaaagaa uuagggaaga aaaagacacc caggaugugc accagagaag 8760

aauucacaag aaaggugaga agcaaugcag ccuugggggc cauauucacu gaugagaaca 8820

aguggaaguc ggcacgugag gcuguugaag auaguagguu uugggagcug guugacaagg 8880

aaaggaaucu ccaucuugaa ggaaagugug aaacaugugu guacaacaug augggaaaaa 8940

gagagaagaa gcuaggggaa uucggcaagg caaaaggcag cagagccaua ugguacaugu 9000

ggcuuggagc acgcuucuua gaguuugaag cccuaggauu cuuaaaugaa gaucacuggu 9060

ucuccagaga gaacucccug aguggagugg aaggagaagg gcugcacaag cuagguuaca 9120

uucuaagaga cgugagcaag aaagagggag gagcaaugua ugccgaugac accgcaggau 9180

gggauacaag aaucacacua gaagaccuaa aaaaugaaga aaugguaaca aaccacaugg 9240

aaggagaaca caagaaacua gccgaggcca uuuucaaacu aacguaccaa aacaaggugg 9300

ugcgugugca aagaccaaca ccaagaggca caguaaugga caucauaucg agaagagacc 9360

aaagagguag uggacaaguu ggcaccuaug gacucaauac uuucaccaau auggaagccc 9420

aacuaaucag acagauggag ggagaaggag ucuuuaaaag cauucagcac cuaacaauca 9480

cagaagaaau cgcugugcaa aacugguuag caagaguggg gcgcgaaagg uuaucaagaa 9540

uggccaucag uggagaugau uguguuguga aaccuuuaga ugacagguuc gcaagcgcuu 9600

uaacagcucu aaaugacaug ggaaagauua ggaaagacau acaacaaugg gaaccuucaa 9660

gaggauggaa ugauuggaca caagugcccu ucuguucaca ccauuuccau gaguuaauca 9720

ugaaagacgg ucgcguacuc guuguuccau guagaaacca agaugaacug auuggcagag 9780

cccgaaucuc ccaaggagca ggguggucuu ugcgggagac ggccuguuug gggaagucuu 9840

acgcccaaau guggagcuug auguacuucc acagacgcga ccucaggcug gcggcaaaug 9900

cuauuugcuc ggcaguacca ucacauuggg uuccaacaag ucgaacaacc ugguccauac 9960

augcuaaaca ugaauggaug acaacggaag acaugcugac agucuggaac agggugugga 10020

uucaagaaaa cccauggaug gaagacaaaa cuccagugga aucaugggag gaaaucccau 10080

acuuggggaa aagagaagac caauggugcg gcucauugau uggguuaaca agcagggcca 10140

ccugggcaaa gaacauccaa gcagcaauaa aucaaguuag aucccuuaua ggcaaugaag 10200

aauacacaga uuacaugcca uccaugaaaa gauucagaag agaagaggaa gaagcaggag 10260

uucuguggua gaaagcaaaa cuaacaugaa acaaggcuag aagucagguc ggauuaagcc 10320

auaguacgga aaaaacuaug cuaccuguga gccccgucca aggacguuaa aagaagucag 10380

gccaucauaa augccauagc uugaguaaac uaugcagccu guagcuccac cugagaaggu 10440

guaaaaaauc cgggaggcca caaaccaugg aagcuguacg cauggcguag uggacuagcg 10500

guuagaggag accccucccu uacaaaucgc agcaacaaug ggggcccaag gcgagaugaa 10560

gcuguagucu cgcuggaagg acuagagguu agaggagacc cccccgaaac aaaaaacagc 10620

auauugacgc ugggaaagac cagagauccu gcugucuccu cagcaucauu ccaggcacag 10680

aacgccagaa aauggaaugg ugcuguugaa ucaacagguu cu 10722

<210> 8
<211> 293
<212> RNA
<213> Artificial sequence

<220>
<223> Circular Artificial sequence to 3UTR (Dengue virus)
(circ_dv_3utr)

<220>
<221> misc_feature
<222> 7..38
<223> /note="hybridization site to 3UTR (Dengue virus)"

<220>
<221> misc_feature
<222> 48..79
<223> /note="hybridization site to 3UTR (Dengue virus)"

<220>
<221> misc_feature

<222> 89..120
<223> /note="hybridization site to 3UTR (Dengue virus)"

<220>
<221> misc_feature
<222> 130..161
<223> /note="hybridization site to 3UTR (Dengue virus)"

<220>
<221> misc_feature
<222> 171..202
<223> /note="hybridization site to 3UTR (Dengue virus)"

<220>
<221> misc_feature
<222> 212..243
<223> /note="hybridization site to 3UTR (Dengue virus)"

<220>
<221> misc_feature
<222> 253..284
<223> /note="hybridization site to 3UTR (Dengue virus)"

<400> 8
gcgccgucuu uggucuuuuu uggcgucagu auguuguuuu uuaaccaucu uugguuuuuc      60

cuggcgucag ugugcuguua uaguaugguc ucugguuuuu uuuagcguua gugugcuguu     120

aaagcuuucu cucuggucuu uuccaguguc aauaugcugu uuagugugcu ucuuugguuu     180

uucuuggugu uggugugcug uuucagcaag uucucugguc uuuucuggcg uugauguguu     240

guucgcuggc gaucuuuggu cuuucucggu gucgauaugu uguuaguggc cca            293


<210> 9
<211> 300
<212> RNA
<213> Artificial sequence


<220>
<223> Circular Artificial sequence to ChP (Dengue virus)
      (circ_dv_cHP_v1)

<220>
<221> misc_feature
<222> 7..39
<223> /note="hybridization site to ChP (Dengue virus)"

<220>
<221> misc_feature
<222> 49..81
<223> /note="hybridization site to ChP (Dengue virus)"

<220>
<221> misc_feature
<222> 91..123
<223> /note="hybridization site to ChP (Dengue virus)"

<220>
<221> misc_feature
<222> 133..165

<223> /note="hybridization site to ChP (Dengue virus)"

<220>
<221> misc_feature
<222> 175..207
<223> /note="hybridization site to ChP (Dengue virus)"

<220>
<221> misc_feature
<222> 217..249
<223> /note="hybridization site to ChP (Dengue virus)"

<220>
<221> misc_feature
<222> 259..291
<223> /note="hybridization site to ChP (Dengue virus)"

<400> 9
gcgccgaugu uggggggugu guuuuuuguc uuuuuucguu gggguguaau guugagaggc     60

guguuuuug cuuuuuuug ugcaccuuau auauugggag gugguguuuu cgcuuuuuc    120

cgucuggcug gcauauuggg gggcguguuu uucguuuuuu uucguuacaa gcuaauauug    180

aagggugugu uuuucgccuu uuuccguguu aaagauaugu ugaaaggcgu guuuuucguu    240

uuuuuccgua aguuuuaaau auuggagggc guguuuuucg cuuuuuucug uagaccggaa    300


<210> 10
<211> 294
<212> RNA
<213> Artificial sequence


<220>
<223> Circular Artificial sequence to ChP2 (Dengue virus)
      (circ_dv_cHP_v2)

<220>
<221> misc_feature
<222> 7..39
<223> /note="hybridization site to ChP2 (Dengue virus)"

<220>
<221> misc_feature
<222> 55..87
<223> /note="hybridization site to ChP2 (Dengue virus)"

<220>
<221> misc_feature
<222> 103..135
<223> /note="hybridization site to ChP2 (Dengue virus)"

<220>
<221> misc_feature
<222> 151..183
<223> /note="hybridization site to ChP2 (Dengue virus)"

<220>
<221> misc_feature
<222> 199..231
<223> /note="hybridization site to ChP2 (Dengue virus)"

<220>
<221> misc_feature
<222> 247..279
<223> /note="hybridization site to ChP2 (Dengue virus)"

<400> 10
gcgccgaugu ugagaggugu guuuuuugcu uuuuuucguu uauuacucca acagauauug 60

agaggcgugu uuuucgcuuu uuuucguaga agguaauaga aaauauuggg gggcguguuu 120

uucgucuuuu uucgugacuc uuacgaguuu auguugaaag gugugguuuu cgcuuuuuuu 180

uguagggaac agucgcaaau auuggggggu guguuuuucg ccuuuuuucg uggcaccgua 240

auccgcaugu ugaaaggugu guuuuucguu uuuuuccguc aacucguucu uaua 294

<210> 11
<211> 11840
<212> RNA
<213> Chikungunya virus

<220>
<221> misc_feature
<222> 14..48
<223> /note="target region of 5UTR"

<220>
<221> misc_feature
<222> 11367..11399
<223> /note="target region of RSE"

<400> 11
auggcugcgu gagacacacg uagccuacca guuucuuacu gcucuacucu gcaaagcaag 60

agauuaauaa cccaucaugg auccugugua cguggacaua gacgcugaca gcgccuuuuu 120

gaaggcccug caacgugcgu accccauguu ugagguggaa ccaaggcagg ucacaccgaa 180

ugaccaugcu aaugcuagag cguucucgca ucuagcuaua aaacuaauag agcaggaaau 240

ugaccccgac ucaaccaucc uggauaucgg cagugcgcca gcaaggagga ugaugucgga 300

caggaaguac cacugcgucu gcccgaugcg cagugcggaa gaucccgaga gacucgccaa 360

uuaugcgaga aagcuagcau cugccgcagg aaaaguccug gacagaaaca ucucuggaaa 420

gaucggggac uuacaagcag uaauggccgu gccagacacg gagacgccaa cauucugcuu 480

acacacagac gucucaugua gacagagagc agacgucgcu auauaccaag acgucuaugc 540

uguacacgca cccacgucgc uauaccacca ggcgauuaaa gggguccgag uggcguacug 600

gguuggguuc gacacaaccc cguucaugua caaugccaug gcgggugccu accccucaua 660

cucgacaaac ugggcagaug agcagguacu gaaggcuaag aacauaggau uauguucaac 720

agaccugacg gaagguagac gaggcaaguu gucuauuaug agagggaaaa agcuaaaacc 780

gugcgaccgu gugcuguucu caguaggguc aacgcucuac ccggaaagcc gcaagcuacu 840

uaagagcugg caccugccau cgguguucca uuuaaagggc aaacucagcu ucacaugccg 900

cugugauaca gugguuucgu gugagggcua cgucguuaag agaauaacga ugagcccagg 960

ccuuuaugga aaaaccacag gguaugcggu aacccaccac gcagacggau uccugaugug 1020

caagacuacc gacacgguug acggcgaaag arugucauuc ucggugugca cauacgugcc 1080

ggcgaccauu ugugaucaaa ugaccggcau ccuugcuaca gaagucacgc cggaggaugc 1140

acagaagcug uugguggggc ugaaccagag aauagugguu aacggcagaa cgcaacggaa 1200

uacgaacacc augaaaaauu aucugcuucc cguggucgcc caagccuuca guaagugggc 1260

aaaggagugc cggaaagaca uggaagauga aaaacuccug ggggucagag aaagaacacu 1320

gaccugcugc ugucuauggg cauucaagaa gcagaaaaca cacacggucu acaagaggcc 1380

ugauacccag ucaauucaga agguucaggc cgaguuugac agcuuugugg uaccgagucu 1440

guggucgucc ggguugucaa ucccuuugag gacuagaauc aaaugguugu uaagcaaggu 1500

gccaaaaacc gaccugaucc cauacagcgg agacgcccga gaagcccggg acgcagaaaa 1560

agaagcagag gaagaacgag aagcagaacu gacucgcgaa gcccuaccac cucuacaggc 1620

agcacaggaa gauguucagg ucgaaaucga cguggaacag cuugaggaca gagcgggcgc 1680

aggaauaaua gagacuccga gaggagcuau caaaguuacu gcccaaccaa cagaccacgu 1740

cgugggagag uaccugguac ucuccccgca gaccguacua cguagccaga agcucagucu 1800

gauucacgcu uuggcggagc aagugaagac gugcacgcac aacggacgag cagggaggua 1860

ugcggucgaa gcguacgacg gccgaguccu agugcccuca ggcuaugcaa ucucgccuga 1920

agacuuccag agucuaagcg aaagcgcaac gaugguguau aacgaaagag aguucguaaa 1980

cagaaagcua caccauauug cgaugcacgg accagcccug aacaccgacg aagagucgua 2040

ugagcuggug agggcagaga ggacagaaca cgaguacguc uacgacgugg aucagagaag 2100

augcuguaag aaggaagaag ccgcaggacu gguacuggug ggcgacuuga cuaauccgcc 2160

cuaccacgaa uucgcauaug aagggcuaaa aauccgcccu gccugcccau acaaaauugc 2220

agucauagga gucuucggag uaccgggauc uggcaaguca gcuauuauca agaaccuagu 2280

uaccaggcag gaccugguga cuagcggaaa gaaagaaaac ugccaagaaa ucaccaccga 2340

cgugaugaga cagagagguc uagagauauc ugcacguacg guugacucgc ugcucuugaa 2400

uggaugcaac agaccagucg acguguugua cguagacgag gcguuugcgu gccacucugg 2460

aacgcuacuu gcuuugaucg ccuuggugag accaaggcag aaaguuguac uuugugguga 2520

cccgaagcag ugcggcuucu ucaauaugau gcagaugaaa gucaacuaua aucacaacau 2580

cugcacccaa guguaccaca aaaguaucuc caggcggugu acacugccug ugaccgccau 2640

ugugucaucg uugcauuacg aaggcaaaau gcgcacuacg aaugaguaca acaagccgau 2700

uguaguggac acuacaggcu caacaaaacc ugacccugga gaccucgugu uaacgugcuu 2760

cagagggugg guuaaacaac ugcaaauuga cuaucgugga uacgagguca ugacagcagc 2820

cgcaucccaa ggguuaacca gaaaaggagu uuacgcaguu agacaaaaag uuaaugaaaa 2880

cccgcucuau gcaucaacgu cagagcacgu caacguacuc cuaacgcgua cggaagguaa 2940

acugguaugg aagacacuuu ccggcgaccc guggauaaag acgcugcaga acccaccgaa 3000

aggaaacuuc aaagcaacua uuaaggagug ggagguggag caugcaucaa uaauggcggg 3060

caucugcagu caccaaauga ccuucgauac auuccaaaau aaagccaacg uuuguugggc 3120

uaagagcuug gucccuaucc ucgaaacagc ggggauaaaa cuaaaugaua ggcagugguc 3180

ucagauaauu caagccuuca aagaagacaa agcauacuca ccugaaguag cccugaauga 3240

aauauguacg cgcauguaug ggguggaucu agacagcggg cuauuuucua aaccguuggu 3300

gucuguguau uacgcggaua accacuggga uaauaggccu ggagggaaaa uguucggauu 3360

uaaccccgag gcagcaucca uucuagaaag aaaguaucca uucacaaaag ggaaguggaa 3420

caucaacaag cagaucugcg ugacuaccag gaggauagaa gacuuuaacc cuaccaccaa 3480

caucauaccg gccaacagga gacuaccaca cucauuagug gccgaacacc gcccaguaaa 3540

aggggaaaga auggaauggc ugguuaacaa gauaaacggc caccacgugc uccuggucag 3600

uggcuauaac cuugcacugc cuacuaagag agucacuugg guagcgccgu uagguguccg 3660

cggagcggac uacacauaca accuagaguu gggucugcca gcaacgcuug guagguauga 3720

ccuagugguc auaaacaucc acacaccuuu ucgcauacac cauuaccaac agugcgucga 3780

ccacgcaaug aaacugcaaa ugcucggggg ugacucauug agacugcuca aaccgggcgg 3840

cucucuauug aucagagcau augguuacgc agauagaacc agugaacgag ucaucugcgu 3900

auugggacgc aaguuuagau cgucuagagc guugaaacca ccauguguca ccagcaacac 3960

ugagauguuu uuccuauuca gcaacuuuga caauggcaga aggaauuuca caacucaugu 4020

caugaacaau caacugaaug cagccuucgu aggacagguc acccgagcag gaugugcacc 4080

gucguaccgg guaaaacgca uggacaucgc gaagaacgau gaagagugcg uagucaacgc 4140

cgcuaacccu cgcggguuac cgggugrcgg uguuugcaag gcaguauaca aaaaauggcc 4200

ggaguccuuu aagaacagug caacaccagu gggaaccgca aaaacaguua ugugcgguac 4260

guauccagua auccacgcug uuggaccaaa cuucucuaau uauucggagu cugaagggga 4320

ccgggaauug gcagcugccu aucgagaagu cgcaaaggaa guaacuaggc ugggaguaaa 4380

uaguguagcu auaccucucc ucuccacagg uguauacuca ggagggaaag acaggcugac 4440

ccagucacug aaccaccucu uuacagccau ggacucgacg gaugcagacg uggucaucua 4500

cugccgcgac aaagaauggg agaagaaaau aucugaggcc auacagaugc ggacccaagu 4560

agagcugcug gaugagcaca ucuccauaga cugcgauauu guucgcgugc acccugacag 4620

cagcuuggca ggcagaaaag gauacagcac cacggaaggc gcacuguacu cauaucuaga 4680

agggacccgu uuucaucaga cggcugugga uauggcggag auacauacua uguggccaaa 4740

gcaaacagag gccaaugagc aagucugccu auaugcccug gggaaaguaa uugaaucgau 4800

caggcagaaa ugcccggugg augaugcaga cgcaucaucu ccccccaaaa cugucccgug 4860

ccuuugccgu uacgcuauga cuccagaacg cgucacccgg cuucgcauga accacgucac 4920

aagcauaauu guguguucuu cguuuccccu cccaaaguac aaaauagaag gagugcaaaa 4980

agucaaaugc ucuaagguaa ugcuauuuga ccacaacgug ccaucgcgcg uaaguccaag 5040

ggaauauaka ucuucccagg agucugcaca ggaggcgagu acaaucacgu cacugacgca 5100

uagucaauuc gaccuaagcg uugauggcga gauacugccc gucccgucag accuggaugc 5160

ugacgcccca gcccuagaac cagcacuaga cgacggggcg acacacacgc ugccauccac 5220

aaccggaaac cuugcggccg ugucugauug gguaaugagc accguaccug ucgcgccgcc 5280

cagaagaagg cgagggagaa accugacugu gacaugugac gagagagaag ggaauauaac 5340

acccauggcu agcguccgau ucuuuagggc agagcugugu ccggucguac aagaaacagc 5400

ggagacgcgu gacacagcaa ugucucuuca ggcaccaccg aguaccgcca cggaaccgaa 5460

ucauccgccg aucuccuucg gagcaucaag cgagacguuc cccauuacau uuggggacuu 5520

caacgaagga gaaaucgaaa gcuugucuuc ugagcuacua acuuucggag acuucuuacc 5580

aggagaagug gaugacuuga cagacagcga cugguccacg ugcucagaca cggacgacga 5640

guuaugacua gacagggcag guggguauau auucucgucg gacaccgguc caggucauuu 5700

acaacagaag ucaguacgcc agucagugcu gccggugaac acccuggagg aaguccacga 5760

ggagaagugu uacccaccua agcuggauga agcaaaggag caacuauuac uuaagaaacu 5820

ccaggagagu gcauccaugg ccaacagaag cagguaucag ucgcgcaaag uagaaaacau 5880

gaaagcagca aucauccaga gacuaaagag aggcuguaga cuauacuuaa ugucagagac 5940

cccaaaaguc ccuacuuacc ggacuacaua uccggcgccu guguacucgc cuccgaucaa 6000

cguccgauug uccaaucccg aguccgcagu ggcagcaugc aaugaguucu uagcuagaaa 6060

cuauccaacu gucucaucau accaaauuac cgacgaguau gaugcauauc uagacauggu 6120

ggacgggucg gagaguugcc uggaccgagc gacauucaau ccgucaaaac ucaggagcua 6180

cccgaaacag cacgcuuacc acgcgcccuc caucagaagc gcuguaccgu ccccauucca 6240

gaacacacua cagaauguac uggcagcagc cacgaaaaga aacugcaacg ucacacagau 6300

gagggaauua cccacuuugg acucagcagu auucaacgug gaguguuuca aaaaauucgc 6360

augcaaccaa gaauacuggg aagaauuugc ugccagcccu auuaggauaa caacugagaa 6420

uuuagcaacc uauguuacua aacuaaaagg gccaaaagca gcagcgcuau ucgcaaaaac 6480

ccauaaucua cugccacuac aggaaguacc aauggauagg uucacaguag auaugaaaag 6540

ggacguaaag gugacuccug guacaaagca uacagaggaa agaccuaagg ugcagguuau 6600

acaggcggcu gaacccuugg cgacagcaua ccuauguggg auucacagag agcugguuag 6660

gaggcugaac gccguccucc uacccaaugu acauacacua uuugacaugu cugccgagga 6720

uuucgaugcc aucauagccg cacacuuuaa gccaggagac acuguuuugg aaacggacau 6780

agccuccuuu gauaagagcc aagaugauuc acuugcgcuu acugcuuuga ugcuguuaga 6840

ggauuuaggg guggaucacu cccugcugga cuugauagag gcugcuuucg gagagauuuc 6900

cagcugucac cuaccgacag guacgcgcuu caaguucggc gccaugauga aaucagguau 6960

guuccuaacu cuguucguca acacauuguu aaacaucacc aucgccagcc gagugcugga 7020

agaucgucug acaaaauccg cgugcgcggc cuucaucggc gacgacaaca uaauacaugg 7080

agucgucucc gaugaauuga uggcagccag augugccacu uggaugaaca uggaagugaa 7140

gaucauagau gcaguuguau ccuugaaagc cccuuacuuu uguggagggu uuauacugca 7200

cgauacugug acaggaacag cuugcagagu ggcagacccg cuaaaaaggc uuuuuaaacu 7260

gggcaaaccg cuagcggcag gugacgaaca agaugaagau agaagacgag cgcuggcuga 7320

cgaagugauc agauggcaac gaacagggcu aauugaugag cuggagaaag cgguauacuc 7380

uagguacgaa gugcagggua uaucaguugu gguaaugucc auggccaccu uugcaagcuc 7440

cagauccaac uucgagaagc ucagaggacc cgucauaacu uuguacggcg guccuaaaua 7500

gguacgcacu acagcuaccu auuuugcaga agccgacagc aaguaucuaa acacuaauca 7560

gcuacaaugg aguucauccc aacccaaacu uuuuacaaua ggagguacca gccucgaccc 7620

uggacuccgc gcccuacuau ccaagucauc aggcccagac cgcgcccuca gaggcaagcu 7680

gggcaacuug cccagcugau cucagcaguu aauaaacuga caaugcgcgc gguaccccaa 7740

cagaagccac gcaggaaucg gaagaauaag aagcaaaagc aaaaacaaca ggcgccacaa 7800

aacaacacaa aucaaaagaa gcagccaccu aaaaagaaac cggcucaaaa gaaaaagaag 7860

ccgggccgca gagagaggau gugcaugaaa aucgaaaaug auuguauuuu cgaagucaag 7920

cacgaaggua agguaacagg uuacgcgugc cugguggggg acaaaguaau gaaaccagca 7980

cacguaaagg ggaccaucga uaacgcggac cuggccaaac uggccuuuaa gcggucaucu 8040

aaguaugacc uugaaugcgc gcagauaccc gugcacauga aguccgacgc uucgaaguuc 8100

acccaugaga aaccggaggg guacuacaac uggcaccacg gagcaguaca guacucagga 8160

ggccgguuca ccaucccuac aggugcuggc aaaccagggg acagcggcag accgaucuuc 8220

gacaacaagg gacgcguggu ggccauaguc uuaggaggag cuaaugaagg agcccguaca 8280

gcccucucgg uggugaccug gaauaaagac auugucacua aaaucacccc cgagggggcc 8340

gaagagugga gucuugccau cccaguuaug ugccuguugg caaacaccac guuccccugc 8400

ucccagcccc cuugcacgcc cugcugcuac gaaaaggaac cggaggaaac ccuacgcaug 8460

cuugaggaca acgucaugag accuggguac uaucagcugc uacaagcauc cuuaacaugu 8520

ucuccccacc gccagcgacg cagcaccaag gacaacuuca augucuauaa agccacaaga 8580

ccauacuuag cucacugucc cgacugugga gaagggcacu cgugccauag ucccguagca 8640

cuagaacgca ucagaaauga agcgacagac gggacgcuga aaauccaggu cuccuugcaa 8700

aucggaauaa agacggauga cagccacgau uggaccaagc ugcguuauau ggacaaccac 8760

augccagcag acgcagagag ggcggggcua uuuguaagaa caucagcacc guguacgauu 8820

acuggaacaa ugggacacuu cauccuggcc cgauguccaa aaggggaaac ucugacggug 8880

ggauucacug acaguaggaa gauuagucac ucauguacgc acccauuuca ccacgacccu 8940

ccugugauag gucgggaaaa auuccauucc cgaccgcagc acgguaaaga gcuaccuugc 9000

agcacguacg ugcagagcac cgccgcaacu accgaggaga uagagguaca caugccccca 9060

gacaccccug aucgcacauu aaugucacaa caguccggca acguaaagau cacagucaau 9120

ggccagacgg ugcgguacaa guguaauugc gguggcucaa augaaggacu aacaacuaca 9180

gacaaaguga uuaauaacug caagguugau caaugucaug ccgcggucac caaucacaaa 9240

aaguggcagu auaacucccc ucuggucccg cguaaugcug aacuugggga ccgaaaagga 9300

aaaauucaca ucccguuucc gcuggcaaau guaacaugca gggugccuaa agcaaggaac 9360

cccaccguga cguacgggaa aaaccaaguc aucaugcuac uguauccuga ccacccaaca 9420

cuccuguccu accggaauau gggagaagaa ccaaacuauc aagaagagug ggugaugcau 9480

aagaaggaag ucgugcuaac cgugccgacu gaagggcucg aggucacgug gggcaacaac 9540

gagccguaua aguauuggcc gcaguuaucu acaaacggua cagcccaugg ccacccgcau 9600

gagauaauuc uguauuauua ugagcuguac cccacuauga cuguaguagu ugugucagug 9660

gccacguuca uacuccuguc gauggugggu auggcagcgg ggaugugcau gugugcacga 9720

cgcagaugca ucacaccgua ugaacugaca ccaggagcua ccgucccuuu ccugcuuagc 9780

cuaauaugcu gcaucagaac agcuaaagcg gccacauacc aagaggcugc gauauaccug 9840

uggaacgagc agcaaccuuu guuuuggcua caagcccuua uuccgcuggc agcccugauu 9900

guucuaugca acugucugag acucuuacca ugcugcugua aaacguuggc uuuuuuagcc 9960

guaaugagcg ucggugccca cacugugagc gcguacgaac acguaacagu gaucccgaac 10020

acggugggag uaccguauaa gacucuaguc aauagaccug gcuacagccc caugguauug 10080

gagauggaac uacugucagu cacuuuggag ccaacacuau cgcuugauua caucacgugc 10140

gaguacaaaa ccgucauccc gucuccguac gugaagugcu gcgguacagc agagugcaag 10200

gacaaaaacc uaccugacua cagcuguaag gucuucaccg gcgucuaccc auuuaugugg 10260

ggcggcgccu acugcuucug cgacgcugaa aacacgcagu ugagcgaagc acacguggag 10320

aaguccgaau caugcaaaac agaauuugca ucagcauaca gggcucauac cgcaucugca 10380

ucagcuaagc uccgcguccu uuaccaagga aauaacauca cuguaacugc cuaugcaaac 10440

ggcgaccaug ccgucacagu uaaggacgcc aaauucauug uggggccaau gucuucagcc 10500

uggacaccuu ucgacaacaa aauuguggug uacaaaggug acgucuauaa cauggacuac 10560

ccgcccuuug gcgcaggaag accaggacaa uuuggcgaua uccaaagucg cacaccugag 10620

aguaaagacg ucuaugcuaa uacacaacug guacugcaga gaccggcugu ggguacggua 10680

cacgugccau acucucaggc accaucuggc uuuaaguauu ggcuaaaaga acgcggggcg 10740

ucgcugcagc acacagcacc auuuggcugc caaauagcaa caaacccggu aagagcggug 10800

aacugcgccg uagggaacau gcccaucucc aucgacauac cggaagcggc cuucacuagg 10860

gucgucgacg cgcccucuuu aacggacaug ucgugcgagg uaccagccug cacccauucc 10920

ucagacuuug ggggcgucgc cauuauuaaa uaugcagcca gcaagaaagg caagugugcg 10980

gugcauucga ugacuaacgc cgucacuauu cgggaagcug agauagaagu ugaagggaau 11040

ucucagcugc aaaucucuuu cucgacggcc uuagccagcg ccgaauuccg cguacaaguc 11100

uguucuacac aaguacacug ugcagccgag ugccaccccc cgaaggacca cauagucaac 11160

uacccggcgu cacauaccac ccucgggguc caggacaucu ccgcuacggc gaugucaugg 11220

gugcagaaga ucacgggagg ugugggacug guuguugcug uugccgcacu gauucuaauc 11280

guggugcuau gcgugucguu cagcaggcac uaacuugaca auuaaguaug aagguauaug 11340

uguccccuaa gagacacacu guacauagca aauaaucuau agaucaaagg gcuacgcaac 11400

cccugaauag uaacaaaaua caaaaucacu aaaaauuaua aaaacagaaa aauacauaaa 11460

uagguauacg uguccccuaa gagacacauu guauguaggu gauaaguaua gaucaaaggg 11520

ccgaauaacc ccugaauagu aacaaaauau gaaaaucaau aaaaaucaua aaauagaaaa 11580

accauaaaca gaaguaguuc aaagggcuau aaaaccccug aauaguaaca aaacauaaaa 11640

uuaauaaaaa ucaaaugaau accauaauug gcaaacggaa gagauguagg uacuuaagcu 11700

uccuaaaagc agccgaacuc acuuugagaa guaggcauag cauaccgaac ucuuccacga 11760

uucuccgaac ccacagggac guaggagaug uuauuuuguu uuuaauauuu caaaaaaaaa 11820

aaaaaaaaaa aaaaaaaaaa 11840

<210> 12
<211> 294
<212> RNA
<213> Artificial sequence

<220>
<223> Circular Artificial sequence to 5UTR (Chikungunya virus)
      (chikv_5utr1)

<220>
<221> misc_feature
<222> 7..41
<223> /note="hybridization site to 5UTR (Chikungunya virus)"

<220>
<221> misc_feature
<222> 55..89
<223> /note="hybridization site to 5UTR (Chikungunya virus)"

<220>
<221> misc_feature
<222> 103..137
<223> /note="hybridization site to 5UTR (Chikungunya virus)"

<220>
<221> misc_feature
<222> 151..185
<223> /note="hybridization site to 5UTR (Chikungunya virus)"

<220>
<221> misc_feature
<222> 199..233
<223> /note="hybridization site to 5UTR (Chikungunya virus)"

<220>
<221> misc_feature
<222> 247..281
<223> /note="hybridization site to 5UTR (Chikungunya virus)"

<400> 12
gcgccgagua gagcagugag gaacuggugg guugcgugug uuuugucacu acguaguaga      60

gugguaggaa auuggugggc ugcgugugug gcuuccagug ccaguagagc ggugagagau     120

ugguagguua uguguguguu cucgcccgaa aguagagugg ugagggguug guagguuacg     180

uguguucaaa uaugacggag uagaguagug gggggcuggu ggguugugug uguucggaga     240

ccagacagua gaguaguaag agguuggugg gcuacgugug ugccacacca cccg           294


<210> 13
<211> 314
<212> RNA
<213> Artificial sequence


<220>
<223> Circular Artificial sequence to 5UTR (Chikungunya virus)
      (chikv_5utr2)

<220>
<221> misc_feature
<222> 7..41
<223> /note="hybridization site to 5UTR (Chikungunya virus)"

<220>
<221> misc_feature
<222> 51..85
<223> /note="hybridization site to 5UTR (Chikungunya virus)"

<220>
<221> misc_feature
<222> 95..129
<223> /note="hybridization site to 5UTR (Chikungunya virus)"

<220>
<221> misc_feature
<222> 139..173
<223> /note="hybridization site to 5UTR (Chikungunya virus)"

<220>
<221> misc_feature
<222> 183..217
<223> /note="hybridization site to 5UTR (Chikungunya virus)"

<220>
<221> misc_feature
<222> 227..261
<223> /note="hybridization site to 5UTR (Chikungunya virus)"

<220>
<221> misc_feature
<222> 271..305
<223> /note="hybridization site to 5UTR (Chikungunya virus)"

<400> 13
gcgccgagua gagugguggg ggguugguag guugcgugug ucuuagcgcc aguagagcag 60

uaagggacug guaggcugcg ugugugguac uuacaguaga gcggugagag acuggugggc 120

uaugugugug ccauccuuag uagaguggua gggaguuggu gggcugugug uguggggccu 180

auaguagagu agugggagau ugguagguua cguguguagc ugcacgagua gagcaguggg 240

aaguuggugg guugugugug ucuccgacga aguagaguag uaagaaauug guggguuaug 300

uguguaccug aguc 314


<210> 14
<211> 288
<212> RNA
<213> Artificial sequence


<220>
<223> Circular Artificial sequence to RSE (Chikungunya virus)
      (chikv_RSE1)

<220>
<221> misc_feature
<222> 7..39
<223> /note="hybridization site to RSE (Chikungunya virus)"

<220>
<221> misc_feature
<222> 54..86
<223> /note="hybridization site to RSE (Chikungunya virus)"

<220>
<221> misc_feature
<222> 101..133
<223> /note="hybridization site to RSE (Chikungunya virus)"

<220>
<221> misc_feature
<222> 148..180
<223> /note="hybridization site to RSE (Chikungunya virus)"

<220>
<221> misc_feature
<222> 195..227
<223> /note="hybridization site to RSE (Chikungunya virus)"

<220>
<221> misc_feature
<222> 242..274
<223> /note="hybridization site to RSE (Chikungunya virus)"

<400> 14
   gcgccguugc guggcucuuu ggucuauaga uuauuuguuu gcggguugcc cucuugcgug 60
   guucuuuggu cuguggauug uuugcuugcc cgcgagcagg uuguguaguc cuuugaucug 120
   uagguuguuu guuguucgua acacuucuug cguagcucuu ugaucuauag guuauuugcu 180
   cuagguucga accguugcgu ggucuuuuga ucuauggguu guuuguucau caaaaccuuu 240
   cuuguguggc cuuuuggucu guagauuguu uguuuccaac gacguacu 288


<210> 15
<211> 288
<212> RNA
<213> Artificial Sequence


<220>
<223> Circular Artificial sequence to RSE2 (Chikungunya virus)
      (chikvRSE)

<220>
<221> misc_feature
<222> 7..39
<223> /note="hybridization site to RSE2 (Chikungunya virus)"

<220>
<221> misc_feature
<222> 54..86
<223> /note="hybridization site to RSE2 (Chikungunya virus)"

<220>
<221> misc_feature
<222> 101..133
<223> /note="hybridization site to RSE2 (Chikungunya virus)"

<220>
<221> misc_feature
<222> 148..180
<223> /note="hybridization site to RSE2 (Chikungunya virus)"

<220>
<221> misc_feature
<222> 195..227
<223> /note="hybridization site to RSE2 (Chikungunya virus)"

<220>
<221> misc_feature
<222> 242..274
<223> /note="hybridization site to RSE2 (Chikungunya virus)"

<400> 15
gcgccguugc guggcucuuu ggucuauggg uuguuugcuu gcggguugcc cucuugugua      60

gcuuuuugau cuguggguua uuugcuugcc cgcgagcagg uugcguaguu cuuugauuug      120

uagguuguuu gcuguucgua acacuucuug cguaguuuuu ugaucuaugg auuguuugcu      180

cuagguucga accguugcgu agcucuuugg uuuauagauu auuugcucau caaaaccuuu      240

cuuguguggu ccuuugaucu auagguuauu ugcuuccaac gacguacu                  288


<210> 16
<211> 620
<212> RNA
<213> Artificial sequence



<220>
<223> Broadspectrum circular RNA to hepatitis C virus and Dengue virus
      (DENV1 cHP_ HCV CDS2_T)

<220>
<221> misc_feature
<222> 16..48
<223> /note="hybridization site to chp (Dengue virus)"

<220>
<221> misc_feature
<222> 58..90
<223> /note="hybridization site to chp (Dengue virus)"

<220>
<221> misc_feature
<222> 100..132
<223> /note="hybridization site to chp (Dengue virus)"

<220>
<221> misc_feature
<222> 142..174
<223> /note="hybridization site to chp (Dengue virus)"

<220>
<221> misc_feature
<222> 184..216
<223> /note="hybridization site to chp (Dengue virus)"

<220>
<221> misc_feature
<222> 226..258
<223> /note="hybridization site to chp (Dengue virus)"

<220>
<221> misc_feature
<222> 268..300
<223> /note="hybridization site to chp (Dengue virus)"

<220>

<221> misc_feature
<222> 330..340
<223> /note="hybridization site to CDS2 (hepatitis C virus)"

<220>
<221> misc_feature
<222> 354..364
<223> /note="hybridization site to CDS2 (hepatitis C virus)"

<220>
<221> misc_feature
<222> 378..388
<223> /note="hybridization site to CDS2 (hepatitis C virus)"

<220>
<221> misc_feature
<222> 402..412
<223> /note="hybridization site to CDS2 (hepatitis C virus)"

<220>
<221> misc_feature
<222> 426..436
<223> /note="hybridization site to CDS2 (hepatitis C virus)"

<220>
<221> misc_feature
<222> 450..460
<223> /note="hybridization site to CDS2 (hepatitis C virus)"

<220>
<221> misc_feature
<222> 474..484
<223> /note="hybridization site to CDS2 (hepatitis C virus)"

<220>
<221> misc_feature
<222> 498..508
<223> /note="hybridization site to CDS2 (hepatitis C virus)"

<220>
<221> misc_feature
<222> 522..532
<223> /note="hybridization site to CDS2 (hepatitis C virus)"

<220>
<221> misc_feature
<222> 546..556
<223> /note="hybridization site to CDS2 (hepatitis C virus)"

<220>
<221> misc_feature
<222> 570..580
<223> /note="hybridization site to CDS2 (hepatitis C virus)"

<220>
<221> misc_feature
<222> 594..604
<223> /note="hybridization site to CDS2 (hepatitis C virus)"

<400> 16
ggauccgcgg cgccgauguu ggggggugug uuuuuugucu uuuuucguug ggguguaaug      60

uugagaggcg uguuuuuugc uuuuuuuugu gcaccuuaua uauugggagg uguguuuuuc      120

gcuuuuuucc gucuggcugg cauauugggg ggcguguuuu ucguuuuuuu ucguuacaag     180

cuaauauuga aggguguguu uuucgccuuu uuccguguua aagauauguu gaaaggcgug     240

uuuuucguuu uuuuccguaa guuuuaaaua uuggagggcg uguuuuucgc uuuuuucugu     300

agaccggaac cgggaauuca cucgagcccc cuggggcucu gaugaggaac cucucugggg     360

uccccacagc gagucuccuu ggggccccua gaaugaaguu cuuugggguu ucauagcacg     420

guucuccugg gguuuucaua cgauugucuc uuggggucuu gcguguuuac ccuuuugggg     480

uccugcuaag ggggcuuucu ggggccuuuc gaauaagucu uuuuggggcc ucguuuuauc     540

acucuucugg gguucccagc cuuucccuuc uuggggcucc uccgaccaug ccccuugggg     600

uucuacccug uaugggaucc                                                620

<210> 17
<211> 298
<212> RNA
<213> Artificial sequence

<220>
<223> Broadspectrum circular RNA to hepatitis C virus and Dengue virus
      (DENV1 cHP_ HCV CDS2_1)

<220>
<221> misc_feature
<222> 7..22
<223> /note="hybridization site to CDS2(2) (hepatitis C virus)"

<220>
<221> misc_feature
<222> 35..67
<223> /note="hybridization site to chp (Dengue virus)"

<220>
<221> misc_feature
<222> 80..95
<223> /note="hybridization site to CDS2(2) (hepatitis C virus)"

<220>
<221> misc_feature
<222> 108..140
<223> /note="hybridization site to chp (Dengue virus)"

<220>
<221> misc_feature
<222> 153..168
<223> /note="hybridization site to CDS2(2) (hepatitis C virus)"

<220>
<221> misc_feature
<222> 181..213
<223> /note="hybridization site to chp (Dengue virus)"

<220>
<221> misc_feature
<222> 226..241

<223> /note="hybridization site to CDS2(2) (hepatitis C virus)"

<220>
<221> misc_feature
<222> 254..286
<223> /note="hybridization site to chp (Dengue virus)"

<400> 17
gcgccgccug gcuugggguu uucccaggac ucuaauauug gaaggcgugu uuuuugucuu     60

uuuucguguc cugccuccuc uuagccuggg guuuuacgac cgcaccuaug uugagaggug    120

uguuuuuugc cuuuuuucgu uugauacccu cauuuaaucu ggggcucuag gugcagcaaa    180

auguuggagg gcguguuuuu cguuuuuuuc uguccugcac gaucucccga uuugggguuc    240

ucguugguga cguauauuga gggguguguu uuucgcuuuu uuucguauug gacgucgu      298


<210> 18
<211> 41
<212> RNA
<213> Artificial Sequence


<220>
<223> Artificial sequence for showing an squematic example of the mode
      of action according to figure 4 wherein the sequence has no
      particular value

<400> 18
aacgccaugc aagcgcgcag aucgagcugu gcgcuuuuuu u                        41


<210> 19
<211> 299
<212> RNA
<213> Artificial sequence


<220>
<223> circ_wnv_sIII_2

<400> 19
ugcaguuaga uaaacuuucc gguuugauuu ucucuucaaa agacaguucu ucgaacuucc     60

cggcuugguu uucuuuucaa aaauggggcc ccucaacuuc cuggucuggu uuucuccuua    120

aaaggcuaua cgaucaacuu ucuggccuga cuuucuucuu aaaaagcuau caucgcaacu    180

ucucggcuug acuuucucuu uaaaacccuc gguaaccaac uuuucggccu gauuuucuuc    240

ucaaaaguuu uggcgacuaa cuucuugguu uggcuuucuc cucaaaaaau auaaacagc     299


<210> 20
<211> 11029
<212> RNA
<213> West Nile virus


<220>
<223> Genome West Nile virus

<400> 20
aguaguucgc cugugugagc ugacaaacuu aguaguguuu gugaggauua acaacaauua 60

acacagugcg agcuguuucu uagcacgaag aucucgaugu cuaagaaacc aggagggccc 120

ggcaagagcc gggcugucaa uaugcuaaaa cgcggaaugc cccgcguguu guccuugauu 180

ggacugaaga gggcuauguu gagccugauc gacggcaagg ggccaauacg auuuguguug 240

gcucucuugg cguucuucag guucacagca auugcuccga cccgagcagu gcuggaucga 300

uggagaggug ugaacaaaca aacagcgaug aaacaccuuc ugaguuuuaa gaaggaacua 360

gggaccuuga ccagugcuau caaucggcgg agcucaaaac aaaagaaaag aggaggaaag 420

accggaauug cagucaugau uggccugauu gccagcguag gagcaguuac ccucucuaac 480

uuccaaggga aggugaugau gacgguaaau gcuacugacg ucacagaugu caucacgauu 540

ccaacagcug cuggaaagaa ccuaugcauu gucagagcaa uggauguggg auacaugugc 600

gaugauacua ucacuuauga augcccagug cugucggcug guaaugaucc agaagacauc 660

gacuguuggu gcacaaaguc agcagucuac gucagguaug gaagaugcac caagacacgc 720

cacucaagac gcagucggag gucacugaca gugcagacac acggagaaag cacucuagcg 780

aacaagaagg gggcuuggau ggacagcacc aaggccacaa gguauuuggu aaaaacagaa 840

ucauggaucu ugaggaaccc uggauaugcc cugguggcag ccgucauugg uuggaugcuu 900

gggagcaaca ccaugcagag aguuguguuu gucgugcuau ugcuuuuggu ggccccagcu 960

uacagcuuca acugccuugg aaugagcaac agagacuucu uggaaggagu gucuggagca 1020

acaugggugg auuugguucu cgaaggcgac agcugcguga cuaucauguc uaaggacaag 1080

ccuaccaucg augugaagau gaugaauaug gaggcggcca accuggcaga gguccgcagu 1140

uauugcuauu uggcuaccgu cagcgaucuc uccaccaaag cugcgugccc gaccauggga 1200

gaagcucaca augacaaacg ugcugaccca gcuuuugugu gcagacaagg agugguggac 1260

aggggcuggg gcaacggcug cggacuauuu ggcaaaggaa gcauugacac augcgccaaa 1320

uuugccugcu cuaccaaggc aauaggaaga accaucuuga aagagaauau caaguacgaa 1380

guggccauuu uuguccaugg accaacuacu guggagucgc acggaaacua cuccacacag 1440

guuggagcca cucaggcagg gagauucagc aucacuccug cggcgccuuc auacacacua 1500

aagcuuggag aauauggaga ggugacagug gacugugaac cacggucagg gauugacacc 1560

aaugcauacu acgugaugac uguuggaaca aagacguucu ugguccaucg ugagugguuc 1620

auggaccuca accucccuug gagcagugcu ggaaguacug uguggaggaa cagagagacg 1680

uuaauggagu uugaggaacc acacgccacg aagcagucug ugauagcauu gggcucacaa 1740

gagggagcuc ugcaucaagc uuuggcugga gccauuccug uggaauuuuc aagcaacacu 1800

gucaaguuga cgucggguca uuugaagugu agagugaaga uggaaaaauu gcaguugaag 1860

ggaacaaccu auggcgucug uucaaaggcu uucaaguuuc uugggacucc cgcagacaca 1920

ggucacggca cugugguguu ggaauugcag uacacuggca cggauggacc uugcaaaguu 1980

ccuaucucgu caguggcuuc auugaacgac cuaacgccag ugggcagauu ggucacuguc 2040

aacccuuuug uuucaguggc cacggccaac gcuaaggucc ugauugaauu ggaaccaccc 2100

uuuggagacu cauacauagu ggugggcaga ggagaacaac agaucaauca ccauuggcac 2160

aagucuggaa gcagcauugg caaagccuuu acaaccaccc ucaaaggagc gcagagacua 2220

gccgcucuag gagacacagc uugggacuuu ggaucaguug gagggguguu caccucaguu 2280

gggaaggcug uccaucaagu guucggagga gcauuccgcu cacuguucgg aggcaugucc 2340

uggauaacgc aaggauugcu gggggcucuc cuguugugga ugggcaucaa ugcucgugau 2400

agguccauag cucucacguu ucucgcaguu ggaggaguuc ugcucuuccu cuccgugaac 2460

gugcacgcug acacugggug ugccauagac aucagccggc aagagcugag auguggaagu 2520

ggaguguuca uacacaauga uguggaggcu uggauggacc gguacaagua uuacccugaa 2580

acgccacaag gccuagccaa gaucauucag aaagcucaua aggaaggagu gugcggucua 2640

cgaucaguuu ccagacugga gcaucaaaug ugggaagcag ugaaggacga gcugaacacu 2700

cuuuugaagg agaauggugu ggaccuuagu gucgugguug agaaacagga gggaauguac 2760

aagucagcac cuaaacgccu caccgccacc acggaaaaau uggaaauugg cuggaaggcc 2820

uggggaaaga guauuuuauu ugcaccagaa cucgccaaca acaccuuugu gguugauggu 2880

ccggagacca aggaaugucc gacucagaau cgcgcuugga auagcuuaga aguggaggau 2940

uuuggauuug gucucaccag cacucggaug uuccugaagg ucagagagag caacacaacu 3000

gaaugugacu cgaagaucau uggaacggcu gucaagaaca acuuggcgau ccacagugac 3060

cuguccuauu ggauugaaag caggcucaau gauacgugga agcuugaaag ggcaguucug 3120

ggugaaguca aaucauguac guggccugag acgcauaccu uguggggcga uggaauccuu 3180

gagagugacu ugauaauacc agucacacug gcgggaccac gaagcaauca caaucggaga 3240

ccuggguaca agacacaaaa ccagggccca uggacgaagg gccgguagaa gauugacuuc 3300

gauuacugcc caggaacuac ggucacccug agugagagcu gcggacaccg uggaccugcc 3360

acucgcacca ccacagagag cggaaaguug auaacagauu ggugcugcag gagcugcacc 3420

uuaccaccac ugcgcuacca aacugacagc ggcuguuggu augguaugga gaucagacca 3480

cagagacaug augaaaagac ccucgugcag ucacaaguga augcuuauaa ugcugauaug 3540

auugacccuu uucaguuggg ccuucugguc guguucuugg ccacccagga gguccuucgc 3600

aagaggugga cagccaagau cagcaugcca gcuauacuga uugcucugcu aguccuggug 3660

uuugggggca uuacuuacac ugauguguua cgcuauguca ucuugguggg ggcagcuuuc 3720

gcagaaucua auucgggagg agacguggua cacuuggcgc ucauggcgac cuucaagaua 3780

caaccagugu uuaugguggc aucguuucuc aaagcgagau ggaccaacca ggagaacauu 3840

uuguugaugu uggcggcugu uuucuuucaa auggcuuauc acgaugcccg ccaaauucug 3900

cucugggaga ucccugaugu guugaauuca cuggcgguag cuuggaugau acugagagcc 3960

auaacauuca caacgacauc aaacgugguu guuccgcugc uagcccugcu aacacccggg 4020

cugagacgcu ugaaucugga uguguacagg auacugcugu ugauggucgg aauaggcagc 4080

uugaucaggg agaagaggag ugcagcugca aaaaagaaag gagcaagucu gcuaugcuug 4140

gcucuagccu caacaggacu uuucaacccc augauccuug cugcuggacu gauugcaugu 4200

gaucccaacc guaaacgcgg auggcccgca acugaaguga ugacagcugu cggccuaaug 4260

uuugccaucg ucggagggcu ggcagagcuu gacauugacu ccauggccau uccaaugacu 4320

aucgcggggc ucauguuugc ugcuuucgug auuucuggga aaucaacaga uauguggauu 4380

gagagaacgg cggacauuuc cugggaaagu gaugcagaaa uuacaggcuc gagcgaaaga 4440

guugaugugc ggcuugauga ugauggaaac uuccagcuca ugaaugaucc aggagcaccu 4500

uggaagauau ggaugcucag aauggucugu cucgcgauua gugcguacac cccuugggca 4560

aucuugcccu caguaguugg auuuuggaua acucuccaau acacaaagag aggaggcgug 4620

uugugggaca cucccucacc aaaggaguac aaaaaggggg acacgaccac cggcgucuac 4680

aggaucauga cucgugggcu gcucggcagu uaucaagcag gagcgggcgu gaugguugaa 4740

ggguguuucc acacccuuug gcauacaaca aaaggagccg cuuugaugag cggagagggc 4800

cgccuggacc cauacugggg cagugucaag gaggaucgac uuuguuacgg aggacccugg 4860

aaauugcagc acaaguggaa cgggcaggau gaggugcaga ugauuguggu ggaaccuggc 4920

aagaacguua agaacguccg gacgaaacca gggguguuca aaacaccuga aggagaaauc 4980

ggggccguga cuuuggacuu ccccacugga acaucaggcu caccaauagu ggacaaaaac 5040

ggugauguga uugggcuuua uggcaaugga gucauaaugc ccaacggcuc auacauaagc 5100

gcgauagugc agggugaaag gauggaugag ccaaucccag ccggauucga accugagaug 5160

cugaggaaaa aacagaucac uguacuggau cuccaucccg gcgccgguaa aacaaggagg 5220

auucugccac agaucaucaa agaggccaua aacagaagac ugagaacagc cgugcuagca 5280

ccaaccaggg uuguggcugc ugagauggcu gaagcacuga gaggacugcc cauccgguac 5340

cagacauccg cagugcccag agaacauaau ggaaaugaga uuguugaugu caugugucau 5400

gcuacccuca cccacaggcu gaugucuccu cacagggugc cgaacuacaa ccuguucgug 5460

auggaugagg cucauuucac cgacccagcu agcauugcag caagagguua cauuuccaca 5520

aaggucgagc uaggggaggc ggcggcaaua uucaugacag ccaccccacc aggcacuuca 5580

gauccauucc cagaguccaa uucaccaauu uccgacuuac agacugagau cccggaucga 5640

gcuuggaacu cuggauacga auggaucaca gaauacaccg ggaagacggu uugguuugug 5700

ccuaguguca agauggggaa ugagauugcc cuuugccuac aacgugcugg aaagaaagua 5760

guccaauuga acagaaaguc guacgagacg gaguacccaa aauguaagaa cgaugauugg 5820

gacuuuguua ucacaacaga cauaucugaa augggggcua acuucaaggc gagcagggug 5880

auugacagcc ggaagagugu gaaaccaacc aucauaacag aaggagaagg gagagugauc 5940

cugggagaac caucugcagu gacagcagcu agugccgccc agagacgugg acguaucggu 6000

agaaauccgu cgcaaguugg ugaugaguac uguuaugggg ggcacacgaa ugaagacgac 6060

ucgaacuucg cccauuggac ugaggcacga aucaugcugg acaacaucaa caugccaaac 6120

ggacugaucg cucaauucua ccaaccagag cgugagaagg uauauaccau ggauggggaa 6180

uaccggcuca gaggagaaga gagaaaaaac uuucuggaac uguugaggac ugcagaucug 6240

ccaguuuggc uggcuuacaa gguugcagcg gcuggagugu cauaccacga ccggaggugg 6300

ugcuuugaug guccuaggac aaacacaauu uuagaagaca acaacgaagu ggaagucauc 6360

acgaagcuug gugaaaggaa gauucugagg ccgcgcugga uugacgccag gguguacucg 6420

gaucaccagg cacuaaaggc guucaaggac uucgccucgg gaaaacguuc ucagauaggg 6480

cucauugagg uucugggaaa gaugccugag cacuucaugg ggaagacaug ggaagcacuu 6540

gacaccaugu acguuguggc cacugcagag aaaggaggaa gagcucacag aauggcccug 6600

gaggaacugc cagaugcucu ucagacaauu gccuugauug ccuuauugag ugugaugacc 6660

augggaguau ucuuccuccu caugcagcgg aagggcauug gaaagauagg uuugggaggc 6720

gcugucuugg gagucgcgac cuuuuucugu uggauggcug aaguuccagg aacgaagauc 6780

gccggaaugu ugcugcucuc ccuucucuug augauugugc uaauuccuga gccagagaag 6840

caacguucgc agacagacaa ccagcuagcc guguuccuga uuugugucau gacccuugug 6900

agcgcagugg cagccaacga gaugguuugg cuagauaaga ccaagaguga cauaagcagu 6960

uuguuugggc aaagaauuga ggucaaggag aauuucagca ugggagaguu ccuucuggac 7020

uugaggccgg caacagccug gucacuguac gcugugacaa cagcgguccu cacuccacug 7080

cuaaagcauu ugaucacguc agauuacauc aacaccucau ugaccucaau aaacguucag 7140

gcaagugcac uauucacacu cgcgcgaggc uuccccuucg ucgauguugg agugucggcu 7200

cuccugcuag cagccggaug cuggggacaa gucacccuca ccguuacggu aacagcggca 7260

acacuccuuu uuugccacua ugccuacaug guucccgguu ggcaagcuga ggcaaugcgc 7320

ucagcccagc ggcggacagc ggccggaauc augaagaacg cuguagugga uggcaucgug 7380

gccacggacg ucccagaauu agagcgcacc acacccauca ugcagaagaa aguuggacag 7440

aucaugcuga ucuugguguc ucuagcugca guaguaguga acccgucugu gaagacagua 7500

cgagaagccg gaauuuugau cacggccgca gcggugacgc uuugggagaa uggagcaagc 7560

ucuguuugga acgcaacaac ugccaucgga cucugccaca ucaugcgugg ggguugguug 7620

ucaugucuau ccauaacaug gacacucaua aagaacaugg aaaaaccagg acuaaaaaga 7680

gguggggcaa aaggacgcac cuugggagag guuuggaaag aaagacucaa ccagaugaca 7740

aaagaagagu ucacuaggua ccgcaaagag gccaucaucg aagucgaucg cucagcggca 7800

aaacacgcca ggaaagaagg caaugucacu ggagggcauc cagucucuag gggcacagca 7860

aaacugagau ggcuggucga acggagguuu cucgaaccgg ucggaaaagu gauugaccuu 7920

ggauguggaa gaggcgguug guguuacuau auggcaaccc aaaaaagagu ccaagaaguc 7980

agaggguaca caaagggcgg ucccggacau gaagagcccc aacuagugca aaguuaugga 8040

uggaacauug ucaccaugaa gaguggagug gauguguucu acagaccuuc ugaguguugu 8100

gacacccucc uuugugacau cggagagucc ucgucaagug cugagguuga agagcauagg 8160

acgauucggg uccuugaaau gguugaggac uggcugcacc gagggccaag ggaauuuugc 8220

gugaaggugc ucugccccua caugccgaaa gucauagaga agauggagcu gcuccaacgc 8280

cgguaugggg ggggacuggu cagaaaccca cucucacgga auuccacgca cgagauguau 8340

ugggugaguc gagcuucagg caauguggua cauucaguga auaugaccag ccaggugcuc 8400

cuaggaagaa uggaaaaaag gaccuggaag ggaccccaau acgaggaaga uguaaacuug 8460

ggaaguggaa ccagggcggu gggaaaaccc cugcucaacu cagacaccag uaaaaucaag 8520

aacaggauug aacgacucag gcgugaguac aguucgacgu ggcaccacga ugagaaccac 8580

ccauauagaa ccuggaacua ucacggcagu uaugauguga agcccacagg cuccgccagu 8640

ucgcugguca auggaguggu caggcuccuc ucaaaaccau gggacaccau cacgaauguu 8700

accaccaugg ccaugacuga cacuacuccc uucgggcagc agcgaguguu caaagagaag 8760

guggacacga aagcuccuga accgccagaa ggagugaagu acgugcucaa cgagaccacc 8820

aacugguugu gggcguuuuu ggccagagaa aaacguccca gaaugugcuc ucgagaggaa 8880

uucauaagaa aggucaacag caaugcagcu uugggugcca uguuugaaga gcagaaucaa 8940

uggaggagcg ccagagaagc aguugaagau ccaaaauuuu gggagauggu ggaugaggag 9000

cgcgaggcac aucugcgggg ggaaugucac acuugcauuu acaacaugau gggaaagaga 9060

gagaaaaaac ccggagaguu cggaaaggcc aagggaagca gagccauuug guucaugugg 9120

cucggagcuc gcuuucugga guucgaggcu cugggguuuc ucaaugaaga ccacuggcuu 9180

ggaagaaaga acucaggagg aggugucgag ggcuugggcc uccaaaaacu ggguuacauc 9240

cugcgugaag uuggcacccg gccugggggc aagaucuaug cugaugacac agcuggcugg 9300

gacacccgca ucacgagagc ugacuuggaa aaugaagcua aggugcuuga gcugcuugau 9360

ggggaacauc ggcgucuugc cagggccauc auugagcuca ccuaucguca caaaguugug 9420

aaagugaugc gcccggcugc ugauggaaga accgucaugg auguuaucuc cagagaagau 9480

cagaggggga guggacaagu ugucaccuac gcccuaaaca cuuucaccaa ccuggccguc 9540

cagcugguga ggaugaugga aggggaagga gugauuggcc cagaugaugu ggagaaacuc 9600

acaaaaggga aaggacccaa agucaggacc uggcuguuug agaaugggga agaaagacuc 9660

agccgcaugg cugucagugg agaugacugu gugguaaagc cccuggacga ucgcuuugcc 9720

accucgcucc acuuccucaa ugcuauguca aagguucgca aagacaucca agaguggaaa 9780

ccgucaacug gaugguauga uuggcagcag guuccauuuu gcucaaacca uuucacugaa 9840

uugaucauga aagauggaag aacacuggug guuccaugcc gaggacagga ugaauuggua 9900

ggcagagcuc gcauaucucc aggggccgga uggaacgucc gcgacacugc uugucuggcu 9960

aagucuuaug cccagaugug gcugcuucug uacuuccaca gaagagaccu gcggcucaug 10020

gccaacgcca uuugcuccgc ugucccugug aauugggucc cuaccggaag aaccacgugg 10080

uccauccaug caggaggaga guggaugaca acagaggaca uguuggaggu cuggaaccgu 10140

guuuggauag aggagaauga auggauggaa gacaaaaccc caguggagaa auggagugac 10200

gucccauauu caggaaaacg agaggacauc ugguguggca gccugauugg cacaagagcc 10260

cgagccacgu gggcagaaaa cauccaggug gcuaucaacc aagucagagc aaucaucgga 10320

gaugagaagu auguggauua caugaguuca cuaaagagau augaagacac aacuuugguu 10380

gaggacacag uacuguagau auuuaaucaa uuguaaauag acaauauaag uaugcauaaa 10440

aguguaguuu uauaguagua uuuaguggug uuaguguaaa uaguuaagaa aauuuugagg 10500

agaaagucag gccgggaagu ucccgccacc ggaaguugag uagacggugc ugccugcgac 10560

ucaaccccag gaggacuggg ugaacaaagc cgcgaaguga uccauguaag cccucagaac 10620

cguuucggaa ggaggacccc acauguugua acuucaaagc ccaaugucag accacgcuac 10680

ggcgugcuac ucugcggaga gugcagucug cgauagugcc ccaggaggac uggguuaaca 10740

aaggcaaacc aacgccccac gcggcccuag ccccgguaau gguguuaacc agggcgaaag 10800

gacuagaggu uagaggagac cccgcgguuu aaagugcacg gcccagccug gcugaagcug 10860

uaggucaggg gaaggacuag agguuagugg agaccccgug ccacaaaaca ccacaacaaa 10920

acagcauauu gacaccuggg auagacuagg agaucuucug cucugcacaa ccagccacac 10980

ggcacagugc gccgacaaug guggcuggug gugcgagaac acaggaucu 11029

<210> 21
<211> 270
<212> RNA
<213> Artificial Sequence

<220>

<223> dchp_wsII_A

<400> 21
ugcaguuaga uaauauuggg agguguguuu uuugccuuuu uccguagauc cucgcgaaac 60

uucuuggccu gguuuucuuu ucaaaagagu ccuuacguau auuggagggu guguuuuucg 120

cuuuuuuccg ucucggaaac gauaacuucc uggcuugauu uuuucuucaa aagcugcguu 180

aucuauauug agaggcgugu uuuucgucuu uuuccguucc aaccuggaga acuuuuuggc 240

cugauuuucu cuucaaaacc aggccguacc 270


<210> 22
<211> 270
<212> RNA
<213> Artificial Sequence


<220>
<223> dchp_wsII_B

<400> 22
ugcaguuaga uaauauugga aggcguguuu uuugcuuuuu uccguagauc cuaacggaac 60

uuccuggccu gauuuucuuc uuaaaagagu ccuuacguau auugaaaggu guguuuuucg 120

ccuuuuuccg ucucggaaac gauaacuuuc cggccugguu uuuuccuuaa aagcugcguu 180

aucuauauug aggggugugu uuuuugucuu uuuccguucc aaccuggaga acuuccuggu 240

uuggcuuuuu ucuugaaacc aggccguacc 270


<210> 23
<211> 270
<212> RNA
<213> Artificial Sequence


<220>
<223> dchp_wsII_C

<400> 23
ugcaguuaga uaauauuggg agguguguuu uuugccuuuu uccguagauc cucgcgaaac 60

uucuuggccu gguuuucuuu ucaaaagagu ccuuacguau auuggagggu guguuuuucg 120

cuuuuuuccg ucucggaaac gauaacuucc uggcuugauu uuuucuucaa aagcugcguu 180

aucuauauug agaggcgugu uuuucgucuu uuuccguucc aaccuggaga acuucccggc 240

cugacuuuuu ccuuaaaacc aggccguacc 270


<210> 24
<211> 299
<212> RNA
<213> Artificial Sequence


<220>

<223> circ_wnv_sIII_1

<400> 24
ugcaguuaga uaaacuuucu gguuugguuu ucuccucaaa agacaguucu ucgaacuucc      60

cgguuugauu uucucuuuaa aaauggggcc ccucaacuuc uuggucugac uuucucuuca     120

aaaggcuaua cgaucaacuu cucggccuga uuuucuuuuc aaaaagcuau caucgcaacu     180

uccuggucug guuuucuucu caaaacccuc gguaaccaac uuuccggcuu gacuuucuuc     240

uuaaaacguu uugcgacaaa cuuuucggcu uggcuuucuc cuuaaaauau guaaacagc      299


<210> 25
<211> 33
<212> RNA
<213> Artificial Sequence


<220>
<223> Target sequence IRES Circ HCV1

<400> 25
cuccgccaug aaucacuccc cugugaggaa cua                                    33


<210> 26
<211> 24
<212> RNA
<213> Artificial Sequence


<220>
<223> Target sequence IRES Circ HCV2

<400> 26
ucucguagac cgugcaccau gagc                                              24


<210> 27
<211> 28
<212> RNA
<213> Artificial Sequence


<220>
<223> Target sequence cHP (CDS1) circ_hcv_cds1

<400> 27
ccaaaagaaa caccaaccgu cgcccaga                                          28


<210> 28
<211> 16
<212> RNA
<213> Artificial Sequence


<220>
<223> Target sequence CDS2 circ_hcv_cds2

<400> 28

ggggccccag guuggg                                                    16

<210> 29
<211> 32
<212> RNA
<213> Artificial Sequence

<220>
<223> Target sequence sHP (3′ UTR) circ_dv_3utr

<400> 29
aacagcauau ugacgcuggg aaagaccaga ga                                  32

<210> 30
<211> 33
<212> RNA
<213> Artificial Sequence

<220>
<223> Target sequence cHP1/2 circ_dv_cHP_v1/2

<400> 30
acggaaaaag gcgaaaaaca cgccuuucaa uau                                 33

<210> 31
<211> 34
<212> RNA
<213> Artificial Sequence

<220>
<223> Target sequence Chikv_re

<400> 31
gcugcuguaa aacguuggcu uuuuuagccg uaau                                34

<210> 32
<211> 294
<212> RNA
<213> Artificial Sequence

<220>
<223> Broad Spectrum Circular Artificial sequence to DENV cHP and HCV
      CDS (circ_dv_cHP_v1_circ_hcv_cds2_2)

<400> 32
gcgccgaugu ugaaaggugu guuuuucguu uuuuucuguc aaguguagga ucuucugggg     60

ucucgggccc cacgaccaau guuggggggc guguuuuucg cuuuuuucug ucaaaauaau    120

guugaccugg gguucuuuac ugaacucauu auguugggag gcguguuuuu ugucuuuuuu    180

uguuuuaucu acacccgucu gggguuuugu cggcgccacg acauguuggg ggguguguuu    240

uuugccuuuu uccgucucag ugcggcgccc uugggguucc cuuucgcauc acgu         294

<210> 33
<211> 35
<212> RNA
<213> Artificial Sequence

<220>
<223> Target sequence
5Ã\204Â\202Ã\202Â\204Ä\202Â\202Ã\202Â\202Ã\204Â\202Ã\202Â\213Ä\202Â\202Ã\202Â
\230Ä\202Â\204Ã\202Â\202Ä\202Â\202Ã\202Â\202Ã\204Â\202Ã\202Â\202Ä\202Â\202Ã
\202Â\200Ä\202Â\204Ã\202Â\202Ä\202Â\202Ã\202Â\202Ã\204Â\202Ã\202Â\202Ä\202Â
\202Ã\202Â\231
      UTR chikv5utr

<400> 33
acacacguag ccuaccaguu ucuuacugcu cuacu                                35


<210> 34
<211> 29903
<212> RNA
<213> Coronaviridae


<220>
<223> NC_045512.2 Severe acute respiratory syndrome coronavirus 2
      (SARS-CoV-2) isolate Wuhan-Hu-1, complete genome

<400> 34
auuaaagguu uauaccuucc cagguaacaa accaaccaac uuucgaucuc uuguagaucu       60

guucucuaaa cgaacuuuaa aaucugugug gcugucacuc ggcugcaugc uuagugcacu      120

cacgcaguau aauuaauaac uaauuacugu cguugacagg acacgaguaa cucgucuauc      180

uucugcaggc ugcuuacggu uucguccgug uugcagccga ucaucagcac aucuagguuu      240

cguccgggug ugaccgaaag guaagaugga gagccuuguc ccugguuuca acgagaaaac      300

acacguccaa cucaguuugc cuguuuuaca gguucgcgac gugcucguac guggcuuugg      360

agacuccgug gaggaggucu uaucagaggc acgucaacau cuuaaagaug gcacuugugg      420

cuuaguagaa guugaaaaag gcguuuugcc ucaacuugaa cagcccuaug uguucaucaa      480

acguucggau gcucgaacug caccucaugg ucauguuaug guugagcugg uagcagaacu      540

cgaaggcauu caguacgguc guagugguga gacacuuggu guccuugucc cucauguggg      600

cgaaauacca guggcuuacc gcaagguucu ucuucguaag aacgguaaua aaggagcugg      660

uggccauagu uacggcgccg aucuaaaguc auuugacuua ggcgacgagc uuggcacuga      720

uccuuaugaa gauuuucaag aaaacuggaa cacuaaacau agcaguggug uuacccguga      780

acucaugcgu gagcuuaacg gaggggcaua cacucgcuau gucgauaaca acuucugugg      840

cccugauggc uacccucuug agugcauuaa agaccuucua gcacgugcug guaaagcuuc      900

augcacuuug uccgaacaac uggacuuuau ugacacuaag aggggguauu acugcugccg      960

ugaacaugag caugaaauug cuugguacac ggaacguucu gaaaagagcu augaauugca     1020

gacaccuuuu gaaauuaaau uggcaaagaa auuugacacc uucaaugggg aauguccaaa 1080

uuuuguauuu cccuuaaauu ccauaaucaa gacuauucaa ccaagguugg aaaagaaaaa 1140

gcuugauggc uuuaugggua gaauucgauc ugucuaucca guugcgucac caaaugaaug 1200

caaccaaaug ugccuuucaa cucucaugaa gugugaucau uguggugaaa cuucauggca 1260

gacgggcgau uuuguuaaag ccacuugcga auuuuguggc acugagaauu ugacuaaaga 1320

aggugccacu acuugugguu acuuacccca aaaugcuguu guuaaaauuu auuguccagc 1380

augucacaau ucagaaguag gaccugagca uagucuugcc gaauaccaua augaaucugg 1440

cuugaaaacc auucuucgua aggguggucg cacuauugcc uuuggaggcu guguguucuc 1500

uuauguuggu ugccauaaca agugugccua uuggguucca cgugcuagcg cuaacauagg 1560

uuguaaccau acagguguug uuggagaagg uuccgaaggu cuuaaugaca accuucuuga 1620

aauacuccaa aaagagaaag ucaacaucaa uauuguuggu gacuuuaaac uuaaugaaga 1680

gaucgccauu auuuuggcau cuuuuucugc uuccacaagu gcuuuugugg aaacugugaa 1740

agguuuggau uauaaagcau ucaaacaaau uguugaaucc ugugguaauu uuaaaguuac 1800

aaaaggaaaa gcuaaaaaag gugccuggaa uauuggugaa cagaaaucaa uacugagucc 1860

ucuuuaugca uuugcaucag aggcugcucg uguuguacga ucaauuuucu cccgcacucu 1920

ugaaacugcu caaaauucug ugcguguuuu acagaaggcc gcuauaacaa uacuagaugg 1980

aauuucacag uauucacuga gacucauuga ugcuaugaug uucacaucug auuuggcuac 2040

uaacaaucua guuguaaugg ccuacauuac aggugguguu guucaguuga cuucgcagug 2100

gcuaacuaac aucuuuggca cuguuuauga aaaacucaaa cccguccuug auuggcuuga 2160

agagaaguuu aaggaaggug uagaguuucu uagagacggu ugggaaauug uuaaauuuau 2220

cucaaccugu gcuugugaaa uugucggugg acaaauuguc accugugcaa aggaaauuaa 2280

ggagaguguu cagacauucu uuaagcuugu aaauaaauuu uuggcuuugu gugcugacuc 2340

uaucauuauu gguggagcua aacuuaaagc cuugaauuua ggugaaacau uugucacgca 2400

cucaaaggga uuguacagaa aguguguuaa auccagagaa gaaacuggcc uacucaugcc 2460

ucuaaaagcc ccaaaagaaa uuaucuucuu agagggagaa acacuuccca cagaaguguu 2520

aacagaggaa guugucuuga aaacugguga uuuacaacca uuagaacaac cuacuaguga 2580

agcuguugaa gcuccauugg uugguacacc aguuuguauu aacgggcuua uguugcucga 2640

aaucaaagac acagaaaagu acugugcccu ugcaccuaau augaugguaa caaacaauac 2700

cuucacacuc aaaggcggug caccaacaaa gguuacuuuu ggugaugaca cugugauaga 2760

agugcaaggu uacaagagug ugaauaucac uuuugaacuu gaugaaagga uugauaaagu 2820

acuuaaugag aagugcucug ccuauacagu ugaacucggu acagaaguaa augaguucgc 2880

cuguguugug gcagaugcug ucauaaaaac uuugcaacca guaucugaau uacuuacacc 2940

acugggcauu gauuuagaug aguggaguau ggcuacauac uacuuauuug augagucugg 3000

ugaguuuaaa uuggcuucac auauguauug uucuuucuac ccuccagaug aggaugaaga 3060

agaaggugau ugugaagaag aagaguuuga gccaucaacu caauaugagu augguacuga 3120

agaugauuac caagguaaac cuuuggaauu uggugccacu ucugcugcuc uucaaccuga 3180

agaagagcaa gaagaagauu gguuagauga ugauagucaa caaacuguug gucaacaaga 3240

cggcagugag gacaaucaga caacuacuau ucaaacaauu guugagguuc aaccucaauu 3300

agagauggaa cuuacaccag uuguucagac uauugaagug aauaguuuua gugguuauuu 3360

aaaacuuacu gacaauguau acauuaaaaa ugcagacauu guggaagaag cuaaaaaggu 3420

aaaaccaaca gugguuguua augcagccaa uguuuaccuu aaacauggag gagguguugc 3480

aggagccuua aauaaggcua cuaacaaugc caugcaaguu gaaucugaug auuacauagc 3540

uacuaaugga ccacuuaaag ugggugguag uuguguuuua agcggacaca aucuugcuaa 3600

acacugucuu cauguugucg gcccaaaugu uaacaaaggu gaagacauuc aacuucuuaa 3660

gagugcuuau gaaaauuuua aucagcacga aguucuacuu gcaccauuau uaucagcugg 3720

uauuuuuggu gcugacccua uacauucuuu aagaguuugu guagauacug uucgcacaaa 3780

ugucuacuua gcugucuuug auaaaaaucu cuaugacaaa cuuguuucaa gcuuuuugga 3840

aaugaagagu gaaaagcaag uugaacaaaa gaucgcugag auuccuaaag aggaaguuaa 3900

gccauuuaua acugaaagua aaccuucagu ugaacagaga aaacaagaug auaagaaaau 3960

caaagcuugu guugaagaag uuacaacaac ucuggaagaa acuaaguucc ucacagaaaa 4020

cuuguuacuu uauauugaca uuaauggcaa ucuucaucca gauucugcca cucuuguuag 4080

ugacauugac aucacuuucu uaaagaaaga ugcuccauau auagugggug auguuguuca 4140

agaggguguu uuaacugcug ugguuauacc uacuaaaaag gcugguggca cuacugaaau 4200

gcuagcgaaa gcuuugagaa aagugccaac agacaauuau auaaccacuu acccggguca 4260

ggguuuaaau gguuacacug uagaggaggc aaagacagug cuuaaaaagu guaaaagugc 4320

cuuuuacauu cuaccaucua uuaucucuaa ugagaagcaa gaaauucuug gaacuguuuc 4380

uuggaauuug cgagaaaugc uugcacaugc agaagaaaca cgcaaauuaa ugccugucug 4440

uguggaaacu aaagccauag uuucaacuau acagcguaaa uauaagggua uuaaaauaca 4500

agagggugug guugauuaug gugcuagauu uuacuuuuac accaguaaaa caacuguagc 4560

gucacuuauc aacacacuua acgaucuaaa ugaaacucuu guuacaaugc cacuuggcua 4620

uguaacacau ggcuuaaauu uggaagaagc ugcucgguau augagaucuc ucaaagugcc 4680

agcuacaguu ucuguuucuu caccugaugc uguuacagcg uauaaugguu aucuuacuuc 4740

uucuucuaaa acaccugaag aacauuuuau ugaaaccauc ucacuugcug guuccuauaa 4800

agauuggucc uauucuggac aaucuacaca acuagguaua gaauuucuua agagagguga 4860

uaaaagugua uauuacacua guaauccuac cacauuccac cuagauggug aaguuaucac 4920

cuuugacaau cuuaagacac uucuuucuuu gagagaagug aggacuauua agguguuuac 4980

aacaguagac aacauuaacc uccacacgca aguuguggac augucaauga cauauggaca 5040

acaguuuggu ccaacuuauu uggauggagc ugauguuacu aaaauaaaac cucauaauuc 5100

acaugaaggu aaaacauuuu auguuuuacc uaaugaugac acucuacgug uugaggcuuu 5160

ugaguacuac cacacaacug auccuaguuu ucuggguagg uacaugucag cauuaaauca 5220

cacuaaaaag uggaaauacc cacaaguuaa ugguuuaacu ucuauuaaau gggcagauaa 5280

caacuguuau cuugccacug cauuguuaac acuccaacaa auagaguuga aguuuaaucc 5340

accugcucua caagaugcuu auuacagagc aagggcuggu gaagcugcua acuuuugugc 5400

acuuaucuua gccuacugua auaagacagu aggugaguua ggugauguua gagaaacaau 5460

gaguuacuug uuucaacaug ccaauuuaga uucuugcaaa agagucuuga acguggugug 5520

uaaaacuugu ggacaacagc agacaacccu uaagggugua gaagcuguua uguacauggg 5580

cacacuuucu uaugaacaau uuaagaaagg uguucagaua ccuuguacgu gugguaaaca 5640

agcuacaaaa uaucuaguac aacaggaguc accuuuuguu augaugucag caccaccugc 5700

ucaguaugaa cuuaagcaug guacauuuac uugugcuagu gaguacacug guaauuacca 5760

guguggucac uauaaacaua uaacuucuaa agaaacuuug uauugcauag acggugcuuu 5820

acuuacaaag uccucagaau acaaaggucc uauuacggau guuuucuaca aagaaaacag 5880

uuacacaaca accauaaaac caguuacuua uaaauuggau gguguuguuu guacagaaau 5940

ugacccuaag uuggacaauu auuauaagaa agacaauucu uauuucacag agcaaccaau 6000

ugaucuugua ccaaaccaac cauauccaaa cgcaagcuuc gauaauuuua aguuuguaug 6060

ugauaauauc aaauuugcug augauuuaaa ccaguuaacu gguuauaaga aaccugcuuc 6120

aagagagcuu aaaguuacau uuuucccuga cuuaaauggu gauguggugg cuauugauua 6180

uaaacacuac acacccucuu uuaagaaagg agcuaaauug uuacauaaac cuauuguuug 6240

gcauguuaac aaugcaacua auaaagccac guauaaacca aauaccuggu guauacguug 6300

ucuuuggagc acaaaaccag uugaaacauc aaauucguuu gauguacuga agucagagga 6360

cgcgcaggga auggauaauc uugccugcga agaucuaaaa ccagucucug aagaaguagu 6420

ggaaaauccu accauacaga aagacguucu ugaguguaau gugaaaacua ccgaaguugu 6480

aggagacauu auacuuaaac cagcaaauaa uaguuuaaaa auuacagaag agguuggcca 6540

cacagaucua auggcugcuu auguagacaa uucuagucuu acuauuaaga aaccuaauga 6600

auuaucuaga guauuagguu ugaaaacccu ugcuacucau gguuuagcug cuguuaauag 6660

ugucccuugg gauacuauag cuaauuaugc uaagccuuuu cuuaacaaag uuguuaguac 6720

aacuacuaac auaguuacac gguguuuaaa ccguguuugu acuaauuaua ugccuuauuu 6780

cuuuacuuua uugcuacaau uguguacuuu uacuagaagu acaaauucua gaauuaaagc 6840

aucuaugccg acuacuauag caaagaauac uguuaagagu gucgguaaau uuugucuaga 6900

ggcuucauuu aauuauuuga agucaccuaa uuuuucuaaa cugauaaaua uuauaauuug 6960

guuuuuacua uuaaguguuu gccuagguuc uuuaaucuac ucaaccgcug cuuuaggugu 7020

uuuaaugucu aauuuaggca ugccuucuua cuguacuggu uacagagaag gcuauuugaa 7080

cucuacuaau gucacuauug caaccuacug uacugguucu auaccuugua guguuugucu 7140

uagugguuua gauucuuuag acaccuaucc uucuuuagaa acuauacaaa uuaccauuuc 7200

aucuuuuaaa ugggauuuaa cugcuuuugg cuuaguugca gagugguuuu uggcauauau 7260

ucuuuucacu agguuuuucu auguacuugg auuggcugca aucaugcaau uguuuuucag 7320

cuauuuugca guacauuuua uuaguaauuc uuggcuuaug ugguuaauaa uuaaucuugu 7380

acaaauggcc ccgauuucag cuaugguuag aauguacauc uucuuugcau cauuuuauua 7440

uguauggaaa aguuaugugc auguuguaga cgguuguaau ucaucaacuu guaugaugug 7500

uuacaaacgu aauagagcaa caagagucga auguacaacu auuguuaaug guguuagaag 7560

guccuuuuau gucuaugcua auggagguaa aggcuuuugc aaacuacaca auuggaauug 7620

uguuaauugu gauacauucu gugcugguag uacauuuauu agugaugaag uugcgagaga 7680

cuugucacua caguuuaaaa gaccaauaaa uccuacugac cagucuucuu acaucguuga 7740

uaguguuaca gugaagaaug guuccaucca ucuuuacuuu gauaaagcug gucaaaagac 7800

uuaugaaaga cauucucucu cucauuuugu uaacuuagac aaccugagag cuaauaacac 7860

uaaagguuca uugccuauua auguuauagu uuuugauggu aaaucaaaau gugaagaauc 7920

aucugcaaaa ucagcgucug uuuacuacag ucagcuuaug ugucaaccua uacuguuacu 7980

agaucaggca uuagugucug auguugguga uagugcggaa guugcaguua aaauguuuga 8040

ugcuuacguu aauacguuuu caucaacuuu uaacguacca auggaaaaac ucaaaacacu 8100

aguugcaacu gcagaagcug aacuugcaaa gaaugugucc uuagacaaug ucuuaucuac 8160

uuuuauuuca gcagcucggc aaggguuugu ugauucagau guagaaacua aagauguugu 8220

ugaaugucuu aaauugucac aucaaucuga cauagaaguu acuggcgaua guuguaauaa 8280

cuauaugcuc accuauaaca aaguugaaaa caugacaccc cgugaccuug gugcuuguau 8340

ugacuguagu gcgcgucaua uuaaugcgca gguagcaaaa agucacaaca uugcuuugau 8400

auggaacguu aaagauuuca ugucauuguc ugaacaacua cgaaaacaaa uacguagugc 8460

ugcuaaaaag aauaacuuac cuuuuaaguu gacaugugca acuacuagac aaguuguuaa 8520

uguuguaaca acaaagauag cacuuaaggg ugguaaaauu guuaauaauu gguugaagca 8580

guuaauuaaa guuacacuug uguuccuuuu uguugcugcu auuuucuauu uaauaacacc 8640

uguucauguc augucuaaac auacugacuu uucaagugaa aucauaggau acaaggcuau 8700

ugaugguggu gucacucgug acauagcauc uacagauacu uguuuugcua acaaacaugc 8760

ugauuuugac acaugguuua gccagcgugg ugguaguuau acuaaugaca aagcuugccc 8820

auugauugcu gcagucauaa caagagaagu ggguuuuguc gugccugguu ugccuggcac 8880

gauauuacgc acaacuaaug gugacuuuuu gcauuucuua ccuagaguuu uuagugcagu 8940

ugguaacauc uguuacacac caucaaaacu uauagaguac acugacuuug caacaucagc 9000

uuguguuuug gcugcugaau guacaauuuu uaaagaugcu ucugguaagc caguaccaua 9060

uuguuaugau accaauguac uagaagguuc uguugcuuau gaaaguuuac gcccugacac 9120

acguuaugug cucauggaug gcucuauuau ucaauuuccu aacaccuacc uugaagguuc 9180

uguuagagug guaacaacuu uugauucuga guacuguagg cacggcacuu gugaaagauc 9240

agaagcuggu guuuguguau cuacuagugg uagaugguac uuaacaaug auuauuacag 9300

aucuuuacca ggaguuuucu gugguguaga ugcuguaaau uuacuuacua auauguuuac 9360

accacuaauu caaccuauug gugcuuugga cauaucagca ucuauaguag cuggugguau 9420

uguagcuauc guaguaacau gccuugccua cuauuuuaug agguuuagaa gagcuuuugg 9480

ugaauacagu cauguaguug ccuuuaauac uuuacuauuc cuuaugucau ucacuguacu 9540

cuguuuaaca ccaguuuacu cauucuuacc ugguguuuau ucuguuauuu acuuguacuu 9600

gacauuuuau cuuacuaaug auguuucuuu uuuagcacau auucagugga ugguuauguu 9660

cacaccuuua guaccuuucu ggauaacaau ugcuuauauc auuuguauuu ccacaaagca 9720

uuucuauugg uucuuuagua auuaccuaaa gagacgugua gucuuuaaug guguuuccuu 9780

uaguacuuuu gaagaagcug cgcugugcac cuuuuuguua aauaaagaaa uguaucuaaa 9840

guugcguagu gaugugcuau uaccucuuac gcaauauaau agauacuuag cucuuuauaa 9900

uaaguacaag uauuuuagug gagcaaugga uacaacuagc uacagagaag cugcuuguug 9960

ucaucucgca aaggcucuca augacuucag uaacucaggu ucugauguuc uuuaccaacc 10020

accacaaacc ucuaucaccu cagcuguuuu gcagaguggu uuuagaaaaa uggcauuccc 10080

aucugguaaa guugaggguu guaugguaca aguaacuugu gguacaacua cacuuaacgg 10140

ucuuuggcuu gaugacguag uuuacugucc aagacaugug aucugcaccu cugaagacau 10200

gcuuaacccu aauuaugaag auuuacucau ucguaagucu aaucauaauu ucuugguaca 10260

ggcugguaau guucaacuca ggguuauugg acauucuaug caaaauugug uacuuaagcu 10320

uaagguugau acagccaauc cuaagacacc uaaguauaag uuuguucgca uucaaccagg 10380

acagacuuuu ucaguguuag cuuguuacaa ugguucacca ucugguguuu accaaugugc 10440

uaugaggccc aauuucacua uuaaggguuc auuccuuaau gguucaugug guaguguugg 10500

uuuuaacaua gauuaugacu gugucucuuu uuguuacaug caccauaugg aauuaccaac 10560

uggaguucau gcuggcacag acuuagaagg uaacuuuuau ggaccuuuug uugacaggca 10620

aacagcacaa gcagcuggua cggacacaac uauuacaguu aauguuuuag cuugguugua 10680

cgcugcuguu auaaauggag acaggugguu ucucaaucga uuuaccacaa cucuuaauga 10740

cuuuaaccuu guggcuauga aguacaauua ugaaccucua acacaagacc auguugacau 10800

acuaggaccu cuuucugcuc aaacuggaau ugccguuuua gauaugugug cuucauuaaa 10860

agaauuacug caaaauggua ugaauggacg uaccauauug gguagugcuu uauuagaaga 10920

ugaauuuaca ccuuuugaug uuguuagaca augcucaggu guuacuuucc aaagugcagu 10980

gaaaagaaca aucaagguua cacaccacug guuguuacuc acaauuuuga cuucacuuuu 11040

aguuuuaguc cagaguacuc aauggucuuu guucuuuuuu uuguaugaaa augccuuuuu 11100

accuuuugcu auggguauua uugcuauguc ugcuuuugca augauguuug ucaaacauaa 11160

gcaugcauuu cucuguuugu uuuuguuacc uucucuugcc acuguagcuu auuuuaauau 11220

ggucuauaug ccugcuaguu gggugaugcg uauuaugaca ugguuggaua ugguugauac 11280

uaguuugucu gguuuuaagc uaaaagacug uguuauguau gcaucagcug uaguguuacu 11340

aauccuuaug acagcaagaa cuguguauga ugauggugcu aggagagugu ggacacuuau 11400

gaaugucuug acacucguuu auaaaguuua uuaugguaau gcuuuagauc aagccauuuc 11460

caugugggcu cuuauaaucu cuguuacuuc uaacuacuca gguguaguua caacugucau 11520

guuuuuggcc agagguauug uuuuuaugug uguugaguau ugcccuauuu ucuucauaac 11580

ugguaauaca cuucagugua uaaugcuagu uuauuguuuc uuaggcuauu uuuguacuug 11640

uuacuuuggc cucuuuuguu uacucaaccg cuacuuuaga cugacucuug guguuuauga 11700

uuacuuaguu ucuacacagg aguuuagaua uaugaauuca cagggacuac ucccacccaa 11760

gaauagcaua gaugccuuca aacucaacau uaaauuguug gguguuggug gcaaaccuug 11820

uaucaaagua gccacuguac agucuaaaau gucagaugua aagugcacau caguagucuu 11880

acucucaguu uugcaacaac ucagaguaga aucaucaucu aaauuguggg cucaaugugu 11940

ccaguuacac aaugacauuc ucuuagcuaa agauacuacu gaagccuuug aaaaaauggu 12000

uucacuacuu ucuguuuugc uuuccaugca gggugcugua gacauaaaca agcuuuguga 12060

agaaaugcug gacaacaggg caaccuuaca agcuauagcc ucagaguuua guucccuucc 12120

aucauaugca gcuuuugcua cugcucaaga agcuuaugag caggcuguug cuaaugguga 12180

uucugaaguu guucuuaaaa aguugaagaa gucuuugaau guggcuaaau cugaauuuga 12240

ccgugaugca gccaugcaac guaaguugga aaagauggcu gaucaagcua ugacccaaau 12300

guauaaacag gcuagaucug aggacaagag ggcaaaaguu acuagugcua ugcagacaau 12360

gcuuuucacu augcuuagaa aguuggauaa ugaugcacuc aacaacauua ucaacaaugc 12420

aagagauggu uguguucccu ugaacauaau accucuuaca acagcagcca aacuaauggu 12480

ugucauacca gacuauaaca cauauaaaaa uacgugugau gguacaacau uuacuuaugc 12540

aucagcauug ugggaaaucc aacagguugu agaugcagau aguaaaauug uucaacuuag 12600

ugaaauuagu auggacaauu caccuaauuu agcauggccu cuuauuguaa cagcuuuaag 12660

ggccaauucu gcugucaaau uacagaauaa ugagcuuagu ccuguugcac uacgacagau 12720

gucuugugcu gccgguacua cacaaacugc uugcacugau gacaaugcgu uagcuuacua 12780

caacacaaca aagggaggua gguuuguacu ugcacuguua uccgauuuac aggauuugaa 12840

augggcuaga uucccuaaga gugauggaac ugguacuauc uauacagaac uggaaccacc 12900

uuguagguuu guuacagaca caccuaaagg uccuaaagug aaguauuuau acuuuauuaa 12960

aggauuaaac aaccuaaaua gagguauggu acuugguagu uuagcugcca caguacgucu 13020

acaagcuggu aaugcaacag aagugccugc caauucaacu guauuaucuu ucugugcuuu 13080

ugcuguagau gcugcuaaag cuuacaaaga uuaucuagcu agugggggac aaccaaucac 13140

uaauuguguu aagauguugu guacacacac ugguacuggu caggcaauaa caguuacacc 13200

ggaagccaau auggaucaag aauccuuugg uggugcaucg uguugucugu acugccguug 13260

ccacauagau cauccaaauc cuaaaggauu uugugacuua aaagguaagu auguacaaau 13320

accuacaacu ugugcuaaug acccuguggg uuuuacacuu aaaaacacag ucuguaccgu 13380

cugcgguaug uggaaagguu auggcuguag uugugaucaa cuccgcgaac ccaugcuuca 13440

gucagcugau gcacaaucgu uuuuaaacgg guuugcggug uaagugcagc ccgucuuaca 13500

ccgugcggca caggcacuag uacugauguc guauacaggg cuuuugacau cuacaaugau 13560

aaaguagcug guuuugcuaa auuccuaaaa acuaauuguu gucgcuucca agaaaaggac 13620

gaagaugaca auuuaauuga uucuuacuuu guaguuaaga gacacacuuu cucuaacuac 13680

caacaugaag aaacaauuua uaauuuacuu aaggauuguc cagcuguugc uaaacaugac 13740

uucuuuaagu uuagaauaga cggugacaug guaccacaua uaucacguca acgucuuacu 13800

aaauacacaa uggcagaccu cgucuaugcu uuaaggcauu uugaugaagg uaauugugac 13860

acauuaaaag aaauacuugu cacauacaau uguugugaug augauuauuu caauaaaaag 13920

gacugguaug auuuuguaga aaacccagau auauuacgcg uauacgccaa cuuaggugaa 13980

cguguacgcc aagcuuuguu aaaaacagua caauucugug augccaugcg aaaugcuggu 14040

auuguuggug uacugacauu agauaaucaa gaucucaaug guaacuggua ugauuucggu 14100

gauuucauac aaaccacgcc agguagugga guuccuguug uagauucuua uuauucauug 14160

uuaaugccua uauuaaccuu gaccagggcu uuaacugcag agucacaugu ugacacugac 14220

uuaacaaagc cuuacauuaa gugggauuug uuaaaauaug acuucacgga agagagguua 14280

aaacucuuug accguuauuu uaaauauugg gaucagacau accacccaaa uuguguuaac 14340

uguuuggaug acagaugcau ucugcauugu gcaaacuuua auguuuuauu cucuacagug 14400

uucccaccua caaguuuugg accacuagug agaaaaauau uuguugaugg uguuccauuu 14460

guaguuucaa cuggauacca cuucagagag cuagguguug uacauaauca ggauguaaac 14520

uuacauagcu cuagacuuag uuuuaaggaa uuacuugugu augcugcuga cccugcuaug 14580

cacgcugcuu cugguaaucu auuacuagau aaacgcacua cgugcuuuuc aguagcugca 14640

cuuacuaaca auguugcuuu ucaaacuguc aaacccggua auuuuaacaa agacuucuau 14700

gacuuugcug ugucuaaggg uuucuuuaag gaaggaaguu cuguugaauu aaaacacuuc 14760

uucuuugcuc aggaugguaa ugcugcuauc agcgauuaug acuacuaucg uuauaaucua 14820

ccaacaaugu gugauaucag acaacuacua uuuguaguug aaguuguuga uaaguacuuu 14880

gauuguuacg augguggcug uauuaaugcu aaccaaguca ucgucaacaa ccuagacaaa 14940

ucagcugguu uuccauuuaa uaaauggggu aaggcuagac uuuauuauga uucaaugagu 15000

uaugaggauc aagaugcacu uuucgcauau acaaaacgua augucauccc uacuauaacu 15060

caaaugaauc uuaaguaugc cauuagugca aagaauagag cucgcaccgu agcugguguc 15120

ucuaucugua guacuaugac caauagacag uuucaucaaa aauuauugaa aucaauagcc 15180

gccacuagag gagcuacugu aguaauugga acaagcaaau ucuauggugg uuggcacaac 15240

auguuaaaaa cuguuuauag ugauguagaa aacccucacc uuauggguug ggauuauccu 15300

aaaugugaua gagccaugcc uaacaugcuu agaauuaugg ccucacuugu ucuugcucgc 15360

aaacauacaa cguguuguag cuugucacac cguuucuaua gauuagcuaa ugagugugcu 15420

caaguauuga gugaaauggu cauguguggc gguucacuau auguuaaacc agguggaacc 15480

ucaucaggag augccacaac ugcuuaugcu aauaguguuu uuaacauuug ucaagcuguc 15540

acggccaaug uuaaugcacu uuuaucuacu gaugguaaca aaauugccga uaaguauguc 15600

cgcaauuuac aacacagacu uuaugagugu cucuauagaa auagagaugu ugacacagac 15660

uuugugaaug aguuuuacgc auauuugcgu aaacauuucu caaugaugau acucucugac 15720

gaugcuguug uguguuucaa uagcacuuau gcaucucaag gucuaguggc uagcauaaag 15780

aacuuuaagu caguucuuua uuaucaaaac aauguuuuua ugucugaagc aaaauguugg 15840

acugagacug accuuacuaa aggaccucau gaauuuugcu cucaacauac aaugcuaguu 15900

aaacagggug augauuaugu guaccuuccu uacccagauc caucaagaau ccuaggggcc 15960

ggcuguuuug uagaugauau cguaaaaaca gaugguacac uuaugauuga acgguucgug 16020

ucuuuagcua uagaugcuua cccacuuacu aaacauccua aucaggagua ugcugauguc 16080

uuucauuugu acuuacaaua cauaagaaag cuacaugaug aguuaacagg acacauguua 16140

gacauguauu cuguuaugcu uacuaaugau aacacuucaa gguauuggga accugaguuu 16200

uaugaggcua uguacacacc gcauacaguc uuacaggcug uuggggcuug uguucuuugc 16260

aauucacaga cuucauuaag auggugugcu ugcauacgua gaccauucuu auguuguaaa 16320

ugcuguuacg accaugucau aucaacauca cauaaauuag ucuugucugu uaauccguau 16380

guuugcaaug cuccagguug ugaugucaca gaugugacuc aacuuuacuu aggagguaug 16440

agcuauuauu guaaaucaca uaaaccaccc auuaguuuuc cauugugugc uaauggacaa 16500

guuuuugguu uauauaaaaa uacauguguu gguagcgaua auguuacuga cuuuaaugca 16560

auugcaacau gugacuggac aaaugcuggu gauuacauuu uagcuaacac cuguacugaa 16620

agacucaagc uuuuugcagc agaaacgcuc aaagcuacug aggagacauu uaaacugucu 16680

uaugguauug cuacuguacg ugaagugcug ucugacagag aauuacaucu uucaugggaa 16740

guugguaaac cuagaccacc acuuaaccga aauuaugucu uuacugguua ucguguaacu 16800

aaaaacagua aaguacaaau aggagaguac accuuugaaa aaggugacua uggugaugcu 16860

guuguuuacc gagguacaac aacuuacaaa uuaaauguug gugauuauuu ugugcugaca 16920

ucacauacag uaaugccauu aagugcaccu acacuagugc cacaagagca cuauguuaga 16980

auuacuggcu uauacccaac acucaauauc ucagaugagu uuucuagcaa uguugcaaau 17040

uaucaaaagg uugguaugca aaaguauucu acacuccagg gaccaccugg uacugguaag 17100

agucauuuug cuauuggccu agcucucuac uacccuucug cucgcauagu guauacagcu 17160

ugcucucaug ccgcuguuga ugcacuaugu gagaaggcau uaaaauauuu gccuauagau 17220

aaauguagua gaauuauacc ugcacgugcu cguguagagu guuuugauaa auucaaagug 17280

aauucaacau uagaacagua ugucuuuugu acuguaaaug cauugccuga gacgacagca 17340

gauauaguug ucuuugauga aauuucaaug gccacaaauu augauuugag uguugucaau 17400

gccagauuac gugcuaagca cuauguguac auuggcgacc cugcucaauu accugcacca 17460

cgcacauugc uaacuaaggg cacacuagaa ccagaauauu ucaauucagu guguagacuu 17520

augaaaacua uagguccaga cauguuccuc ggaacuuguc ggcguugucc ugcugaaauu 17580

guugacacug ugagugcuuu gguuuaugau aauaagcuua aagcacauaa agacaaauca 17640

gcucaaugcu uuaaaauguu uuauaagggu guuaucacgc augauguuuc aucugcaauu 17700

aacaggccac aaauaggcgu gguaagagaa uuccuuacac guaacccugc uuggagaaaa 17760

gcugucuuua uuucaccuua uaauucacag aaugcuguag ccucaaagau uuugggacua 17820

ccaacucaaa cuguugauuc aucacagggc ucagaauaug acuaugucau auucacucaa 17880

accacugaaa cagcucacuc uuguaaugua aacagauuua auguugcuau uaccagagca 17940

aaaguaggca uacuuugcau aaugucugau agagaccuuu augacaaguu gcaauuuaca 18000

agucuugaaa uuccacguag gaauguggca acuuuacaag cugaaaaugu aacaggacuc 18060

uuuaaagauu guaguaaggu aaucacuggg uuacauccua cacaggcacc uacacaccuc 18120

aguguugaca cuaaauucaa aacugaaggu uuauguguug acauaccugg cauaccuaag 18180

gacaugaccu auagaagacu caucucuaug auggguuuua aaaugaauua ucaaguuaau 18240

gguuacccua acauguuuau cacccgcgaa gaagcuauaa gacauguacg ugcauggauu 18300

ggcuucgaug ucgaggggug ucaugcuacu agagaagcug uugguaccaa uuuaccuuua 18360

cagcuagguu uuucuacagg uguuaaccua guugcuguac cuacagguua uguugauaca 18420

ccuaauaaua cagauuuuuc cagaguuagu gcuaaaccac cgccuggaga ucaauuuaaa 18480

caccucauac cacuuaugua caaaggacuu ccuuggaaug uagugcguau aaagauugua 18540

caaauguuaa gugacacacu uaaaaaucuc ucugacagag ucguauuugu cuuaugggca 18600

cauggcuuug aguugacauc uaugaaguau uuugugaaaa uaggaccuga gcgcaccugu 18660

ugucuaugug auagacgugc cacaugcuuu uccacugcuu cagacacuua ugccuguugg 18720

caucauucua uuggauuuga uuacgucuau aauccguuua ugauugaugu ucaacaaugg 18780

gguuuuacag guaaccuaca aagcaaccau gaucuguauu gucaagucca ugguaaugca 18840

cauguagcua guugugaugc aaucaugacu aggugucuag cuguccacga gugcuuuguu 18900

aagcguguug acuggacuau ugaauauccu auaauuggug augaacugaa gauuaaugcg 18960

gcuuguagaa agguucaaca caugguuguu aaagcugcau uauuagcaga caaauuccca 19020

guucuucacg acauugguaa cccuaaagcu auuaagugug uaccucaagc ugauguagaa 19080

uggaaguucu augaugcaca gccuuguagu gacaaagcuu auaaaauaga agaauuauuc 19140

uauucuuaug ccacacauuc ugacaaauuc acagauggug uaugccuauu uuggaauugc 19200

aaugucgaua gauauccugc uaauuccauu guuuguagau uugacacuag agugcuaucu 19260

aaccuuaacu ugccugguug ugaugguggc aguuuguaug uaaauaaaca ugcauuccac 19320

acaccagcuu uugauaaaag ugcuuuuguu aauuuaaaac aauuaccauu uuucuauuac 19380

ucugacaguc caugugaguc ucauggaaaa caaguagugu cagauauaga uuauguacca 19440

cuaaagucug cuacguguau aacacguugc aauuuaggug gugcugucug uagacaucau 19500

gcuaaugagu acagauugua ucucgaugcu uauaacauga ugaucucagc uggcuuuagc 19560

uuguggguuu acaaacaauu ugauacuuau aaccucugga acacuuuuac aagacuucag 19620

aguuuagaaa auguggcuuu uaauguugua aauaagggac acuuugaugg acaacagggu 19680

gaaguaccag uuucuaucau uaauaacacu guuuacacaa aaguugaugg uguugaugua 19740

gaauuguuug aaaauaaaac aacauuaccu guuaauguag cauuugagcu uugggcuaag 19800

cgcaacauua aaccaguacc agaggugaaa auacucaaua auuugggugu ggacauugcu 19860

gcuaauacug ugaucuggga cuacaaaaga gaugcuccag cacauauauc uacuauuggu 19920

guuuguucua ugacugacau agccaagaaa ccaacugaaa cgauuugugc accacucacu 19980

gucuuuuuug augguagagu ugauggucaa guagacuuau uuagaaaugc ccguaauggu 20040

guucuuauua cagaagguag uguuaaaggu uuacaaccau cuguaggucc caaacaagcu 20100

agucuuaaug gagucacauu aauuggagaa gccguaaaaa cacaguucaa uuauuauaag 20160

aaaguugaug guguugucca acaauuaccu gaaacuuacu uuacucagag uagaaauuua 20220

caagaauuua aacccaggag ucaaauggaa auugauuucu uagaauuagc uauggaugaa 20280

uucauugaac gguauaaauu agaaggcuau gccuucgaac auaucguuua uggagauuuu 20340

agucauaguc aguuaggugg uuuacaucua cugauuggac uagcuaaacg uuuuaaggaa 20400

ucaccuuuug aauuagaaga uuuuauuccu auggacagua caguuaaaaa cuauuucaua 20460

acagaugcgc aaacagguuc aucuaagugu guguguucug uuauugauuu auuacuugau 20520

gauuuuguug aaauaauaaa aucccaagau uuaucuguag uuucuaaggu ugucaaagug 20580

acuauugacu auacagaaau uucauuuaug cuuuggugua aagauggcca uguagaaaca 20640

uuuuacccaa aauuacaauc uagucaagcg uggcaaccgg guguugcuau gccuaaucuu 20700

uacaaaaugc aaagaaugcu auuagaaaag ugugaccuuc aaaauuaugg ugauagugca 20760

acauuaccua aaggcauaau gaugaauguc gcaaaauaua cucaacugug ucaauauuua 20820

aacacauuaa cauuagcugu acccuauaau augagaguua uacauuuugg ugcugguucu 20880

gauaaaggag uugcaccagg uacagcuguu uuaagacagu gguugccuac ggguacgcug 20940

cuugucgauu cagaucuuaa ugacuuuguc ucugaugcag auucaacuuu gauuggugau 21000

ugugcaacug uacauacagc uaauaaaugg gaucucauua uuagugauau guacgacccu 21060

aagacuaaaa auguuacaaa agaaaaugac ucuaaagagg guuuuuucac uuacauuugu 21120

ggguuuauac aacaaaagcu agcucuugga gguuccgugg cuauaaagau aacagaacau 21180

ucuuggaaug cugaucuuua uaagcucaug ggacacuucg caugguggac agccuuuguu 21240

acuaauguga augcgucauc aucugaagca uuuuuaauug gauguaauua ucuuggcaaa 21300

ccacgcgaac aaauagaugg uuaugucaug caugcaaauu acauauuuug gaggaauaca 21360

aauccaauuc aguugucuuc cuauucuuua uuugacauga guaaauuucc ccuuaaauua 21420

agggguacug cuguuauguc uuuaaaagaa ggucaaauca augauaugau uuuaucucuu 21480

cuuaguaaag guagacuuau aauuagagaa aacaacagag uuguuauuuc uagugauguu 21540

cuuguuaaca acuaaacgaa caauguuugu uuuucuuguu uuauugccac uagucucuag 21600

ucaguguguu aaucuuacaa ccagaacuca auuacccccu gcauacacua auucuuucac 21660

acgugguguu uauuacccug acaaaguuuu cagauccuca guuuuacauu caacucagga 21720

cuuguucuua ccuuucuuuu ccaauguuac uugguuccau gcuauacaug ucucugggac 21780

caaugguacu aagagguuug auaacccugu ccuaccauuu aaugauggug uuuauuuugc 21840

uuccacugag aagucuaaca uaauaagagg cuggauuuuu gguacuacuu uagauucgaa 21900

gacccagucc cuacuuauug uuaauaacgc uacuaauguu guuauuaaag ucugugaauu 21960

ucaauuuugu aaugauccau uuuugggugu uuauuaccac aaaaacaaca aaaguuggau 22020

ggaaagugag uucagaguuu auucuagugc gaauaauugc acuuuugaau augucucuca 22080

gccuuuucuu auggaccuug aaggaaaaca ggguaauuuc aaaaaucuua gggaauuugu 22140

guuuaagaau auugaugguu auuuuaaaau auauucuaag cacacgccua uuaauuuagu 22200

gcgugaucuc ccucaggguu uuucggcuuu agaaccauug guagauuugc caauagguau 22260

uaacaucacu agguuucaaa cuuuacuugc uuuacauaga aguuauuuga cuccugguga 22320

uucuucuuca gguuggacag cuggugcugc agcuuauuau gugggguauc uucaaccuag 22380

gacuuuucua uuaaaauaua augaaaaugg aaccauuaca gaugcuguag acugugcacu 22440

ugacccucuc ucagaaacaa aguguacguu gaaauccuuc acuguagaaa aaggaaucua 22500

ucaaacuucu aacuuuagag uccaaccaac agaaucuauu guuagauuuc cuaauauuac 22560

aaacuugugc ccuuuuggug aaguuuuuaa cgccaccaga uuugcaucug uuuaugcuug 22620

gaacaggaag agaaucagca acuguguugc ugauuauucu guccuauaua auuccgcauc 22680

auuuuccacu uuuaaguguu auggaguguc uccuacuaaa uuaaaugauc ucugcuuuac 22740

uaaugucuau gcagauucau uuguaauuag aggugaugaa gucagacaaa ucgcuccagg 22800

gcaaacugga aagauugcug auuauaauua uaaauuacca gaugauuuua caggcugcgu 22860

uauagcuugg aauucuaaca aucuugauuc uaagguuggu gguaauuaua auuaccugua 22920

uagauuguuu aggaagucua aucucaaacc uuuugagaga gauauuucaa cugaaaucua 22980

ucaggccggu agcacaccuu guaauggugu ugaagguuuu aauuguuacu uuccuuuaca 23040

aucauauggu uuccaaccca cuaauggugu ugguuaccaa ccauacagag uaguaguacu 23100

uucuuuugaa cuucuacaug caccagcaac uguuugugga ccuaaaaagu cuacuaauuu 23160

gguuaaaaac aaauguguca auuucaacuu caaugguuua acaggcacag guguucuuac 23220

ugagucuaac aaaaaguuuc ugccuuucca acaauuuggc agagacauug cugacacuac 23280

ugaugcuguc cgugauccac agacacuuga gauucuugac auuacaccau guucuuuugg 23340

uggugucagu guuauaacac caggaacaaa uacuucuaac cagguugcug uucuuuauca 23400

ggauguuaac ugcacagaag ucccuguugc uauucaugca gaucaacuua cuccuacuug 23460

gcguguuuau ucuacagguu cuaauguuuu ucaaacacgu gcaggcuguu uaauaggggc 23520

ugaacauguc aacaacucau augaguguga cauacccauu ggugcaggua uaugcgcuag 23580

uuaucagacu cagacuaauu cuccucggcg ggcacguagu guagcuaguc aauccaucau 23640

ugccuacacu augucacuug gugcagaaaa uucaguugcu uacucuaaua acucuauugc 23700

cauacccaca aauuuuacua uuaguguuac cacagaaauu cuaccagugu cuaugaccaa 23760

gacaucagua gauuguacaa uguacauuug uggugauuca acugaaugca gcaaucuuuu 23820

guugcaauau ggcaguuuuu guacacaauu aaaccgugcu uuaacuggaa uagcuguuga 23880

acaagacaaa aacacccaag aaguuuuugc acaagucaaa caaauuuaca aaacaccacc 23940

aauuaaagau uuugguguuu uuaauuuuuc acaaauauua ccagauccau caaaaccaag 24000

caagagguca uuuauugaag aucuacuuuu caacaaagug acacuugcag augcuggcuu 24060

caucaaacaa uauggugauu gccuugguga uauugcugcu agagaccuca uuugugcaca 24120

aaaguuuaac ggccuuacug uuuugccacc uuugcucaca gaugaaauga uugcucaaua 24180

cacuucugca cuguuagcgg guacaaucac uucugguugg accuuuggug caggugcugc 24240

auuacaaaua ccauuugcua ugcaaauggc uuauagguuu aaugguauug gaguuacaca 24300

gaauguucuc uaugagaacc aaaaauugau ugccaaccaa uuuaauagug cuauuggcaa 24360

aauucaagac ucacuuucuu ccacagcaag ugcacuugga aaacuucaag auguggucaa 24420

ccaaaaugca caagcuuuaa acacgcuugu uaaacaacuu agcuccaauu uuggugcaau 24480

uucaaguguu uuaaaugaua uccuuucacg ucuugacaaa guugaggcug aagugcaaau 24540

ugauagguug aucacaggca gacuucaaag uuugcagaca uaugugacuc aacaauuaau 24600

uagagcugca gaaaucagag cuucugcuaa ucuugcugcu acuaaaaugu cagagugugu 24660

acuuggacaa ucaaaaagag uugauuuuug uggaaagggc uaucaucuua uguccuuccc 24720

ucagucagca ccucauggug uagucuucuu gcaugugacu uaugucccug cacaagaaaa 24780

gaacuucaca acugcuccug ccauuuguca ugauggaaaa gcacacuuuc cucgugaagg 24840

ugucuuuguu ucaaauggca cacacugguu uguaacacaa aggaauuuuu augaaccaca 24900

aaucauuacu acagacaaca cauuuguguc ugguaacugu gauguuguaa uaggaauugu 24960

caacaacaca guuuaugauc cuuugcaacc ugaauuagac ucauucaagg aggaguuaga 25020

uaaauauuuu aagaaucaua caucaccaga uguugauuua ggugacaucu cuggcauuaa 25080

ugcuucaguu guaaacauuc aaaaagaaau ugaccgccuc aaugagguug ccaagaauuu 25140

aaaugaaucu cucaucgauc uccaagaacu uggaaaguau gagcaguaua uaaaauggcc 25200

augguacauu uggcuagguu uuauagcugg cuugauugcc auaguaaugg ugacaauuau 25260

gcuuugcugu augaccaguu gcuguaguug ucucaagggc uguuguucuu guggauccug 25320

cugcaaauuu gaugaagacg acucugagcc agugcucaaa ggagucaaau uacauuacac 25380

auaaacgaac uuauggauuu guuuaugaga aucuucacaa uuggaacugu aacuuugaag 25440

caaggugaaa ucaaggaugc uacuccuuca gauuuuguuc gcgcuacugc aacgauaccg 25500

auacaagccu cacucccuuu cggauggcuu auuguuggcg uugcacuucu ugcuguuuuu 25560

cagagcgcuu ccaaaaucau aacccucaaa aagagauggc aacuagcacu cuccaagggu 25620

guucacuuug uuugcaacuu gcuguuguug uuuguaacag uuuacucaca ccuuuugcuc 25680

guugcugcug gccuugaagc cccuuuucuc uaucuuuaug cuuuagucua cuucuugcag 25740

aguauaaacu uuguaagaau aauaaugagg cuuuggcuuu gcuggaaaug ccguuccaaa 25800

aacccauuac uuuaugaugc caacuauuuu cuuugcuggc auacuaauug uuacgacuau 25860

uguauaccuu acaauagugu aacuucuuca auugucauua cuucagguga uggcacaaca 25920

aguccuauuu cugaacauga cuaccagauu ggugguuaua cugaaaaaug ggaaucugga 25980

guaaaagacu guguuguauu acacaguuac uucacuucag acuauuacca gcuguacuca 26040

acucaauuga guacagacac ugguguugaa cauguuaccu ucuucaucua caauaaaauu 26100

guugaugagc cugaagaaca uguccaaauu cacacaaucg acgguucauc cggaguuguu 26160

aauccaguaa uggaaccaau uuaugaugaa ccgacgacga cuacuagcgu gccuuuguaa 26220

gcacaagcug augaguacga acuuauguac ucauucguuu cggaagagac agguacguua 26280

auaguuaaua gcguacuucu uuuucuugcu uucgugguau ucuugcuagu uacacuagcc 26340

auccuuacug cgcuucgauu gugugcguac ugcugcaaua uuguuaacgu gagucuugua 26400

aaaccuucuu uuuacguuua cucucguguu aaaaaucuga auucuucuag aguuccugau 26460

cuucuggucu aaacgaacua aauauuauau uaguuuuucu guuuggaacu uuaauuuuag 26520

ccauggcaga uuccaacggu acuauuaccg uugaagagcu uaaaaagcuc cuugaacaau 26580

ggaaccuagu aauagguuuc cuauuccuua cauggauuug ucuucuacaa uuugccuaug 26640

ccaacaggaa uagguuuuug uauauaauua aguuaauuuu ccucuggcug uuauggccag 26700

uaacuuuagc uuguuuugug cuugcugcug uuuacagaau aaauuggauc accgguggaa 26760

uugcuaucgc aauggcuugu cuuguaggcu ugauguggcu cagcuacuuc auugcuucuu 26820

ucagacuguu ugcgcguacg cguuccaugu ggucauucaa uccagaaacu aacauucuuc 26880

ucaacgugcc acuccauggc acuauucuga ccagaccgcu ucuagaaagu gaacucguaa 26940

ucggagcugu gauccuucgu ggacaucuuc guauugcugg acaccaucua ggacgcugug 27000

acaucaagga ccugccuaaa gaaaucacug uugcuacauc acgaacgcuu ucuuauuaca 27060

aauugggagc uucgcagcgu guagcaggug acucagguuu ugcugcauac agucgcuaca 27120

ggauuggcaa cuauaaauua aacacagacc auuccaguag cagugacaau auugcuuugc 27180

uuguacagua agugacaaca gauguuucau cucguugacu uucagguuac uauagcagag 27240

auauuacuaa uuauuaugag gacuuuuaaa guuuccauuu ggaaucuuga uuacaucaua 27300

aaccucauaa uuaaaaauuu aucuaaguca cuaacugaga auaaauauuc ucaauuagau 27360

gaagagcaac caauggagau ugauuaaacg aacaugaaaa uuauucuuuu cuuggcacug 27420

auaacacucg cuacuuguga gcuuuaucac uaccaagagu guguuagagg uacaacagua 27480

cuuuuaaaag aaccuugcuc uucuggaaca uacgagggca auucaccauu ucauccucua 27540

gcugauaaca aauuugcacu gacuugcuuu agcacucaau uugcuuuugc uuguccugac 27600

ggcguaaaac acgucuauca guuacgugcc agaucaguuu caccuaaacu guucaucaga 27660

caagaggaag uucaagaacu uuacucucca auuuuucuua uuguugcggc aauaguguuu 27720

auaacacuuu gcuucacacu caaaagaaag acagaaugau ugaacuuuca uuaauugacu 27780

ucuauuugug cuuuuuagcc uuucugcuau uccuuguuuu aauuaugcuu auuaucuuuu 27840

gguucucacu ugaacugcaa gaucauaaug aaacuuguca cgccuaaacg aacaugaaau 27900

uucuuguuuu cuuaggaauc aucacaacug uagcugcauu ucaccaagaa uguaguuuac 27960

agucauguac ucaacaucaa ccauauguag uugaugaccc guguccuauu cacuucuauu 28020

cuaaauggua uauuagagua ggagcuagaa aaucagcacc uuuaauugaa uugugcgugg 28080

augaggcugg uucuaaauca cccauucagu acaucgauau cgguaauuau acaguuuccu 28140

guuuaccuuu uacaauuaau ugccaggaac cuaaauuggg uagucuugua gugcguuguu 28200

cguucuauga agacuuuuua gaguaucaug acguucgugu uguuuuagau uucaucuaaa 28260

cgaacaaacu aaaaugucug auaauggacc ccaaaaucag cgaaaugcac cccgcauuac 28320

guuuggugga cccucagauu caacuggcag uaaccagaau ggagaacgca guggggcgcg 28380

aucaaaacaa cgucggcccc aagguuuacc caauaauacu gcgucuuggu ucaccgcucu 28440

cacucaacau ggcaaggaag accuuaaauu cccucgagga caaggcguuc caauuaacac 28500

caauagcagu ccagaugacc aaauuggcua cuaccgaaga gcuaccagac gaauucgugg 28560

uggugacggu aaaaugaaag aucucagucc aagaugguau uucuacuacc uaggaacugg 28620

gccagaagcu ggacuucccu auggugcuaa caaagacggc aucauauggg uugcaacuga 28680

gggagccuug aauacaccaa aagaucacau uggcacccgc aauccugcua acaaugcugc 28740

aaucgugcua caacuuccuc aaggaacaac auugccaaaa ggcuucuacg cagaagggag 28800

cagaggcggc agucaagccu cuucucguuc cucaucacgu agucgcaaca guucaagaaa 28860

uucaacucca ggcagcagua ggggaacuuc uccugcuaga auggcuggca auggcgguga 28920

ugcugcucuu gcuuugcugc ugcuugacag auugaaccag cuugagagca aaaugucugg 28980

uaaaggccaa caacaacaag gccaaacugu cacuaagaaa ucugcugcug aggcuucuaa 29040

gaagccucgg caaaaacgua cugccacuaa agcauacaau guaacacaag cuuucggcag 29100

acguggucca gaacaaaccc aaggaaauuu uggggaccag gaacuaauca gacaaggaac 29160

ugauuacaaa cauuggccgc aaauugcaca auuugccccc agcgcuucag cguucuucgg 29220

aaugucgcgc auuggcaugg aagucacacc uucgggaacg ugguugaccu acacaggugc 29280

caucaaauug gaugacaaag auccaaauuu caaagaucaa gucauuuugc ugaauaagca 29340

uauugacgca uacaaaacau ucccaccaac agagccuaaa aaggacaaaa agaagaaggc 29400

ugaugaaacu caagccuuac cgcagagaca gaagaaacag caaacuguga cucuucuucc 29460

ugcugcagau uuggaugauu ucuccaaaca auugcaacaa uccaugagca gugcugacuc 29520

aacucaggcc uaaacucaug cagaccacac aaggcagaug ggcuauauaa acguuuucgc 29580

uuuuccguuu acgauauaua gucuacucuu gugcagaaug aauucucgua acuacauagc 29640

acaaguagau guaguuaacu uuaaucucac auagcaaucu uuaaucagug uguaacauua 29700

gggaggacuu gaaagagcca ccacauuuuc accgaggcca cgcggaguac gaucgagugu 29760

acagugaaca augcuaggga gagcugccua uauggaagag cccuaaugug uaaaauuaau 29820

uuuaguagug cuauccccau gugauuuuaa uagcuucuua ggagaaugac aaaaaaaaaa 29880

aaaaaaaaaa aaaaaaaaaa aaa 29903

<210> 35
<211> 33
<212> RNA
<213> Artificial Sequence

<220>
<223> Target sequence RSE Circ chikvRSE

<400> 35
agcaaauaau cuauagauca aagggcuacg caa 33

<210> 36
<211> 294
<212> RNA
<213> Artificial Sequence

<220>
<223> Artificial circular ARN 1; target disruption structure comprised
      in SARS-CoV-2 3'UTR replication site

<400> 36
ugcaguuaga uacugcuugu guuauguggu ugugaggguu uauuuugaga uccugcuggu 60

uacuugugcu guguaguuau gagaguuugu uuuggauacc uuuagccugc uuguguugug 120

ugguugcgag gauuuauucu gcucggaaac gauuuguuug uguuauguag uuguggggau 180

ucauucuggc ugcuuccucu uuauuugugu uguguaguua cgggaguucg uuuuguccca 240

cauagaguug cuugugcuau gugguuacgg ggauuuauuu ugccagggcc aaac 294

<210> 37
<211> 29
<212> RNA
<213> Artificial Sequence

<220>

<223> Target sequence SL III 3′ UTR circ_wnv_slII_1/2

<400> 37
uuuugaggag aaagucaggc cgggaaguu                                    29


<210> 38
<211> 294
<212> RNA
<213> Artificial Sequence


<220>
<223> Artificial circular ARN 2; target disruption structure comprised
      in SARS-CoV-2 3′UTR replication site

<400> 38
ugcaguuaga uacugcuugu guuauguggu ugugaggguu uauuuugaga uccugcuggu      60

uacuugugcu guguaguuau gagaguuugu uuuggauacc uuuagccugc uuguguugug     120

ugguugcgag gauuuauucu gcucggaaac gauuuguuug uguuauguag uuguggggau     180

ucauucuggc ugcuuccucu uuguuugugu uguguaguua cggggguucg uuuuguccca      240

cauagaguua cuugugcuau guaguuauga ggauucauuu ugccagggcc aaac           294


<210> 39
<211> 294
<212> RNA
<213> Artificial Sequence


<220>
<223> Circ Chikv_re

<400> 39
gcgccgauua uggcuaaaag agucaacguu uuguaguagc ugcggguugc ccucauuacg      60

guuaagaggg uuaauguuuu auagcagcug cccgcgagca ggauuauggu uggaaagguu     120

aacguuuugc agcagcguuc guaacacuuc auuauggcua aggaagccaa cguuuugugg     180

uagccuaggu ucgaaccgau uauggcuaga ggggucagcg uuuuauggua gccaucaaaa     240

ccuuucauua cggcugagag agccaauguu uuacaguagc uccaacgacg uacu           294


<210> 40
<211> 294
<212> RNA
<213> Artificial Sequence


<220>
<223> Artificial circular RNA 3; target disruption structure comprised
      in SARS-CoV-2 3′UTR replication site

<400> 40
ugcaguuaga uacugcuugu guuauguggu ugugaggguu uauuuugaga uccugcuggu      60

uguuugugcu auguaguuac gaggguucau uuuggauacc uuuagccugc uuguguugug     120

ugguugcgag gauuuauucu gcucggaaac gauuuguuug uguuauguag uuguggggau    180

ucauucuggc ugcuuccucu cugcuugugc uguguaguua ugagaguuug uuuuguccca    240

cauagaguua uuuguguugu guaguugugg gaguuuguuu ugccagggcc aaac    294

<210> 41
<211> 294
<212> RNA
<213> Artificial Sequence

<220>
<223> Artificial circular RNA 4; target disruption structure comprised
      in SARS-CoV-2 3'UTR replication site

<400> 41
ugcaguuaga uauuguuugu gcuauguggu uguggggauu cauucugaug ucguauuguu    60

ugcuuguguu gugugguuac gggaauuugu uuugaacucu uaguuguugu uugugcugug    120

uaguuguggg gguuuguucu gauguucuga ucuuuguuug uguuauguag uuacgaggau    180

uuauucuggu gcaacaaguu cuauuugugu uaugugguug cggggguucg uuuugcccgg    240

ccuucgcuua cuuguguugu guaguuguga ggauucguuu uggaugcauc gaac    294

<210> 42
<211> 294
<212> RNA
<213> Artificial Sequence

<220>
<223> Artificial circular RNA 5; target disruption structure comprised
      in SARS-CoV-2 3'UTR replication site

<400> 42
ugcaguuaga uacuauuugu gcuauguagu ugcggggguu uguuuugugg cuuucuuacc    60

uguuuguguu augugguugu gaggauuugu uuuggccgac ucguuccugc uugugcugug    120

uaguuguggg aguucauuuu guucagaguu agccuacuug uguuauguag uuaugagagu    180

ucguuuugga gagacgcaca cuguuugugu ugugugguua uggggauucg uuuugugggu    240

ccggcuccug uuugugcugu gugguuauga gaauuuguuc ugaagauuua uucc    294

<210> 43
<211> 292
<212> RNA
<213> Artificial Sequence

<220>
<223> Artificial circular RNA 1; target disruption structure comprised
      in SARS-CoV-2 5'UTR (SlII)

<400> 43

ugcaguuaga uaagaucugc aggggguuga ggguugguug auccaggcug cuagaucuau 60

aagggguugg gaguugguuc uuuagcuauc ccagauuugu gagggguugg ggguugguua 120

ggcuuacagu uaagaucuau gggagguugg agguugguua ccgccgaguu auagauuuau 180

aggggaucgg aaguugguua aagcuagggc gaagaucugc gggggguugg aaguugguua 240

uacaacgaau uaagauuuau ggggggucgg ggguugguuu aguaguugga ug 292

<210> 44
<211> 292
<212> RNA
<213> Artificial Sequence

<220>
<223> Artificial circular RNA 2; target disruption structure comprised
      in SARS-CoV-2 5'UTR (SlII)

<400> 44
ugcaguuaga uaggaucuau aagggguugg aaguugguua uguggucgcg cgaggucugu 60

aagagauuga aaguugguug ucacugacgu aaagauuuac gagagaucga ggguugguug 120

aaugucgguc ugggguuugu gggggguugg agguugguua uugguauaac ggaggucuau 180

gagagguuga ggguugguuc gcgauagaca ccgggucugc aggaggucgg aaguugguua 240

guugaacaga cuggguuuau ggggggucga gaguugguua uuuguuccga gg 292

<210> 45
<211> 292
<212> RNA
<213> Artificial Sequence

<220>
<223> Artificial circular RNA 3; target disruption structure comprised
      in SARS-CoV-2 5'UTR (SlII)

<400> 45
ugcaguuaga uaagguuugc gagggauugg aaguugguug aaauguggaa ggggauuugc 60

gagagguuga aaguugguuu uuauuaaagg uaggaucuac aagggauuga agguugguua 120

cccaguagcu cagguuuuac aggaggucgg agguugguuu uucuggauag aaaggucuau 180

aagagguuga ggguugguuu ggagauugua acggauuuau gagagaucga gaguugguua 240

aaaacaagga acggauuuac aagggguugg ggguugguua uacuuagcgg ug 292

<210> 46
<211> 282
<212> RNA
<213> Artificial Sequence

<220>
<223> Artificial circular RNA 1; target hybridization region:

SARS-CoV-2 Target A

<400> 46
ugcaguuaga uauggugcug ugucgucguc uauucuaagu uugggagauc cugcuggugg 60

uauuauguua cugucuguuc uaaauuugag gauaccuuua gcuggugucg uguuauuguc 120

uguuuuggac uuaggcucgg aaacgauugg uacuguguca cugucuguuu uaggcuugaa 180

gcugcuuccu cuugguguua ugucaucguc uauuuugaac uuggguccca cauagagugg 240

uaccaugucg uugucuguuc uggauuuaaa ccagggccaa ac 282

<210> 47
<211> 282
<212> RNA
<213> Artificial Sequence

<220>
<223> Artificial circular RNA 2; target hybridization region:
      SARS-CoV-2 Target A

<400> 47
ugcaguuaga uaugguacug ugucacuguc uguucugaac uugagcguuc acgucucugg 60

ugcuauguug ucgucuauuc uaaacuuaag aacuagguau aguggugucg uguuaucguc 120

uauuuugggc uugggggcuc cccuuggugg uaccauguca ccgucuauuc ugaauuuaaa 180

ucucccauag ugugguaucg uguugccguc uauuuuagac uuggaccacc gucuguuugg 240

uacuauguug cugucuguuu ugaacuugag aucagauuaa gg 282

<210> 48
<211> 282
<212> RNA
<213> Artificial Sequence

<220>
<223> Artificial circular RNA 3; target hybridization region:
      SARS-CoV-2 Target A

<400> 48
ugcaguuaga uaugguaccg ugucgucguc uauuuuggau uuaaaagauc cugcuggugg 60

uauuaugucg uuguuuguuc ugaguuuaaa gauaccuuua gcugguacca uguuaccguc 120

uguucuaagu uuaaacucgg aaacgauugg uauuguguug ucguuuguuc uagauuuaaa 180

gcugcuuccu cuugguaucg ugucgccguc uauucugagc uuaaauccca cauagagugg 240

uacuauguug uugucuauuu ugaguuuaaa ccagggccaa ac 282

<210> 49
<211> 282
<212> RNA
<213> Artificial Sequence

<220>
<223> Artificial circular RNA 4; target hybridization region:
      SARS-CoV-2 Target A

<400> 49
ugcaguuaga uaugguaccg ugucgccguu uguucugagc uuaaaguucu ugcgcucugg      60

uaucauguug cuguuuauuc uaggcuuaaa ucggccgucu ggugguauug ugucguuguc     120

uguucugagu uuaaaucucu uucccggugg uacuguguug ucgucuauuc uggguuuaaa     180

acucgacccc ggugguacca ugucaccguc uguuuuagac uuaaaguuac aagucgcugg     240

uauuauguug cugucuauuc uagauuuaaa auucccccag cc                        282


<210> 50
<211> 299
<212> RNA
<213> Artificial Sequence


<220>
<223> Artificial circular RNA 1; target hybridization region:
      SARS-CoV-2 Target C

<400> 50
ugcaguuaga uauagaggcc uugguggcag guuucuuggu gaagugagua uaguagaggu      60

cuuaguagca gguuuuuuag ugauguaacc uauguagaag ccucagcagc agauuucuug     120

gugggggguc aaggguaggg gucuuggugg uagauuuuuu gguggcacuu gaggaguagg     180

aguuucggcg guggauuucu uagugggcua ggaaauuugg gagcuuuggu gguagguuuu     240

uuggugaggu gggaggguug ggggcuucgg uagcggauuu uuuaguggaa cugaaagcg      299


<210> 51
<211> 292
<212> RNA
<213> Artificial Sequence


<220>
<223> Artificial circular RNA 2; target hybridization region:
      SARS-CoV-2 Target C

<400> 51
ugcaguuaga uauaggaguu ucggcggcgg guuuuuuggu gaaaguugaa uuuagaaguu      60

ucgguggcgg guuucuuagu gagaaauuca gauagaaguu uuggcagugg auuucuuggu     120

gaggaauggg uuuggaagcc uugguagugg auuuuuuagu ggccacccuu gguagaagcu     180

uuggugguag auuuuuuggu ggguaacuua auugggaguu uugguggcag guuuuuuagu     240

gauaggagac cguagggguu ucagcaguag auuucuuagu gggagacaga cu             292


<210> 52
<211> 292

<212> RNA
<213> Artificial Sequence

<220>
<223> Artificial circular RNA 3; target hybridization region:
      SARS-CoV-2 Target C

<400> 52
ugcaguuaga uauaggaguc ucggcagcgg auuuuuuagu ggacaugacu uuuagaggcc      60

uugguagcgg auuucuuagu ggucuacagc gaugggaguu uuggcggugg guuuuuuggu     120

gaccucuaau guuggaggcu ucagcagcag guuuuuuagu gauaagagua gguaggggcc     180

uuaguagcag auuucuuggu gggauucgaa uuuagagguu uuaguaguag guuucuuagu     240

gaagggcuuu auuggggguu uuagcggcag auuuuuuggu ggauucgauc ug             292

<210> 53
<211> 292
<212> RNA
<213> Artificial Sequence

<220>
<223> Artificial circular RNA 4; target hybridization region:
      SARS-CoV-2 Target C

<400> 53
ugcaguuaga uauggaaguu uuggcagugg guuucuuagu ggugggacuu gauagaaguc      60

uuagugguag guuucuuggu ggggcggagu guuggaggcu uugguagcgg guuuuuuagu     120

ggggcuaucu cgugggaguc uugguaguag auuucuuggu gaaggaugcg gguggaaguc     180

uuagcggcgg auuuuuuggu ggccaguuag uauggggguu uugguagcag guuuuuuggu     240

gauaguagac ucuggaggcu ucgguagcag auuuuuuagu gaguuauaaa gg             292

<210> 54
<211> 270
<212> RNA
<213> Artificial Sequence

<220>
<223> Artificial circular RNA 1; target hybridization region:
      SARS-CoV-2 Target D

<400> 54
ugcaguuaga uaaaaccgua agcagccugu agaagguaga cgaguccaga cguacaaacc      60

guaggcagcu ugcagaagau agacgaagaa cgaguacuaa accgugggca guuugcgggg     120

gauagacgaa gcuuggagac uaaaccguaa guagucugug ggggauggac gaauuucugg     180

caauaaaccg uagguaguuu gcagagggua gacgagcucg uagcuagaaa ccgugaguag     240

ccuguagagg auggacgacu gagaccucaa                                      270

<210> 55
<211> 270
<212> RNA
<213> Artificial Sequence

<220>
<223> Artificial circular RNA 2; target hybridization region:
      SARS-CoV-2 Target D

<400> 55
ugcaguuaga uaaaaccgua ggcagccugu aggagguaga cgaguuuucu cacggaaacc      60

guggguagcu uguaggagau agacgaguug aggaccagaa accguaagug guuugcagga     120

gauggacgag uuugggcagu uaaaccguga gcagucugca ggggguagac gaauguucgg     180

agcaaaaccg ugaguaguuu guagaaggug gacgagagga gaguaggaaa ccguaagcag     240

ucuguagggg auggacgauu uauaaucagu                                       270

<210> 56
<211> 270
<212> RNA
<213> Artificial Sequence

<220>
<223> Artificial circular RNA 3; target hybridization region:
      SARS-CoV-2 Target D

<400> 56
ugcaguuaga uaaagccgug agcggcuugu ggaagauaga ugaaaggauu ggaaaagacc      60

guaagcagcu uguagaagau gggugacaag uaauggaaaa auuguaagug gucugcggaa     120

ggugggugga uaaggaacuu gagaucguag guaguuugua gaggguaggu gggacuaugg     180

ggaugaacug ugggcggucu gcaggaggug gacgagagag guacauaaga ccguaaguag     240

uuuguggggg augggcgaug uauugcacac                                       270

<210> 57
<211> 270
<212> RNA
<213> Artificial Sequence

<220>
<223> Artificial circular RNA 4; target hybridization region:
      SARS-CoV-2 Target D

<400> 57
ugcaguuaga uaaagccgug agcggcuugu ggaagauaga ugaaaggauu ggaaaagacc      60

guaagcagcu uguagaagau gggugacaag uaauggaaaa auuguaagug gucugcggaa     120

ggugggugga uaaggaacuu gagaucguag guaguuugua gaggguaggu gggacuaugg     180

ggaugaacug ugggcggucu gcaggaggug gacgagagag guacauaaag ccguaaguag     240

cuuguagggg auagauggug uauugcacac                                    270

<210> 58
<211> 27
<212> RNA
<213> Artificial Sequence

<220>
<223> SARS-CoV-2 target hybridization region - comprised in
5′Ã\204Â\202Ã\202Â\202Ä\202Â\202Ã\202Â\200Ã\204Â\202Ã\202Â\202Ä\202Â\202Ã\202
Â\231UTR SLII

<400> 58
aaccaacuuu cgaucucuug uagaucu                                          27

<210> 59
<211> 33
<212> RNA
<213> Artificial Sequence

<220>
<223> SARS-CoV-2 target hybridization region - Target A

<400> 59
uuuaaguuua gaauagacgg ugacauggua cca                                    33

<210> 60
<211> 30
<212> RNA
<213> Artificial Sequence

<220>
<223> SARS-CoV-2 target hybridization region - Target C

<400> 60
ucacuaagaa aucugcugcu gaggcuucua                                       30

<210> 61
<211> 31
<212> RNA
<213> Artificial Sequence

<220>
<223> SARS-CoV-2 target hybridization region - Target D

<400> 61
ucgucuaucu ucugcaggcu gcuuacgguu u                                     31

<210> 62
<211> 35
<212> RNA
<213> Artificial Sequence

<220>
<223> SARS-CoV-2 target hybridization region - comprised in
      3Ã\204Â\202Ã\213Â\230Ä\202Â\202Ã\202Â\200Ä\202Â\202Ã\202Â\231UTR
Replication Site

<400> 62
cagaaugaau ucucguaacu acauagcaca aguag                                35


<210> 63
<211> 289
<212> RNA
<213> Artificial Sequence


<220>
<223> Artificial circular RNA against WNV and DENV (circWD1-in vitro)

<400> 63
   ugcaguuaga uaugcaguua gauaauauug ggaggugugu uuuuugccuu uuuccguaga      60
   uccucgcgaa acuucuuggc cugguuuucu uuucaaaaga guccuuacgu auauuggagg     120
   guguguuuuu cgcuuuuuuc cgucucggaa acgauaacuu ccuggcuuga uuuuuucuuc     180
   aaaagcugcg uuaucuauau ugagaggcgu guuuuucguc uuuuuccguu ccaaccugga     240
   gaacuuuuug gccugauuuu cucuucaaaa ccaggccgua ccgacaguc                 289


<210> 64
<211> 307
<212> RNA
<213> Artificial Sequence


<220>
<223> Artificial circular RNA against HCV (circHCV-in vitro)

<400> 64
ugcaguuaga uacccccugg ggcucugaug aggaaccucu cugggguccc cacagcgagu      60

cuccuugggg ccccuagaau gaaguucuuu gggguuucau agcacgguuc uccugggguu    120

uucauacgau ugucucuugg ggucuugcgu guuuacccuu uugggguccu gcuaaggggg    180

cuuucugggg ccuuucgaau aagucuuuuu ggggccucgu uuuaucacuc uucugggguu     240

cccagccuuu cccuucuugg ggcuccuccg accaugcccc uugggguucu acccuguaug     300

gacaguc                                                               307